(19) Europäisches Patentamt
European Patent Office
Office européen des brevets

(11) Publication number: **0 444 964 B1**

# (12) EUROPEAN PATENT SPECIFICATION

(45) Date of publication of patent specification: **26.10.94**

(51) Int. Cl.[5]: **C07D 493/22**, A61K 31/365, A01N 43/90, //(C07D493/22, 313:00,311:00,311:00,307:00)

(21) Application number: **91301748.9**

(22) Date of filing: **01.03.91**

The file contains technical information submitted after the application was filed and not included in this specification

## (54) Milbemycin ether derivatives, their preparation and their anthelmintic uses.

(30) Priority: **01.03.90 JP 50761/90**

(43) Date of publication of application: **04.09.91 Bulletin 91/36**

(45) Publication of the grant of the patent: **26.10.94 Bulletin 94/43**

(84) Designated Contracting States: **AT BE CH DE DK ES FR GB GR IT LI LU NL SE**

(56) References cited:
**EP-A- 0 110 667**
**EP-A- 0 184 173**
**EP-A- 0 186 043**
**EP-A- 0 246 739**
**EP-A- 0 357 460**

(73) Proprietor: **Sankyo Company Limited**
**5-1 Nihonbashi Honcho 3-chome**
**Chuo-ku**
**Tokyo (JP)**

Proprietor: **CIBA-GEIGY AG**

**CH-4002 Basel (CH)**

(72) Inventor: **Morisawa, Yasuhiro**
**c/o Sankyo Company Ltd.**
**2-58, Hiromachi 1-chome**
**Shinagawa-ku Tokyo 140 (JP)**
Inventor: **Saito, Akio**
**c/o Sankyo Company Ltd.**
**2-58, Hiromachi 1-chome**
**Shinagawa-ku Tokyo 140 (JP)**
Inventor: **Naito, Satoru**
**c/o Sankyo Company Ltd.**
**2-58, Hiromachi 1-chome**
**Shinagawa-ku Tokyo 140 (JP)**
Inventor: **Toyama, Toshimitsu**
**c/o Sankyo Company Ltd.**
**2-58, Hiromachi 1-chome**
**Shinagawa-ku Tokyo 140 (JP)**
Inventor: **Kaneko, Susumu**
**c/o Sankyo Company Ltd.**
**2-58, Hiromachi 1-chome**
**Shinagawa-ku Tokyo 140 (JP)**
Inventor: **Molleyres, Louis-Pierre**
**Walter-Fürst-Strasse 5**
**CH-4102 Binningen (CH)**
Inventor: **Gehret, Jean-Claude**
**Im Aeschfeld 12**
**CH-4147 Aesch (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

(74) Representative: **Gibson, Christian John Robert et al**
**MARKS & CLERK,**
**57/60 Lincoln's Inn Fields**
**London WC2A 3LS (GB)**

## Description

The present invention relates to a series of new macrolide compounds which are chemically related to certain known classes of macrolides including those known as the milbemycins and the avermectins. These compounds have valuable acaricidal, insecticidal and anthelmintic activities. The invention also provides methods of preparing these compounds and compositions and methods for using them.

There are several classes of known compounds with a structure based on a 16-membered macrolide ring, which compounds are obtained by fermentation of various microorganisms or are obtained semi-synthetically by chemical derivatization of such natural fermentation products, and which exhibit acaricidal, insecticidal, anthelmintic and antiparasitic activities. The milbemycins and avermectins are examples of two such classes of known compounds, but various others also exist and are identified in the art by different names or code numbers. The names for these various macrolide compounds have generally been taken from the names or code numbers of the microorganisms which produce the naturally occurring members of each class, and these names have then been extended to cover the chemical derivatives of the same class, with the result that there has been no standardized systematic nomenclature for such compounds generally.

In order to avoid confusion, a standardized system of nomenclature will be used herein, which follows the normal rules for naming derivatives of organic compounds as recommended by the International Union of Pure and Applied Chemistry (IUPAC), Organic Chemistry Division, Commission on Nomenclature of Organic Chemistry, and which is based primarily on the hypothetical parent compound hereby defined as "milbemycin" and represented by the formula (A):

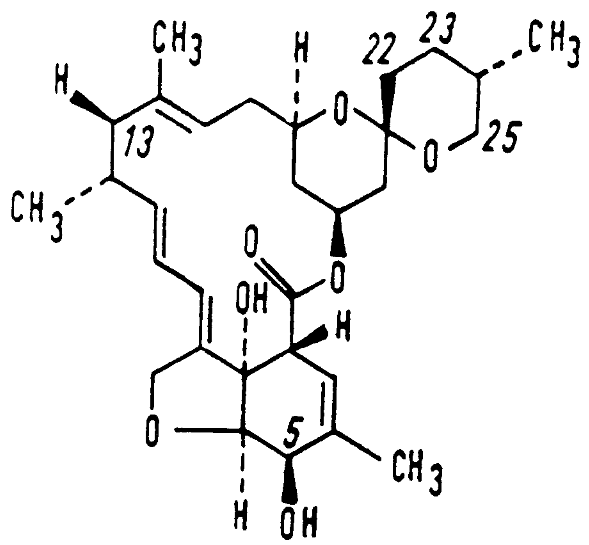

(A)

For the avoidance of doubt, formula (A) also shows the numbering of positions of the macrolide ring system applied to those positions most relevant to the compounds of the present invention and of the prior art.

The naturally produced milbemycins are a series of macrolide compounds known to have anthelmintic, acaricidal and insecticidal activities. Milbemycin D was disclosed in US Patent No. 4,346,171, where it was referred to as "Compound B-41D", and milbemycins $A_3$ and $A_4$ were disclosed in US Patent No. 3,950,360. These compounds may be represented by the above formula (A) in which there is a hydrogen atom at position 13 and position 25 is substituted with a methyl group, an ethyl group or an isopropyl group, these compounds being designated as milbemycin $A_3$, milbemycin $A_4$ and milbemycin D, respectively. The milbemycin analogue having a hydrogen atom at position 13 and substituted at position 25 with a sec-butyl group was disclosed in US Patent No. 4,173,571, where it was known as "13-deoxy-22,23-dihydroavermectin $B_{1a}$ aglycone". Certain of the compounds of the present invention are named as derivatives of this and related compounds, the numbering system being as shown above on formula (A).

Subsequently, various derivatives of the original milbemycins and avermectins have been prepared and their activities investigated. For example, 5-esterified milbemycins have been disclosed in US Patents No. 4,201,861, No. 4,206,205, No. 4,173,571, No. 4,171,314, No. 4,203,976, No. 4,289,760, No. 4,457,920, No. 4,579,864 and No. 4,547,491, in European Patent Publications No. 8184, No. 102,721, No. 115,930, No. 180,539 and No. 184,989 and in Japanese Patent Applications Kokai (i.e. as laid open to public inspection) No. 57-120589 and 59-16894.

13-Hydroxy-5-ketomilbemycin derivatives have been disclosed in US Patent No. 4,423,209. Milbemycin 5-oxime derivatives were disclosed in US Patent No. 4,547,520 and in European Patent Publication No. 203 832.

Milbemycins having an ether linkage at the 13 position are of particular relevance to the present invention and the lower alkyl, phenyl and benzyl ethers are described in general terms in US Patent 4 696 945, but only the methyl and ethyl ethers are specifically described in the Examples. Certain other milbemycin derivatives having a 13-ether group are disclosed in European Patent Publication No. 357 460, (an intermediate document) and European Patent Publication No. 246,739.

Like the milbemycins, the avermectins are based upon the same 16-membered ring macrolide compound. The avermectins are disclosed, for example in J. Antimicrob. Agents Chemother., 15(3), 361 - 367 (1979). These compounds may be represented by the above formula (A) but with a carbon-carbon double bond at positions 22 and 23, and having position 13 substituted with a 4'-(α-L-oleandrosyl)-α-L-oleandrosyloxy group. Position 25 may be substituted with an isopropyl group or a sec-butyl group, these compounds being designated as avermectin $B_{1b}$ and avermectin $B_{1a}$, respectively. 22,23-Dihydroavermectins $B_{1a}$ and $B_{1b}$ may be obtained by reduction of the double bond between the 22 and 23 positions and are disclosed in US Patent No. 4,199,569. The aglycone derivatives of the avermectins, which are milbemycin analogues, have sometimes been referred to in the literature as C-076 compounds, and various derivatives of these are known. For example, US Patent No. 4,201,861 discloses such derivatives substituted with a lower alkanoyl group at position 13.

European Patent Publication No. 170 006 discloses a family of bioactive compounds produced by fermentation, identified collectively by the code number LL-F28249. Some of these have a 16-membered macrolide structure corresponding to the above formula (A), substituted with a hydroxy group at position 23 and with a 1-methyl-1-propenyl, 1-methyl-1-butenyl or 1,3-dimethyl-1-butenyl group at position 25. In these compounds, the hydroxy group at position 5 may also be replaced by a methoxy group.

British Patent Publication No. 2,176,182 discloses another group of macrolide antibiotics corresponding to the above formula (A) with a hydroxy or substituted hydroxy group at position 5, a hydroxy, substituted hydroxy or keto group at position 23, and an α-branched alkenyl group at position 25.

The various classes of milbemycin-related macrolide compounds described above are all disclosed as having one or more types of activity as antibiotic, anthelmintic, ectoparasiticidal, acaricidal or other pesticidal agents. However, there is still a continuing need to provide such agents with improved activity against one or more classes of pests.

It has now been discovered that the activity of such milbemycin-related derivatives can be improved by appropriately selecting the combination of substituents on the macrolide ring system, especially the substituents at position 13. In particular, it has now been found that the activity of the compounds can be improved upon by appropriate selection of certain highly specific ether groups at the 13 position, which are cinnamyl ether groups or derivatives thereof, as specified below.

Accordingly, the present invention provides compounds having the formula (I):

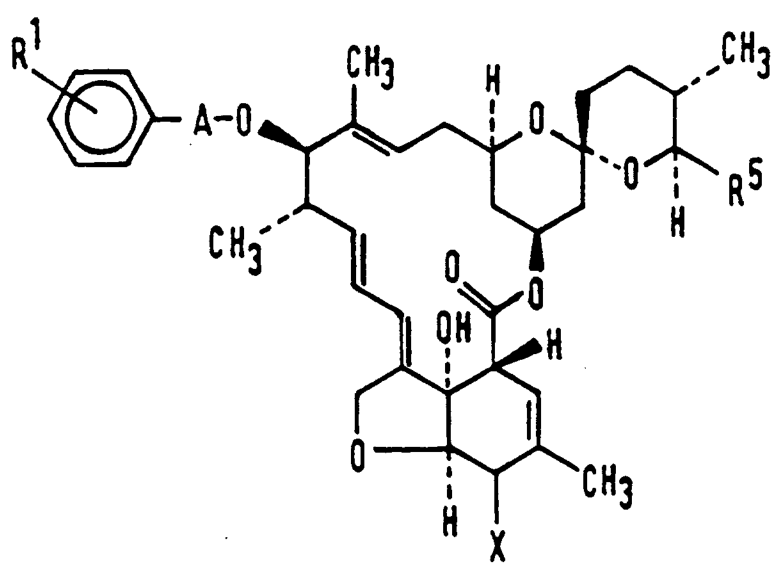

(I)

in which:
$R^1$ represents: a hydrogen atom; a halogen atom; a cyano group; a nitro group; an alkyl group which has from 1 to 4 carbon atoms and which is unsubstituted or has at least one substituent selected from substituents (a); an alkoxy group having from 1 to 4 carbon atoms; an alkoxyalkoxy group having a total of from 2 to 6 carbon atoms; or a group having one of the following formulae:

$-(CH_2)_nNHR^9$
$-(CH_2)_nNR^9COR^6$
$-(CH_2)_nNR^9COCOR^6$
$-(CH_2)_nNR^9COCOOR^7$
$-(CH_2)_nNR^9CHR^6NHCOR^6$
$-(CH_2)_nNR^9CHR^6NHCONHR^6$
$-(CH_2)_nNR^9CHR^6NHCOOR^7$
$-(CH_2)_nNR^9C(=Y)YR^6$
$-(CH_2)_nNR^9C(=Y)NR^6R^6$
$-(CH_2)_nNR^9C(=Y)NR^6NR^6R^6$
$-(CH_2)_nNR^9C(=Y)NR^6NHZ$
$-(CH_2)_nNR^9C(=NR^{11})NHR^{11}$
$-(CH_2)_nNR^9C(=NR^{11})R^6$
$-(CH_2)_nNR^9SO_mR^6$
$-CONHR^6$

or

$-COOR^7$

wherein:
m is 1 or 2;
n is 0, 1 or 2;
$R^6$ represents a hydrogen atom; an alkyl group having from 1 to 8 carbon atoms; a substituted alkyl group having from 1 to 8 carbon atoms and having at least one substituent selected from substituents (b); an aliphatic hydrocarbon group having from 2 to 8 carbon atoms and having one or two carbon-carbon double or triple bonds; a cycloalkyl group having from 2 to 8 carbon atoms; a substituted cycloalkyl group having from 2 to 8 carbon atoms and having at least one substituent selected from substituents (c); an aryl group which has from 6 to 14 ring carbon atoms and which is unsubstituted or which has at least one substituent selected from substituents (c); an aryloxy group which has from 6 to 14 ring carbon atoms and which is unsubstituted or which has at least one substituent selected from substituents (c); or an arylthio group which has from 6 to 14 ring carbon atoms and which is unsubstituted or which has at least one substituent selected from substituents (c); and, where there are two or more groups or atoms represented by $R^6$, these are the same or different from each other;
$R^7$ represents an alkyl group having from 1 to 4 carbon atoms, a cycloalkyl group having from 3 to 7 carbon atoms, or an aralkyl group in which an alkyl group having from 1 to 4 carbon atoms is substituted by from 1 to 3 aryl groups which have from 6 to 10 ring carbon atoms and which are unsubstituted or are substituted by at least one substituent selected from substituents (c);
$R^9$ represents a hydrogen atom or an alkyl group having from 1 to 4 carbon atoms;
$R^{11}$ represents any of the groups or atoms defined above for $R^6$, or it represents a cyano group, a nitro group, a group of formula $-COOR^7$ (wherein $R^7$ is as defined above), or a group of formula $-COR^6$ (wherein $R^6$ is as defined above);
Y represents an oxygen atom or a sulphur atom; and, where there are two or more groups represented by Y, these are the same or different from each other;
Z represents a group of formula $-COOR^7$ (wherein $R^7$ is as defined above), a group of formula $-COR^6$ -(wherein $R^6$ is as defined above) or a group of formula $-SO_2R^6$ (wherein $R^6$ is as defined above);
A represents a group having one of the following formulae:

$-CHR^2-CHR^3-CHR^4-$
$-CR^2=CR^3-CHR^4-$

or

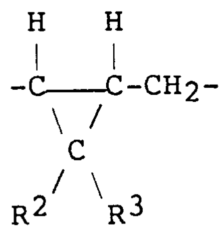

wherein $R^2$, $R^3$ and $R^4$ are independently selected from hydrogen atoms, halogen atoms, alkyl groups having from 1 to 4 carbon atoms, and alkoxy groups having from 1 to 4 carbon atoms;

$R^5$ represents a methyl group, an ethyl group, an isopropyl group or a sec-butyl group; and

X represents: a hydroxy group; an alkanoyloxy group which has from 1 to 5 carbon atoms, and which is unsubstituted or has at least one substituent selected from substituents (d); or a hydroxyimino group;

substituents (a):

halogen atoms, alkoxy groups having from 1 to 4 carbon atoms, alkylthio groups having from 1 to 4 carbon atoms, and alkanoyloxy groups having from 1 to 5 carbon atoms;

substituents (b):

cycloalkyl groups having from 3 to 8 carbon atoms; alkoxy groups having from 1 to 4 carbon atoms; alkylthio groups having from 1 to 4 carbon atoms; cyanoalkylthio groups having from 2 to 5 carbon atoms; alkoxycarbonyl groups having from 2 to 5 carbon atoms; halogen atoms; cyano groups; nitro groups; amino groups; aryl groups which have from 6 to 14 ring carbon atoms and which are unsubstituted or have at least one substituent selected from substituents (c); a pyridyl, thienyl, furyl, pyrrolyl, imidazolyl, triazolyl, tetrazolyl, thiazolyl, oxazolyl, indolyl group, or such a group substituted by at least one substituent selected from substituents (c); aryloxy groups which have from 6 to 14 ring carbon atoms and which are unsubstituted or have at least one substituent selected from substituents (c); and arylthio groups which have from 6 to 14 ring carbon atoms and which are unsubstituted or have at least one substituent selected from substituents (c);

substituents (c):

alkyl groups having from 1 to 4 carbon atoms, alkoxy groups having from 1 to 4 carbon atoms, alkylthio groups having from 1 to 4 carbon atoms, alkanoyloxy groups having from 1 to 5 carbon atoms, alkoxycarbonyl groups having from 2 to 5 carbon atoms, halogen atoms, cyano groups, nitro groups, amino groups, alkylamino groups in which the alkyl part has from 1 to 4 carbon atoms, dialkylamino groups in which each alkyl part is independently selected from alkyl groups having from 1 to 4 carbon atoms, carbamoyl groups, alkylcarbamoyl groups in which the alkyl part has from 1 to 4 carbon atoms, dialkylcarbamoyl groups in which each alkyl part is independently selected from alkyl groups having from 1 to 4 carbon atoms, and alkanoylamino groups having from 1 to 5 carbon atoms;

substituents (d):

halogen atoms, alkoxy groups having from 1 to 4 carbon atoms, alkoxycarbonyl groups having from 2 to 5 carbon atoms, and carboxy groups;

and salts and esters thereof.

The invention still further provides an anthelmintic, acaricidal and insecticidal composition comprising an anthelmintic, acaricidal and insecticidal compound in admixture with a pharmaceutically, agriculturally, veterinarily or horticulturally acceptable carrier or diluent, wherein said compound is selected from compounds of formula (I) and salts and esters thereof.

The invention still further provides the use of a compound of formula (I) or a salt or ester thereof in therapy.

The invention still further provides the use of a compound of formula (I) or a salt or ester thereof for the manufacture of a medicament for the treatment of animals parasitized by helminths, acarids or insects.

The invention still further provides a method of protecting animals or plants from damage by parasites selected from acarids, helminths and insects, which comprises applying an active compound to said animals, to said plants or to seeds of said plants or to a locus including said animals, plants or seeds, wherein the active compound is selected from at least one compound of formula (I) and salts and esters thereof.

In the compounds of the present invention, where $R^1$ represents a halogen atom, this may be a fluorine, chlorine, bromine or iodine atom, preferably a chlorine or bromine atom.

When $R^1$ represents an alkyl group, this may be a straight or branched chain alkyl group having from 1 to 4 carbon atoms, and examples include the methyl, ethyl, propyl, isopropyl, butyl, isobutyl, sec-butyl and

t-butyl groups, of which we prefer the methyl, ethyl, propyl, isopropyl, butyl and sec-butyl groups, most preferably the methyl group or the ethyl group. Such groups may be unsubstituted or they may have at least one substituent selected from substituents (a), defined above and exemplified below. Where the group is substituted, there is no particular restriction on the number of substituents, except such as may be imposed by the number of substitutable positions, and, possibly, by steric constraints. Examples of groups and atoms which may be represented by substituents (a) include:

halogen atoms, such as the fluorine, chlorine, bromine and iodine atoms;

alkoxy groups having from 1 to 4 carbon atoms, which may be straight or branched chain alkoxy groups having from 1 to 4 carbon atoms, and examples include the methoxy, ethoxy, propoxy, isopropoxy, butoxy, isobutoxy, sec-butoxy and t-butoxy groups, of which we prefer the methoxy, ethoxy, propoxy, isopropoxy and butoxy groups, most preferably the methoxy group;

alkylthio groups having from 1 to 4 carbon atoms, which may be straight or branched chain alkylthio groups having from 1 to 4 carbon atoms, and examples include the methylthio, ethylthio, propylthio, isopropylthio, butylthio, isobutylthio, sec-butylthio and t-butylthio groups, of which we prefer the methylthio, ethylthio, propylthio, isopropylthio, butylthio and isobutylthio groups, most preferably the methylthio group; and

alkanoyloxy groups having from 1 to 5 carbon atoms, which may be straight or branched chain groups, such as the formyloxy, acetoxy, propionyloxy, butyryloxy, isobutyryloxy, valeryloxy, isovaleryloxy and pivaloyloxy groups, of which we prefer the formyloxy, propionyloxy, butyryloxy, isovaleryloxy and pivaloyloxy groups.

When $R^1$ represents an alkoxy group, this may be a straight or branched chain alkoxy group having from 1 to 4 carbon atoms, and examples include the methoxy, ethoxy, propoxy, isopropoxy, butoxy, isobutoxy, sec-butoxy and t-butoxy groups, of which we prefer the methoxy, ethoxy, propoxy, isopropoxy, butoxy and sec-butoxy groups, most preferably the methoxy group.

Where $R^1$ represents an alkoxyalkoxy group, this has a total of from 2 to 6 carbon atoms, and each alkoxy part may be a straight or branched chain group, although each is preferably straight chain; preferably each alkoxy part has from 1 to 4 carbon atoms, provided that they total no more than 6. Examples of such groups include the methoxymethoxy, ethoxymethoxy, propoxymethoxy, butoxymethoxy, methoxyethoxy, ethoxyethoxy and butoxyethoxy groups.

Where $R^6$ represents an alkyl group this may be a straight or branched chain alkyl group having from 1 to 8, preferably from 1 to 4, carbon atoms, and the group may be unsubstituted or it may be substituted by at least one of substituents (b), defined above and exemplified below. Examples of such alkyl groups include the methyl, ethyl, propyl, isopropyl, butyl, isobutyl, sec-butyl, t-butyl, pentyl, isopentyl, neopentyl, hexyl, isohexyl, sec-hexyl, t-hexyl, heptyl, isoheptyl and octyl groups, preferably the methyl, ethyl, propyl, isopropyl, butyl, sec-butyl, pentyl, isopentyl, hexyl, sec-hexyl and heptyl groups. Examples of the groups and atoms which may be included in substituents (b) include:

cycloalkyl groups having from 3 to 8 carbon atoms, such as the cyclopropyl, cyclobutyl, cyclopentyl, cyclohexyl and cycloheptyl groups;

halogen atoms, alkoxy groups having from 1 to 4 carbon atoms and alkylthio groups having from 1 to 4 carbon atoms, such as those exemplified in relation to substituents (a);

cyanoalkylthio groups having from 2 to 5 carbon atoms, such as the cyanomethylthio, 1-cyanoethylthio, 2-cyanoethylthio, 1-cyanopropylthio, 2-cyanopropylthio and 1-cyanobutylthio groups;

alkoxycarbonyl groups having from 2 to 5 carbon atoms such as the methoxycarbonyl, ethoxycarbonyl, propoxycarbonyl, isopropoxycarbonyl, butoxycarbonyl, isobutoxycarbonyl, sec-butoxycarbonyl and t-butoxycarbonyl groups, preferably the methoxycarbonyl, ethoxycarbonyl, propoxycarbonyl and butoxycarbonyl groups;

cyano groups, nitro groups and amino groups;

carbocyclic aryl groups which have from 6 to 14, preferably from 6 to 10 and more preferably 6 or 10, ring carbon atoms and which are unsubstituted or have at least one substituent selected from substituents (c), defined and exemplified below, such as the phenyl, naphthyl (1- or 2-) and anthryl groups, of which the phenyl and naphthyl groups are preferred and the phenyl group is most preferred; such groups may be unsubstituted or substituted;

aromatic heterocyclic groups which have 5 or 6 ring atoms and which are unsubstituted or which have at least one substituent selected from substituents (c); examples of the unsubstituted groups include the pyridyl, thienyl, furyl, pyrrolyl, imidazolyl, triazolyl, tetrazolyl, thiazolyl and oxazolyl groups, and such groups which are fused to at least one benzene ring, such as the indolyl group; any of these groups may be unsubstituted or they may be substituted by at least one of substituents (c), defined above and exemplified below;

aryloxy groups which have from 6 to 14 ring carbon atoms and which are unsubstituted or have at least one substituent selected from substituents (c); and arylthio groups which have from 6 to 14 ring carbon atoms and which are unsubstituted or have at least one substituent selected from substituents (c); in each case, the aryl part of these groups may be as exemplified above in relation to the aryl groups.

Examples of the groups and atoms which may be represented by substituents (c) include:

alkyl groups having from 1 to 4 carbon atoms, alkoxy groups having from 1 to 4 carbon atoms, alkylthio groups having from 1 to 4 carbon atoms, alkanoyloxy groups having from 1 to 5 carbon atoms, alkoxycarbonyl groups having from 2 to 5 carbon atoms and halogen atoms, such as those exemplified above in relation to substituents (a) and/or (b);

cyano groups, nitro groups and amino groups;

alkylamino groups and dialkylamino groups in which the or each alkyl part has from 1 to 4 carbon atoms, such as the methylamino, ethylamino, propylamino, isopropylamino, dimethylamino, diethylamino, N-butyl-N-methylamino, N-t-butyl-N-methylamino, N-methyl-N-propylamino, N-ethyl-N-propylamino, dipropylamino, diisopropylamino, butylamino, isobutylamino, dibutylamino and diisobutylamino groups, especially the methylamino, ethylamino, propylamino, butylamino, dimethylamino and diethylamino groups;

carbamoyl groups, alkylcarbamoyl groups and dialkylcarbamoyl groups in which the or each alkyl part is independently selected from alkyl groups having from 1 to 4 carbon atoms, such as the carbamoyl, methylcarbamoyl, ethylcarbamoyl, propylcarbamoyl, isopropylcarbamoyl, dimethylcarbamoyl, diethylcarbamoyl, N-butyl-N-methylcarbamoyl, N-t-butyl-N-methylcarbamoyl, N-methylN-propylcarbamoyl, N-ethyl-N-propylcarbamoyl, dipropylcarbamoyl, diisopropylcarbamoyl, butylcarbamoyl, isobutylcarbamoyl, dibutylcarbamoyl and diisobutylcarbamoyl groups, especially the methylcarbamoyl, ethylcarbamoyl, propylcarbamoyl, butylcarbamoyl, dimethylcarbamoyl and diethylcarbamoyl groups; and

alkanoylamino groups having from 1 to 5 carbon atoms, which may be straight or branched chain groups, such as the formamido, acetamido, propionamido, butyramido, isobutyramido, valeramido, isovaleramido and pivaloylamino groups, of which we prefer the formamido, acetamido, propionamido and butyramido groups.

Where $R^6$ represents an aliphatic hydrocarbon group having one or two carbon-carbon double or triple bonds, this is preferably an alkenyl group, an alkadienyl group or an alkynyl group, and may be a straight or branched chain group having from 2 to 8, preferably from 2 to 6, and more preferably 3 or 4, carbon atoms. Examples of the alkenyl groups include the vinyl, allyl, methallyl, 1-propenyl, isopropenyl, 1-butenyl, 2-butenyl, 3-butenyl, 1-pentenyl, 2-pentenyl, 3-pentenyl and 4-pentenyl groups, of which the allyl, methallyl, 1-propenyl, isopropenyl and butenyl groups are preferred, the allyl and 2-butenyl groups being most preferred. Examples of the alkadienyl groups include groups having from 3, preferably from 4, to 8 carbon atoms, such as the butadienyl and hexadienyl groups, more preferably the hexadienyl group. Examples of the alkynyl groups include the ethynyl, propargyl (2-propynyl), 1-propynyl, 1-butynyl, 2-butynyl, 3-butynyl, 1-pentynyl, 2-pentynyl, 3-pentynyl and 4-pentynyl groups, of which the propynyl and butynyl groups are preferred, the propargyl and 2-butynyl groups being most preferred.

Where $R^6$ represents an aryl, aryloxy or arylthio group, this may be as exemplified above in relation to substituents (b).

Where $R^1$ represents a group of formula

$$-(CH_2)_nNR^9C(=Y)NR^6R^6$$

the two groups $R^6$ attached to a single nitrogen atom may be the same or different and may be selected from those groups represented by $R^6$ and defined and exemplified above.

Where $R^1$ represents a group of formula

$$-(CH_2)_nNR^9C(=Y)NR^6NR^6R^6$$

the two groups $R^6$ attached to a single nitrogen atom may be the same or different and may be selected from those groups represented by $R^6$ and defined and exemplified above.

Where $R^7$ represents an alkyl group having from 1 to 4 carbon atoms, this may be as defined and exemplified above in relation to the groups which may be represented by $R^1$.

Where $R^7$ represents an aralkyl group, the alkyl part has from 1 to 4 carbon atoms and may be any of the alkyl groups exemplified above. The aryl part or parts has or have from 6 to 10 carbon atoms in its ring and again, may be any of the aryl groups exemplified above. The aryl part or parts may be substituted or unsubstituted and, if substituted, the substituents are chosen from substituents (c), defined and exemplified above. However, the unsubstituted groups are preferred. There may be from 1 to 3 such aryl groups.

Examples of such aralkyl groups include the benzyl, phenethyl, α-methylbenzyl, 1-phenylpropyl, 2-phenylpropyl, 3-phenylpropyl, 4-phenylbutyl, benzhydryl and trityl groups, of which the benzyl and phenethyl groups are preferred.

Where X represents an alkanoyloxy group, it contains from 1 to 5 carbon atoms and may be a straight or branched chain group. Examples of such groups include the formyloxy, acetoxy, propionyloxy, butyryloxy, isobutyryloxy, valeryloxy, isovaleryloxy and pivaloyloxy groups. Such groups may be unsubstituted, or they may have at least one substituent selected from substituents (d), defined above and exemplified below.

Examples of groups and atoms which may be represented by substituents (d) include:

halogen atoms and alkoxy groups having from 1 to 4 carbon atoms, as exemplified in relation to substituents (a);

alkoxycarbonyl groups having from 2 to 5 carbon atoms, as exemplified in relation to substituents (b); and

carboxy groups.

In general, in the discussion above, where reference is made to a substituted group, there is no particular restriction on the number of substituents, except such as may be imposed by the number of substitutable positions, or possibly by steric constraints, each of which is well recognised by those skilled in the art. However, as a general rule, we normally find it convenient to have no more than 3 such substituents, and sometimes fewer, i.e. 1, 2 or 3. More preferably, the number of the substituents is 1, 2 or 3 where the substituent is a halogen atom, and 1 in other cases.

Where $R^7$ represents a hydrogen atom or substituent (d) is a carboxy group, the compounds can form salts with various sorts of bases. Such salts includes, for example: salts with an alkali metal, such as lithium, sodium or potassium; salts with an alkaline earth metal, such as calcium or barium; salts with another metal, such as magnesium or aluminium; and salts with an organic amine, such as triethylamine or triethanolamine.

Of the compounds of formula (I) of the present invention, representative preferred classes are as follows:

(1) those wherein $R^1$ represents: a hydrogen atom, an alkyl group having from 1 to 3 carbon atoms, such as the methyl, ethyl, propyl and isopropyl groups; an alkoxy group having from 1 to 3 carbon atoms, such as the methoxy, ethoxy, propoxy and isopropoxy groups; the fluorine and chlorine atoms; and the nitro and amino groups;

(2) those wherein $R^1$ represents a group of formula $-NR^{9a}COR^{6a}$

wherein:

$R^{9a}$ represents a hydrogen atom or a methyl group; and

$R^{6a}$ represents a hydrogen atom, an alkyl group having from 1 to 4 carbon atoms, such as the methyl, ethyl, propyl, isopropyl and butyl groups; a cycloalkyl group having from 3 to 5 carbon atoms, such as the cyclopropyl, cyclobutyl and cyclopentyl groups; an alkyl group having from 1 to 3 carbon atoms, and substituted with a halogen atom, a cyano group, an alkoxy group having from 1 to 3 carbon atoms, an alkylthio group having from 1 to 3 carbon atoms, a cyanomethylthio group or a phenoxy group, such as the fluoromethyl, bromoethyl, difluoromethyl, cyanomethyl, cyanopropyl, methoxymethyl, ethoxymethyl, methylthiomethyl, cyanomethylthiomethyl and phenoxymethyl groups; an alkenyl group having from 2 to 4 carbon atoms, such as the vinyl and allyl groups; a phenyl group; or a phenyl group substituted with an alkyl group having from 1 to 3 carbon atoms, an alkoxy group having from 1 to 3 carbon atoms, a halogen atom or a nitro group, such as the tolyl, methoxyphenyl, fluorophenyl and nitrophenyl groups;

(3) those wherein $R^1$ represents a group of formula $-NR^{9a}COCOR^{6b}$

wherein:

$R^{9a}$ represents a hydrogen atom or a methyl group; and

$R^{6b}$ represents a hydrogen atom, an alkyl group having from 1 to 4 carbon atoms, such as the methyl, ethyl, propyl, isopropyl and butyl groups; a cycloalkyl group having from 3 to 5 carbon atoms, such as the cyclopropyl, cyclobutyl and cyclopentyl groups; an alkenyl group having from 2 to 4 carbon atoms, such as the vinyl and allyl groups; a phenyl group; or a phenyl group substituted with an alkyl group having from 1 to 3 carbon atoms, an alkoxy group having from 1 to 3 carbon atoms, a halogen atom or a nitro group, such as the tolyl, methoxyphenyl, fluorophenyl and nitrophenyl groups;

(4) those wherein $R^1$ represents a group of formula $-NR^{9a}C(=Y)YR^{6c}$

wherein:

$R^{9a}$ represents a hydrogen atom or a methyl group;

Y represents an oxygen atom; and

$R^{6c}$ represents: an alkyl group having from 1 to 4 carbon atoms, such as the methyl, ethyl, propyl, isopropyl and butyl groups; an alkyl group having from 1 to 4 carbon atoms, and substituted with a halogen atom or an alkoxy group having from 1 to 3 carbon atoms, such as the fluoroethyl, trichloroethyl, methoxyethyl and ethoxyethyl groups; a vinyl group; an allyl group; a benzyl group; a methoxybenzyl group; nitrobenzyl group; or a phenyl group;

(5) those wherein $R^1$ represents a group of formula $-NR^{9a}C(=Y)NR^{6d}R^{6e}$

wherein:

$R^{9a}$ represents a hydrogen atom or a methyl group;

Y represents an oxygen atom or a sulphur atom; and

$R^{6d}$ and $R^{6e}$ are independently selected from: hydrogen atoms; alkyl groups having from 1 to 4 carbon atoms, such as the methyl, ethyl, propyl, isopropyl and butyl groups; cycloalkyl groups having from 3 to 6 carbon atoms, such as the cyclopropyl, cyclobutyl, cyclopentyl and cyclohexyl groups; phenyl groups; phenyl groups substituted with an alkyl group having from 1 to 3 carbon atoms, an alkoxy group having from 1 to 3 carbon atoms, a halogen atom or a nitro group, such as the tolyl, methoxyphenyl, fluorophenyl and nitrophenyl groups;

(6) those wherein $R^1$ represents a group of formula $-NR^{9a}C(=Y)NR^{6f}NR^{6g}R^{6h}$

wherein:

$R^{9a}$ represents a hydrogen atom or a methyl group;

Y represents an oxygen atom or a sulphur atom; and

$R^{6f}$, $R^{6g}$ and $R^{6h}$ are independently selected from: hydrogen atoms, alkyl groups having from 1 to 4 carbon atoms, such as the methyl, ethyl, propyl, isopropyl and butyl groups; cycloalkyl groups having from 3 to 6 carbon atoms, such as the cyclopropyl, cyclobutyl, cyclopentyl and cyclohexyl groups; phenyl groups; and phenyl groups substituted with an alkyl group having from 1 to 3 carbon atoms, an alkoxy group having from 1 to 3 carbon atoms, a halogen atom or a nitro group, such as the tolyl, methoxyphenyl, fluorophenyl and nitrophenyl groups;

(7) those wherein $R^1$ represents a group of formula $-NR^{9a}C(=Y)NR^{6j}NHZ$

wherein:

$R^{9a}$ represents a hydrogen atom or a methyl group;

Y represents an oxygen atom or a sulphur atom;

$R^{6j}$ represents an alkyl group having from 1 to 4 carbon atoms, such as the methyl, ethyl, propyl, isopropyl and butyl groups, or a cycloalkyl group having from 3 to 6 carbon atoms, such as the cyclopropyl, cyclobutyl, cyclopentyl and cyclohexyl groups;

Z represents a group of formula $-COOR^{7a}$ (wherein: $R^{7a}$ represents an alkyl group having from 1 to 4 carbon atoms, such as the methyl, ethyl, propyl, isopropyl and butyl groups, or a cycloalkyl group having from 3 to 6 carbon atoms, such as the cyclopropyl, cyclobutyl, cyclopentyl and cyclohexyl groups; or a benzyl group); a group of formula $-COR^{6k}$ (wherein $R^{6k}$ represents: an alkyl group having from 1 to 4 carbon atoms, such as the methyl, ethyl, propyl, isopropyl and butyl groups; a cycloalkyl group having from 3 to 6 carbon atoms, such as the cyclopropyl, cyclobutyl, cyclopentyl and cyclohexyl groups; a phenyl group; or a phenyl group substituted with an alkyl group having from 1 to 3 carbon atoms, an alkoxy group having from 1 to 3 carbon atoms, a halogen atom or a nitro group, such as the tolyl, methoxyphenyl, fluorophenyl and nitrophenyl groups), or a group of formula $-SO_2R^{6m}$ (wherein $R^{6m}$ represents: an alkyl group having from 1 to 4 carbon atoms, such as the methyl, ethyl, propyl, isopropyl and butyl groups; a phenyl group; or a phenyl group substituted with an alkyl group having from 1 to 3 carbon atoms, an alkoxy group having from 1 to 3 carbon atoms, a halogen atom or a nitro group, such as the tolyl, methoxyphenyl, fluorophenyl and nitrophenyl groups);

(8) those wherein $R^1$ represents a group of formula $-NR^{9a}C(=NR^{11a})NHR^{11b}$

wherein:

$R^{9a}$ represents a hydrogen atom or a methyl group;

$R^{11a}$ and $R^{11b}$ are independently selected from: hydrogen atoms; alkyl groups having from 1 to 4 carbon atoms, such as the methyl, ethyl, propyl, isopropyl and butyl groups; phenyl groups; and phenyl groups substituted with an alkyl group having from 1 to 3 carbon atoms, an alkoxy group having from 1 to 3 carbon atoms, a halogen atom or a nitro group, such as the tolyl, methoxyphenyl, fluorophenyl and nitrophenyl groups; groups of formula $-COOR^{7b}$ (wherein $R^{7b}$ represents: an alkyl group having from 1 to 4 carbon atoms, such as the methyl, ethyl, propyl, isopropyl and butyl groups; a cycloalkyl group having from 3 to 6 carbon atoms, such as the cyclopropyl, cyclobutyl, cyclopentyl and cyclohexyl groups; or a benzyl group); groups of formula $-COR^{6n}$ (wherein $R^{6n}$ represents: an alkyl group having from 1 to 4 carbon atoms, such as the methyl, ethyl, propyl, isopropyl and butyl groups; a cycloalkyl group having from 3 to 6 carbon atoms, such as the cyclopropyl, cyclobutyl, cyclopentyl and cyclohexyl groups;

phenyl groups; and phenyl groups substituted with an alkyl group having from 1 to 3 carbon atoms, an alkoxy group having from 1 to 3 carbon atoms, a halogen atom or a nitro group, such as the tolyl, methoxyphenyl, fluorophenyl and nitrophenyl groups);

(9) those wherein $R^1$ represents a group of formula $-NR^{9a}C(=NR^{11c})R^{6p}$

wherein:

$R^{9a}$ represents a hydrogen atom or a methyl group;

$R^{11c}$ represents: a hydrogen atom; an alkyl group having from 1 to 4 carbon atoms, such as the methyl, ethyl, propyl, isopropyl and butyl groups; a phenyl group; a phenyl group substituted with an alkyl group having from 1 to 3 carbon atoms, an alkoxy group having from 1 to 3 carbon atoms, a halogen atom or a nitro group, such as the tolyl, methoxyphenyl, fluorophenyl and nitrophenyl groups; groups of formula $-COOR^{7c}$ (wherein $R^{7c}$ represents: an alkyl group having from 1 to 4 carbon atoms, such as the methyl, ethyl, propyl, isopropyl and butyl groups; a cycloalkyl group having from 3 to 6 carbon atoms, such as the cyclopropyl, cyclobutyl, cyclopentyl and cyclohexyl groups; or a benzyl group); a group of formula $-COR^{6q}$ (wherein $R^{6q}$ represents: an alkyl group having from 1 to 4 carbon atoms, such as the methyl, ethyl, propyl, isopropyl and butyl groups; a cycloalkyl group having from 3 to 6 carbon atoms, such as the cyclopropyl, cyclobutyl, cyclopentyl and cyclohexyl groups; a phenyl group; or a phenyl group substituted with an alkyl group having from 1 to 3 carbon atoms, an alkoxy group having from 1 to 3 carbon atoms, a halogen atom or a nitro group, such as the tolyl, methoxyphenyl, fluorophenyl and nitrophenyl groups);

$R^{6p}$ represents: an alkyl group having from 1 to 4 carbon atoms, such as the methyl, ethyl, propyl, isopropyl and butyl groups; or a cycloalkyl group having from 3 to 6 carbon atoms, such as the cyclopropyl, cyclobutyl, cyclopentyl and cyclohexyl groups; a phenyl group; or a phenyl group substituted with an alkyl group having from 1 to 3 carbon atoms, an alkoxy group having from 1 to 3 carbon atoms, a halogen atom or a nitro group, such as the tolyl, methoxyphenyl, fluorophenyl and nitrophenyl groups);

(10) those wherein $R^1$ represents a group of formula $-NR^{9a}SO_mR^{6r}$

wherein:

$R^{9a}$ represents a hydrogen atom or a methyl group;

$R^{6r}$ represents: an alkyl group having from 1 to 4 carbon atoms, such as the methyl, ethyl, propyl, isopropyl and butyl groups; an alkyl group substituted with a cyano group, such as the cyanomethyl and cyanoethyl groups; a phenyl group; or a phenyl group substituted with an alkyl group having from 1 to 3 carbon atoms, an alkoxy group having from 1 to 3 carbon atoms, a halogen atom or a nitro group, such as the tolyl, methoxyphenyl, fluorophenyl and nitrophenyl groups; and

m is 1 or 2;

(11) compounds as defined in any one of (2) to (10) above, wherein A represents a group of formula:

$-CHR^2-CHR^3-CHR^4-$

wherein $R^2$, $R^3$ and $R^4$ are independently selected from hydrogen atoms and methyl groups;

(12) compounds as defined in any one of (2) to (10) above, wherein A represents a group of formula:

$-CR^2=CR^3-CHR^4-$

wherein $R^2$, $R^3$ and $R^4$ are independently selected from hydrogen atoms and methyl groups;

(13) compounds as defined in any one of (2) to (10) wherein A represents a group of formula:

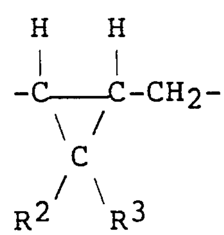

wherein $R^2$ and $R^3$ are independently selected from hydrogen atoms, chlorine atoms and methyl groups;

(14) compounds as defined in (11) above, where the substituent $R^1$ is at the meta or the ortho position;
(15) compounds as defined in (12) above, where the substituent $R^1$ is at the meta or ortho position;
(16) those wherein $R^5$ represents an ethyl group;
(17) mixtures of a compound wherein $R^5$ represents an ethyl group and the corresponding compound wherein $R^5$ represents a methyl group;
(18) those wherein $R^3$ and $R^4$ each represent hydrogen atoms; and
(19) those wherein X represents a hydroxy group.

Typical examples of compounds of the present invention are as follows:

13-(3-Phenylpropoxy)milbemycin $A_4$,
13-(3-Phenylpropoxy)milbemycin $A_3$,
13-(3-Phenylpropoxy)milbemycin D,
13-Deoxy-13-(3-phenylpropoxy)-22,23-dihydroavermectin $B_{1a}$ aglycone,
13-Cinnamyloxymilbemycin $A_4$,
13-Cinnamyloxymilbemycin $A_3$,
13-Cinnamyloxymilbemycin D,
13-Deoxy-13-cinnamyloxy-22,23-dihydroavermectin $B_{1a}$ aglycone,
13-(3-Phenylbutyloxy)milbemycin $A_4$,
13-(3-Bromo-3-phenylpropoxy)milbemycin $A_4$,
13-(3-Methoxy-3-phenylpropoxy)milbemycin $A_4$,
13-(2-Phenylcyclopropylmethoxy)milbemycin $A_4$,
13-(3,3-Dichloro-2-phenylcyclopropylmethoxy)milbemycin $A_4$,
13-(3,3-Dimethyl-2-phenylcyclopropylmethoxy)milbemycin $A_4$,
13-($\beta$-Methylcinnamyloxy)milbemycin $A_4$,
13-($\beta$-Bromocinnamyloxy)milbemycin $A_4$,
13-($\gamma$-Methylcinnamyloxy)milbemycin $A_4$,
13-($\gamma$-Methoxycinnamyloxy)milbemycin $A_4$,
13-[(Z)-3-Phenyl-2-methylpropenyloxy]milbemycin $A_4$,
13-[(Z)-3-Phenyl-3-methylpropenyloxy]milbemycin $A_4$,
13-[3-(4-Nitrophenyl)propoxy]milbemycin $A_4$,
13-[3-(3-Nitrophenyl)propoxy]milbemycin $A_4$,
13-[3-(2-Nitrophenyl)propoxy]milbemycin $A_4$,
13-[3-(4-Aminophenyl)propoxy]milbemycin $A_4$,
13-[3-(3-Aminophenyl)propoxy]milbemycin $A_4$,
13-[3-(2-Aminophenyl)propoxy]milbemycin $A_4$,
13-[3-(4-Acetamidophenyl)propoxy]milbemycin $A_4$,
13-[3-(3-Acetamidophenyl)propoxy]milbemycin $A_4$,
13-[3-(2-Acetamidophenyl)propoxy]milbemycin $A_4$,
13-[3-(3-Cyanoacetamidophenyl)propoxy]milbemycin $A_4$,
13-[3-(2-Cyanoacetamidophenyl)propoxy]milbemycin $A_4$,
13-[3-(4-Ethoxycarbonylaminophenyl)propoxy]milbemycin $A_4$,
13-[3-(3-Ethoxycarbonylaminophenyl)propoxy]milbemycin $A_4$,
13-[3-(2-Ethoxycarbonylaminophenyl)propoxy]milbemycin $A_4$,
13-[3-(4-Isopropoxycarbonylaminophenyl)propoxy]milbemycin $A_4$,
13-[3-(3-Isopropoxycarbonylaminophenyl)propoxy]milbemycin $A_4$,
13-[3-(2-Isopropoxycarbonylaminophenyl)propoxy]milbemycin $A_4$,
13-[3-(4-Methanesulphonylaminophenyl)propoxy]milbemycin $A_4$,
13-[3-(3-Methanesulphonylaminophenyl)propoxy]milbemycin $A_4$,
13-[3-(2-Methanesulphonylaminophenyl)propoxy]milbemycin $A_4$,
13-{3-[4-(3-Methylureido)phenyl]propoxy}milbemycin $A_4$,
13-{3-[3(3-Methylureido)phenyl]propoxy}milbemycin $A_4$,
13-{3-[2-(3-Methylureido)phenyl]propoxy}milbemycin $A_4$,
13-{3-[3-(3-Cyclopropylureido)phenyl]propoxy}milbemycin $A_4$,
13-{3-[4-(3-Thiomethylureido)phenyl]propoxy}milbemycin $A_4$,
13-{3-[3-(3-Methylthioureido)phenyl]propoxy}milbemycin $A_4$,
13-{3-[2-(3-Methylthioureido)phenyl]propoxy}milbemycin $A_4$,
13-{3-[4-(3-Phenylureido)phenyl]propoxy}milbemycin $A_4$,
13-{3-[3-(3-Phenylureido)phenyl]propoxy}milbemycin $A_4$,
13-{3-[2-(3-Phenylureido)phenyl]propoxy}milbemycin $A_4$,

13-{3-[4-(3-p'-Toluylureido)phenyl]propoxy}milbemycin $A_4$,
13-{3-[3-(3-p'-Toluylureido)phenyl]propoxy}milbemycin $A_4$,
13-{3-[2-(3-p'-Toluylureido)phenyl]propoxy}milbemycin $A_4$,
13-(3-Nitrocinnamyloxy)milbemycin $A_4$,
13-(2-Nitrocinnamyloxy)milbemycin $A_4$,
13-(3-Methoxycinnamyloxy)milbemycin $A_4$,
13-(4-Aminocinnamyloxy)milbemycin $A_4$,
13-(3-Aminocinnamyloxy)milbemycin $A_4$,
13-(2-Aminocinnamyloxy)milbemycin $A_4$,
13-(4-Methylaminocinnamyloxy)milbemycin $A_4$,
13-(4-Acetamidocinnamyloxy)milbemycin $A_4$,
13-(3-Acetamidocinnamyloxy)milbemycin $A_4$,
13-(3-Acetamidocinnamyloxy)milbemycin $A_4$ 5-oxime,
13-(2-Acetamidocinnamyloxy)milbemycin $A_4$,
13-[3-(N-Acetyl-N-methylamino)cinnamyloxy]milbemycin $A_4$,
13-(3-Formamidocinnamyloxy)milbemycin $A_4$,
13-(3-Propionamidocinnamyloxy)milbemycin $A_4$,
13-(3-Butyramidocinnamyloxy)milbemycin $A_4$,
13-(3-Chloroacetamidocinnamyloxy)milbemycin $A_4$,
13-(3-Methoxyacetamidocinnamyloxy)milbemycin $A_4$,
13-[3-(N-Methoxyacetyl-N-methylamino)cinnamyloxy]milbemycin $A_4$,
13-(3-Fluoroacetamidocinnamyloxy)milbemycin $A_4$,
13-(3-Difluoroacetamidocinnamyloxy)milbemycin $A_4$,
13-(3-Trifluoroacetamidocinnamyloxy)milbemycin $A_4$,
13-(3-Cyanoacetamidocinnamyloxy)milbemycin $A_4$,
13-[3-(N-Cyanoacetyl-N-methylamino)cinnamyloxy]milbemycin $A_4$,
13-[3-(2-Cyanopropionylamino)cinnamyloxy]milbemycin $A_4$,
13-(3-Methoxalylaminocinnamyloxy)milbemycin $A_4$,
13-(3-Pyruvoylaminocinnamyloxy)milbemycin $A_4$,
13-(3-Methoxycarbonylacetamidocinnamyloxy)milbemycin $A_4$,
13-(3-Phenylacetamidocinnamyloxy)milbemycin $A_4$,
13-(3-Phenoxyacetamidocinnamyloxy)milbemycin $A_4$,
13-(3-Acryloylaminocinnamyloxy)milbemycin $A_4$,
13-(3-Methacryloylaminocinnamyloxy)milbemycin $A_4$,
13-(3-Tetrolylaminocinnamyloxy)milbemycin $A_4$,
13-[3-(Cinnamoylamino)cinnamyloxy)milbemycin $A_4$,
13-(3-Cyclopropylcarbonylaminocinnamyloxy)milbemycin $A_4$,
13-(3-Cyclobutylcarbonylaminocinnamyloxy)milbemycin $A_4$,
13-(3-Cyclopentylcarbonylaminocinnamyloxy)milbemycin $A_4$,
13-(3-Cyclohexanecarbonylaminocinnamyloxy)milbemycin $A_4$,
13-(3-Benzoylaminocinnamyloxy)milbemycin $A_4$,
13-[3-(p-Toluoylamino)cinnamyloxy]milbemycin $A_4$,
13-[3-(p-Anisoylamino)cinnamyloxy]milbemycin $A_4$,
13-[3-(4-Fluorobenzoylamino)cinnamyloxy]milbemycin $A_4$,
13-[3-(4-Chlorobenzoylamino)cinnamyloxy]milbemycin $A_4$,
13-[3-(4-Aminobenzoylamino)cinnamyloxy]milbemycin $A_4$,
13-[3-(4-Acetamidobenzoylamino)cinnamyloxy]milbemycin $A_4$,
13-[3-(4-Cyanobenzoylamino)cinnamyloxy)milbemycin $A_4$,
13-[3-(4-Methoxycarbonylbenzoylamino)cinnamyloxy]milbemycin $A_4$,
13-[3-(Glycylamino)cinnamyloxy]milbemycin $A_4$,
13-[3-(N-Acetylglycylamino)cinnamyloxy]milbemycin $A_4$,
13-[3-(N-Ethoxycarbonylglycylamino)cinnamyloxy]milbemycin $A_4$,
13-{3-[(3-Methylureido)acetamido]cinnamyloxy}milbemycin $A_4$,
13-[3-(3-Oxo-1,2,4-triazolo[4,3-a]pyridin-2-ylcarbonylamino]cinnamyloxy]milbemycin $A_4$,
13-(3-Methoxycarbonylaminocinnamyloxy)milbemycin $A_4$,
13-{3-[(N-Methoxycarbonyl)methylamino]cinnamyloxy}milbemycin $A_4$,
13-(3-Methoxycarbonylaminomethylcinnamyloxy)milbemycin $A_4$,
13-(3-Ethoxycarbonylaminocinnamyloxy)milbemycin $A_4$,

13-{3-[(N-Ethoxycarbonyl)methylamino]cinnamyloxy}milbemycin $A_4$,
13-(2-Ethoxycarbonylaminocinnamyloxy)milbemycin $A_4$,
13-(4-Ethoxycarbonylaminocinnamyloxy)milbemycin $A_4$,
13-(3-Ethoxycarbonylaminocinnamyloxy)milbemycin D,
13-Deoxy-13-(3-ethoxycarbonylaminocinnamyloxy)-22,23-dihydroavermectin $B_{1a}$ aglycone
13-(3-propoxycarbonylaminocinnamyloxy)milbemycin $A_4$,
13-(3-Isopropoxycarbonylaminocinnamyloxy)milbemycin $A_4$,
13-(3-Butoxycarbonylaminocinnamyloxy)milbemycin $A_4$,
13-(3-Cyclopropyloxycarbonylaminocinnamyloxy)milbemycin $A_4$,
13-(3-(N-Cyclopropylcarbonyl-N-methylamino)cinnamyloxy]milbemycin $A_4$,
13-(3-Cyclobutyloxycarbonylaminocinnamyloxy)milbemycin $A_4$,
13-{3-(N-Cyclobutylcarbonyl-N-methylamino)cinnamyloxy}milbemycin $A_4$,
13-(3-Cyclopentyloxycarbonylaminocinnamyloxy)milbemycin $A_4$,
13-(3-yclohexyloxycarbonylaminocinnamyloxy)milbemycin $A_4$,
13-(3-Vinyloxycarbonylaminocinnamyloxy)milbemycin $A_4$,
13-(3-Allyloxycarbonylaminocinnamyloxy)milbemycin $A_4$,
13-(3-Cyclohexyloxycarbonylaminocinnamyloxy)milbemycin $A_4$,
13-(3-Benzyloxycarbonylaminocinnamyloxy)milbemycin $A_4$,
13-[3-(4-Nitrobenzyloxycarbonylamino)cinnamyloxy]milbemycin $A_4$,
13-[3-(4-Methoxybenzyloxycarbonylamino)cinnamyloxy]milbemycin $A_4$,
13-(3-Methoxycarbonylaminomethylcinnamyloxy)milbemycin $A_4$,
13-(3-Ethoxycarbonylaminomethylcinnamyloxy)milbemycin $A_4$,
13-(3-Ethylthiocarbonylaminocinnamyloxy)milbemycin $A_4$,
13-(3-Ethylthiothiocarbonylaminocinnamyloxy)milbemycin $A_4$,
13-(3-Methanesulphonylaminocinnamyloxy)milbemycin $A_4$,
13-{3-[(N-Methanesulphonyl)methylamino]cinnamyloxy}milbemycin $A_4$,
13-(3-Ethanesulphonylaminocinnamyloxy)milbemycin $A_4$,
13-(3-Propanesulphonylaminocinnamyloxy)milbemycin $A_4$,
13-(3-Cyanomethanesulphonylaminocinnamyloxy)milbemycin $A_4$,
13-(3-Benzenesulphonylaminocinnamyloxy)milbemycin $A_4$,
13-[3-(4-Methylbenzenesulphonylamino)cinnamyloxy]milbemycin $A_4$,
13-[3-(4-Methoxybenzenesulphonylamino)cinnamyloxy]milbemycin $A_4$,
13-(3-Benzenesulphinylaminocinnamyloxy)milbemycin $A_4$,
13-[3-(3-Methylureido)cinnamyloxy]milbemycin $A_4$,
13-[2-(3-Methylureido)cinnamyloxy]milbemycin $A_4$,
13-[4-(3-Methylureido)cinnamyloxy]milbemycin $A_4$,
13-[3-(3-Methylureido)cinnamyloxy]milbemycin $A_4$ 5-oxime,
13-[3-(3-Methylureido)cinnamyloxy]milbemycin D,
13-Deoxy-13-[3-(3-methylureido)cinnamyloxy]-22,23-dihydroavermectin $B_{1a}$ aglycone
13-[3-(3-Chloromethylureido)cinnamyloxy]milbemycin $A_4$,
13-(3-Ureidocinnamyloxy)milbemycin $A_4$,
13-[3-(1,3-Dimethylureido)cinnamyloxy]milbemycin $A_4$,
13-[3-(1,3,3-Trimethylureido)cinnamyloxy]milbemycin $A_4$,
13-[3-(3,3-Dimethylureido)cinnamyloxy]milbemycin $A_4$,
13-[3-(3-Ethylureido)cinnamyloxy]milbemycin $A_4$,
13-[3-(3-Methoxycarbonylmethylureido)cinnamyloxy]milbemycin $A_4$,
13-{3-[3-(2-Chloroethyl)ureido]cinnamyloxy}milbemycin $A_4$,
13-{3-[3-(2-Hydroxyethyl)ureido]cinnamyloxy}milbemycin $A_4$,
13-{3-[3-(2-Mercaptoethyl)ureido]cinnamyloxy}milbemycin $A_4$,
13-[3-(3-Propylureido)cinnamyloxy]milbemycin $A_4$,
13-[3-(3-Isopropylureido)cinnamyloxy]milbemycin $A_4$,
13-[3-(3-Butylureido)cinnamyloxy]milbemycin $A_4$,
13-[3-(3-Cyclopropylureido)cinnamyloxy]milbemycin $A_4$,
13-[3-(3-Cyclopropyl-1-methylureido)cinnamyloxy]milbemycin $A_4$,
13-[3-(3-Cyclobutylureido)cinnamyloxy]milbemycin $A_4$,
13-[3-(3-Cyclobutyl-1-methylureido)cinnamyloxy]milbemycin $A_4$,
13-[3-(3-Cyclohexylureido)cinnamyloxy]milbemycin $A_4$,
13-[3-(3-Allylureido)cinnamyloxy]milbemycin $A_4$,

13-[3-(3-Phenylureido)cinnamyloxy]milbemycin $A_4$,
13-[3-(3-Phenylureido)cinnamyloxy]milbemycin D,
13-Depxy-13-[3-(3-phenylureido)cinnamyloxy]-22,23-dihydroavermectin $B_{1a}$ aglycone
13-{3-[3-(4-Toluyl)ureido]cinnamyloxy}milbemycin $A_4$,
13-{3-[3-(4-Fluorophenyl)ureido]cinnamyloxy]milbemycin $A_4$,
13-{3-[3-(3-Fluorophenyl)ureido]cinnamyloxy)milbemycin $A_4$,
13-{3-[3-(2-Fluorophenyl)ureido]cinnamyloxy}milbemycin $A_4$,
13-{3-[3-(4-Chlorophenyl)ureido]cinnamyloxy}milbemycin $A_4$,
13-{3-[3-(4-Nitrophenyl)ureido]cinnamyloxy}milbemycin $A_4$,
13-{3-[3-(4-Cyanophenyl)ureido]cinnamyloxy}milbemycin $A_4$,
13-{3-[3-(4-Methoxyphenyl)ureido]cinnamyloxy}milbemycin $A_4$,
13-{3-[3-(4-Aminophenyl)ureido]cinnamyloxy}milbemycin $A_4$,
13-{3-[3-(4-Acetamidophenyl)ureido]cinnamyloxy}milbemycin $A_4$,
13-{3-[3-(1-Naphthyl)ureido]cinnamyloxy}milbemycin $A_4$,
13-[3-(3-Propionylureido)cinnamyloxy]milbemycin $A_4$,
13-[3-(3-Benzoylureido)cinnamyloxy]milbemycin $A_4$,
13-[3-(3-Methanesulphonylureido)cinnamyloxy]milbemycin $A_4$,
13-[3-(3-Methylureidomethyl)cinnamyloxy]milbemycin $A_4$,
13-[3-(3-Phenylureidomethyl)cinnamyloxy]milbemycin $A_4$,
13-[3-(3-Methylthioureido)cinnamyloxy]milbemycin $A_4$,
13-[3-(3-Methylthioureido)cinnamyloxy]milbemycin D,
13-Deoxy-13-[3-(3-methylthioureido)cinnamyloxy]-22,23-dihydroavermectin $B_{1a}$ aglycone
13-[3-(1,3-Dimethylthioureido)cinnamyloxy]milbemycin $A_4$,
13-[3-(3-Ethylthioureido)cinnamyloxy]milbemycin $A_4$,
13-[3-(3-Propylthioureido)cinnamyloxy]milbemycin $A_4$,
13-[3-(3-Isopropylthioureido)cinnamyloxy]milbemycin $A_4$,
13-[3-(3-Allylthioureido)cinnamyloxy]milbemycin $A_4$,
13-[3-(3-Phenylthioureido)cinnamyloxy]milbemycin $A_4$,
13-[3-(Formimidoylamino)cinnamyloxy]milbemycin $A_4$,
13-[3-(Acetimidoylamino)cinnamyloxy]milbemycin $A_4$,
13-[3-(Benzimidoylamino)cinnamyloxy]milbemycin $A_4$,
13-[3-(Carbazoylamino)cinnamyloxy]milbemycin $A_4$,
13-[3-(3,3-Dimethylcarbazoylamino)cinnamyloxy]milbemycin $A_4$,
13-{3-[N-(3,3-Dimethylcarbazoyl)methylamino]cinnamyloxy}milbemycin $A_4$,
13-[3-(3-Phenylcarbazoylamino)cinnamyloxy]milbemycin $A_4$,
13-[3-(3-Acetylcarbazoylamino)cinnamyloxy]milbemycin $A_4$,
13-[3-(3-Benzoylcarbazoylamino)cinnamyloxy]milbemycin $A_4$,
13-{3-[3-(Hexahydro-1<u>H</u>-azepin-1-yl)ureido]cinnamyloxy}milbemycin $A_4$,
13-{3-[3-(Methoxycarbonyl)guanidino]cinnamyloxy}milbemycin $A_4$, and
13-{3-[2,3-Bis(methoxycarbonyl)guanidino]cinnamyloxy}milbemycin $A_4$,

Of the compounds listed above, the preferred compounds are:

13-(3-Aminocinnamyloxy)milbemycin $A_4$
13-(2-Aminocinnamyloxy)milbemycin $A_4$
13-(3-Acetamidocinnamyloxy)milbemycin $A_4$
13-[3-(3-Methylthioureido)cinnamyloxy]milbemycin $A_4$
13-(3-Cyanoacetamidocinnamyloxy)milbemycin $A_4$
13-(3-Methanesulphonylaminocinnamyloxy)milbemycin $A_4$
13-[3-<u>N</u>-(Methanesulphonyl)methylaminocinnamyloxy]milbemycin $A_4$
13-(3-Methylaminocinnamyloxy)milbemycin $A_4$ and
13-[3-(3,3-Dimethylcarbazoylamino)cinnamyloxy]milbemycin $A_4$,

of which the most preferred compounds are:

13-(3-Cyanoacetamidocinnamyloxy)milbemycin $A_4$
13-(3-Methanesulphonylaminocinnamyloxy)milbemycin $A_4$ and
13-[3-<u>N</u>-(Methanesulphonyl)methylaminocinnamyloxy]milbemycin $A_4$.

Also preferred are salts, where available of the above compounds.

The compounds of the present invention may be prepared by a variety of processes known in the art for the preparation of compounds of this type. In general terms a suitable preparative procedure comprises subjecting a compound of formula (II) in either order to steps (a) and (b):

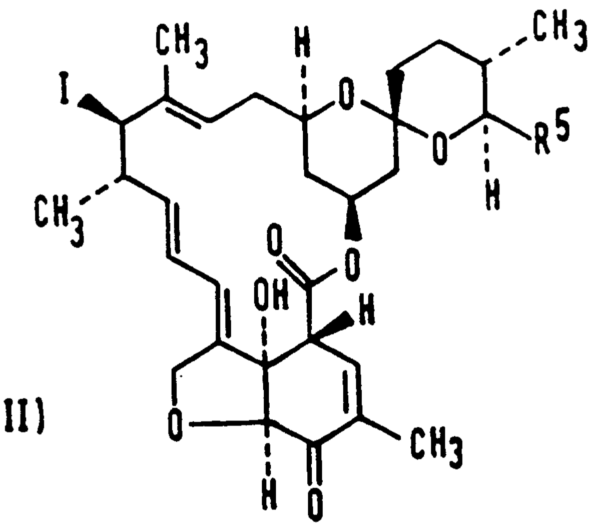

(II)

in which $R^5$ is as defined above,

(a) reaction with an alcohol of formula (IIa):

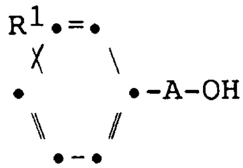

(IIa)

in which $R^1$ is as defined above; and

(b) reaction either

($b^i$) with a reducing agent to reduce the oxygen atom at the 5-position to a hydroxy group, followed, if desired by reaction with an acylating agent, to give a compound of formula (I) in which X represents an alkanoyloxy group which has from 1 to 5 carbon atoms, and which is unsubstituted or has at least one substituent selected from substituents (d); or

($b^{ii}$) with hydroxylamine or with a salt thereof, to give a compound of formula (I) in which X represents a hydroxyimino group;

and then, if required, subjecting the product to one or both of steps (c) and (d):

(c) converting a group represented by $R^1$ to any other group so represented; and

(d) salifying or esterifying the product.

In the above process, step (a) may be carried out before step (b), or step (b) may be carried out before step (a); we prefer that step (a) should be carried out before step (b).

In more detail, in this preferred embodiment, the compounds of formula (I) of the present invention can be prepared from a 13-iodo- milbemycin of formula (II) as shown in the following Reaction Scheme A:

Reaction Scheme A:

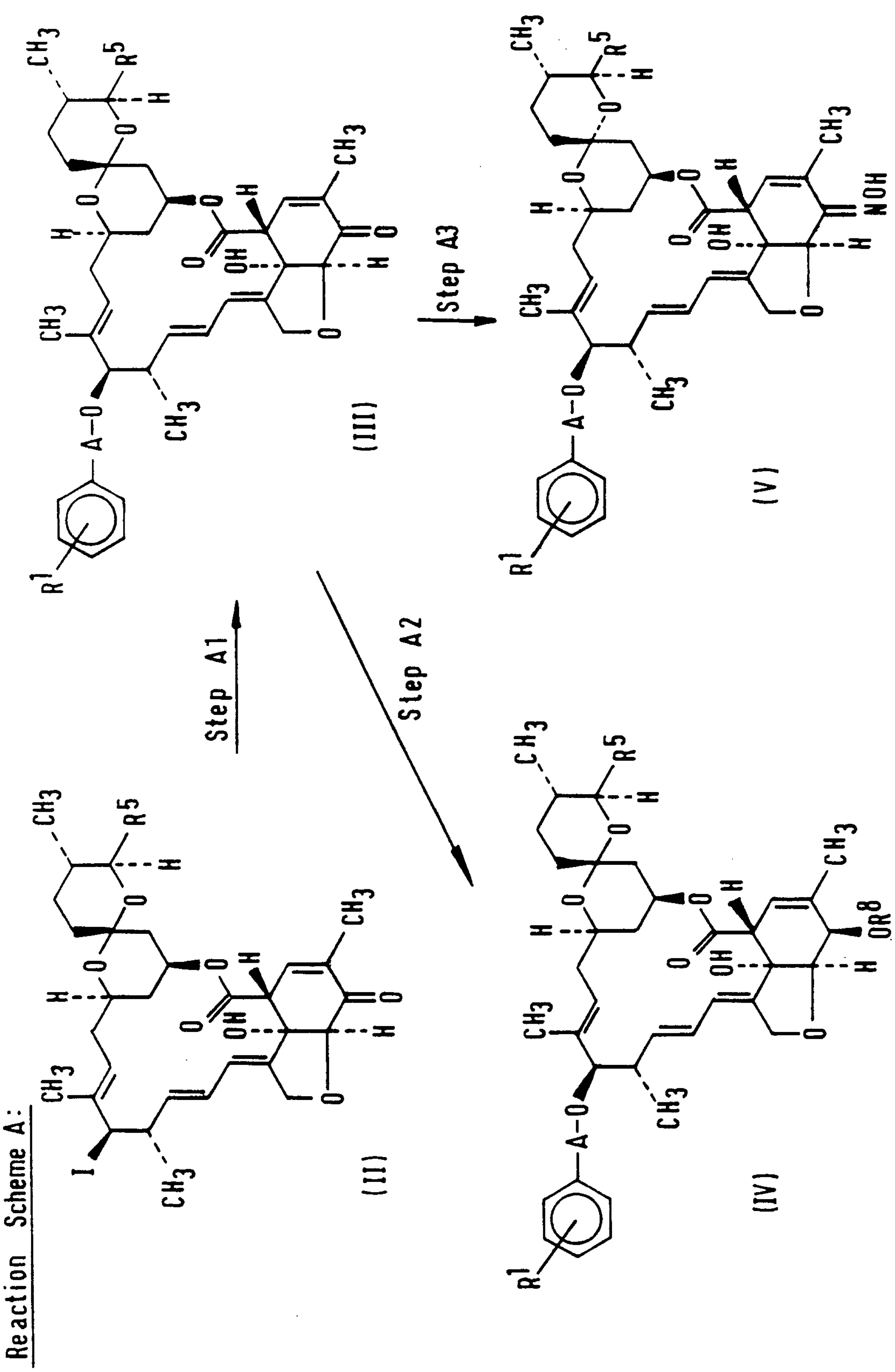


(II) (III) (IV) (V)

In the above formulae, $R^1$, $R^5$ and A are as defined above and $R^8$ represents a hydrogen atom or a $C_1$ - $C_5$ alkanoyl group or substituted $C_1$ - $C_5$, preferably $C_2$ - $C_5$, alkanoyl group having at least one substituent selected from substituents (d), defined above (i.e. the alkanoyl groups defined above for the alkanoyloxy groups of X).

In Step A1, a compound of formula (III) is prepared by reacting a compound of formula (II) with a cinnamyl alcohol or derivative thereof of formula (IIa) in the. presence of a catalyst. Any catalyst capable of catalysing such etherification reactions, as are well known in the art, may equally be employed in this reaction, without any particular restriction. Examples of suitable catalysts include oxides and salts of mercury or silver, preferably a silver compound such as silver oxide, silver perchlorate or silver trifluoromethanesulphonate, or a mercury compound such as mercury oxide, mercury iodide, mercury bromide or mercury trifluoromethanesulphonate.

In certain cases, the reaction may be accelerated by addition of an acid-binding agent. There is no particular limitation on the nature of such an acid-binding agent, provided that it has no adverse effect on the reaction, but 2,6-lutidine and calcium carbonate are preferred examples.

The reaction is normally and preferably effected in the presence of a solvent. There is no particular limitation on the nature of the solvent employed in the reaction, provided that it has no adverse effect on the reaction and that it is capable of solubilizing the starting compound, at least to some extent. Examples of suitable solvents include: aromatic hydrocarbons, such as benzene, toluene or xylene; halogenated hydrocarbons, especially halogenated aliphatic hydrocarbons, such as methylene chloride, 1,2-dichloroethane or chloroform; esters, such as ethyl acetate or propyl acetate; ethers, such as diethyl ether, tetrahydrofuran, dioxane or dimethoxyethane; amides, such as dimethylformamide, dimethylacetamide or hexamethylphosphoric triamide; and sulphoxides, such as dimethyl sulphoxide.

The reaction can take place over a wide range of temperatures, and the precise reaction temperature is not critical to the invention. In general, we find it convenient to carry out the reaction at a temperature from -10°C to 100°C, preferably from 0°C to 50°C. The time required for the reaction may also vary widely, depending on many factors, notably the reaction temperature and the nature of the reagents and of the solvent. However, provided that the reaction is effected under the preferred conditions outlined above, a period of from 1 hour to 2 days will usually suffice.

After completion of the reaction, the reaction product may be recovered from the reaction mixture by conventional means. For example, the reaction mixture may be diluted with a water-immiscible organic solvent, after which, if necessary, the insoluble materials are removed by filtration. The filtrate may then be washed, for example successively with an aqueous solution of potassium iodide, an acid and water, and the solvent may be removed by distillation to afford the desired product. The product may, if required, be further purified by such conventional techniques as recrystallization or the various chromatography techniques, notably column chromatography.

In Step A2, a compound of formula (IV) is prepared by reducing the carbonyl group at the 5-position of the compound of formula (III) to a hydroxy group, which, if required, may then be subjected to acylation to give a compound of formula (IV) in which $R^8$ represents an alkanoyl group. There is no particular limitation on the nature of the reducing agent to be used in this reduction, provided that it can reduce the carbonyl group and has no adverse effect on the other functional groups in the compound of formula (III). Such reducing agents include, for example, hydride-producing agents, such as sodium borohydride or diborane, preferably sodium borohydride.

The reaction is normally and preferably effected in the presence of a solvent, and there is equally no particular limitation on the nature of the solvent, provided that it has no adverse effect on the reaction, but a lower alcohol (such as methanol, ethanol or propanol) is preferably used, especially when sodium borohydride is employed as the reducing agent.

The reaction can take place over a wide range of temperatures, and the precise reaction temperature is not critical to the invention. In general, we find it convenient to carry out the reaction at a temperature from 0°C to 50°C. The time required for the reaction may also vary widely, depending on many factors, notably the reaction temperature and the nature of the reagents. However, provided that the reaction is effected under the preferred conditions outlined above, a period of from 5 minutes to 2 hours will usually suffice.

After completion of the reaction, the reaction product can be recovered easily from the reaction mixture by conventional means. For example, the reaction mixture may be diluted with a water-immiscible organic solvent and washed with water, after which the solvent may be removed by distillation to afford the desired product. The product may, if required, be further purified by such conventional techniques as recrystallization or the various chromatography techniques, notably column chromatography.

The reduction product thus prepared may, if required, be acylated to produce a compound in which $R^8$ is an alkanoyl group. This may take place in an inert solvent, using as the acylating agent an acid corresponding to the alkanoyl group which it is desired to introduce or using a reactive derivative of such an acid. The reaction can be carried out using conventional esterification techniques. Examples of suitable active derivatives of the acid include any of those commonly used for esterification such as acid halides (e.g. an acid chloride or acid bromide), acid anhydrides, mixed acid anhydrides, reactive esters (e.g. the N-hydroxybenztriazole ester) and reactive amides (e.g. the imidazolide).

Where the acid itself is employed, a dehydrating agent (such as dicyclohexylcarbodiimide, p-toluenesulphonic acid or sulphuric acid) is preferably also present. Where a reactive derivative of an acid is employed, an acid-binding agent is preferably also employed. There is no particular limitation on the nature of the acid-binding agent to be used, provided that it has the ability to eliminate an acid, for example, an organic amine such as triethylamine, N,N-diethylaniline, pyridine, 4-dimethylaminopyridine or 1,8-diazabicyclo[5.4.0]undecene-7, may be used.

There is no particular restriction on the nature of the solvent to be employed, provided that it has no adverse effect on the reaction or on the reagents involved and that it is capable of dissolving the reagents, at least to some extent. Examples of suitable solvents include: hydrocarbons, which may be aliphatic, aromatic or cycloaliphatic, such as hexane, benzene, toluene or xylene; halogenated hydrocarbons, especially halogenated aliphatic hydrocarbons, such as methylene chloride, 1,2-dichloroethane or chloroform; esters, such as ethyl acetate or propyl acetate; and ethers, such as diethyl ether, tetrahydrofuran, dioxane or dimethoxyethane.

After completion of the reaction, the reaction product can easily be recovered from the reaction mixture by conventional means. For example, the reaction mixture may be diluted with a water-immiscible organic solvent and washed successively with an acid, an alkali and water, after which the solvent may be removed by distillation to afford the desired product. The product may, if required, be further purified by such conventional techniques as recrystallization or the various chromatography techniques, notably column chromatography.

In Step A3 a compound of formula (V) is prepared by oximation at the 5-position of the compound of formula (III) with hydroxylamine or with a salt thereof (e.g. a salt with a mineral acid such as hydrochloric acid, nitric acid or sulphuric acid).

The reaction is usually carried out in an inert solvent, the nature of which is not critical, provided that it has no adverse effect on the reaction or on the reagents involved and that it is capable of dissolving the reagents, at least to some extent. Examples of suitable solvents include: alcohols, such as methanol or ethanol; ethers, such as tetrahydrofuran or dioxane; aliphatic acids, such as acetic acid; or a mixture of water with any one or more of these solvents.

The reaction can take place over a wide range of temperatures, and the precise reaction temperature is not critical to the invention. In general, we find it convenient to carry out the reaction at a temperature from 10°C to 80°C. The time required for the reaction may also vary widely, depending on many factors, notably the reaction temperature and the nature of the reagents. However, provided that the reaction is effected under the preferred conditions outlined above, a period of from 1 to 24 hours will usually suffice.

After completion of the reaction, the reaction product can easily be recovered from the reaction mixture by conventional means. For example, the reaction mixture may be diluted with a water-immiscible organic solvent and washed with water, after which the solvent may be removed by distillation to afford the desired product. The product may, if required, be further purified by such conventional techniques as recrystallization or the various chromatography techniques, notably column chromatography.

As an alternative to the above, the reaction of step A2 or A3 may be carried out first, to prepare a compound in which X represents a hydroxy, alkanoyloxy or hydroxyimino group, after which the iodine atom at the 13 position is replaced by the optionally substituted cinnamyloxy group.

The compound of formula (IV) wherein $R^1$ is a substituted amino group can be prepared as illustrated in the following Reaction Scheme B:

Reaction Scheme B:

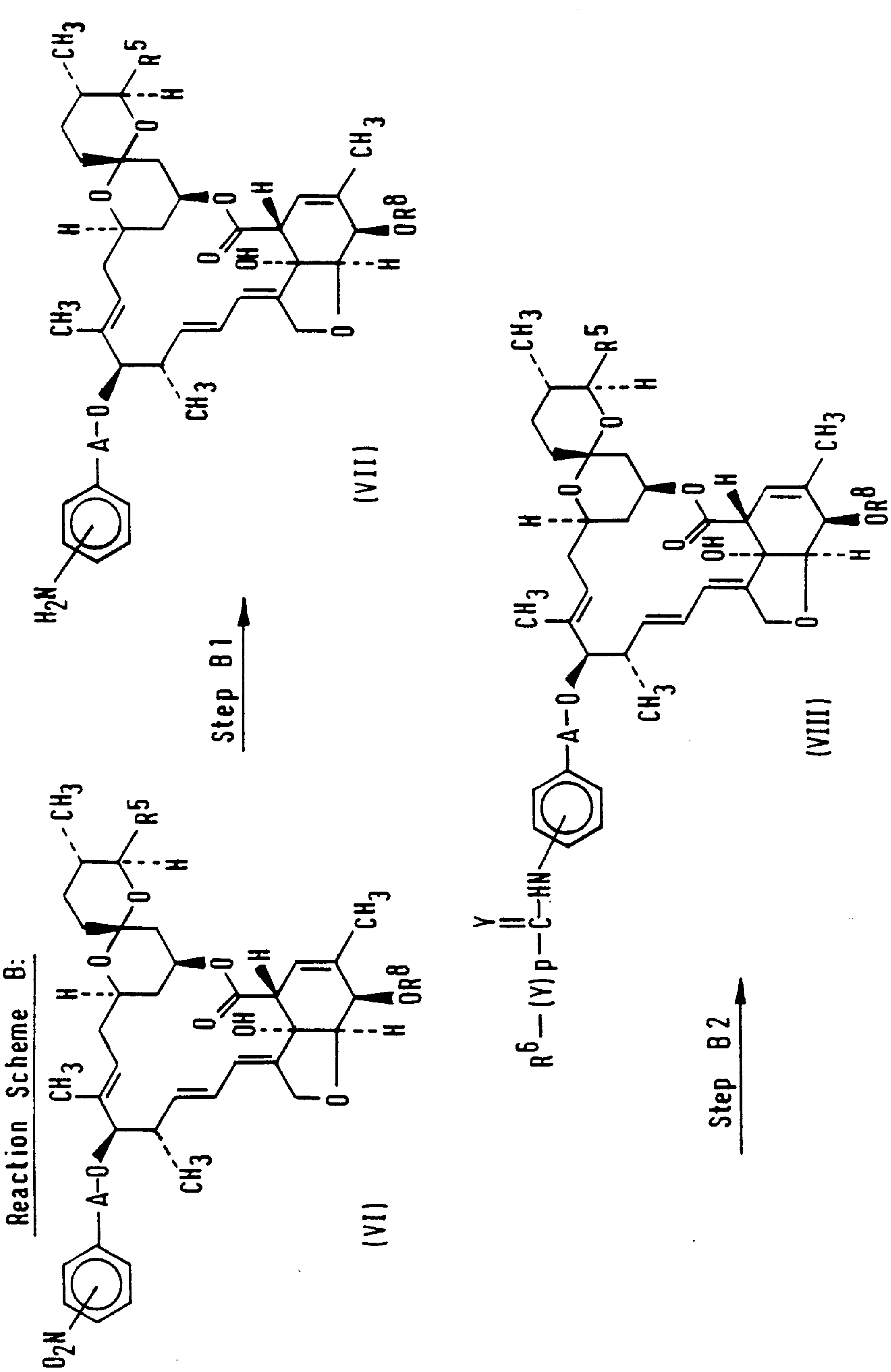


(VI)

(VII)

(VIII)

In the above formulae, $R^5$, $R^6$ and $R^8$ are as defined above, Y represents an oxygen atom, a sulphur atom or an imino group, and the two symbols Y may be the same or different, and p represents 0 or 1.

In Step B1 a compound of formula (VII) is prepared by reducing the nitro group of a compound of formula (VI) to give an amino group. This may by effected by a conventional reducing method for reducing a nitro group to an amino group. One such method is catalytic reduction using a precious metal catalyst.

Examples of catalysts which are preferably employed include palladium-on-carbon, palladium-on-barium sulphate and platinum oxide.

The reaction is normally and preferably effected in the presence of a solvent, and there is no particular restriction on the nature of the solvent to be employed, provided that it has no adverse effect on the reaction or on the reagents involved. Examples of suitable solvents include: alcohols, such as methanol or ethanol; ethers, such as tetrahydrofuran or dioxane; and esters, such as ethyl acetate.

The reaction can take place over a wide range of temperatures, and the precise reaction temperature is not critical to the invention. In general, we find it convenient to carry out the reaction at a temperature from 10 °C to 80 °C. The time required for the reaction may also vary widely, depending on many factors, notably the reaction temperature and the nature of the reagents. However, provided that the reaction is effected under the preferred conditions outlined above, a period of from 10 minutes to 5 hours will usually suffice.

An alternative preferred reducing method is reduction with zinc powder in acetic acid. This reaction is preferably carried out at a temperature ranging from 0 °C to room temperature, and the reaction time is usually in the range of from 10 minutes to 2 hours.

After completion of the reaction, the reaction product can easily be recovered from the reaction mixture by conventional means. For example, the reaction mixture may be diluted with a water-immiscible organic solvent, and the insoluble materials, if necessary, removed by filtration. The filtrate may then be washed with water, and the solvent may be removed by distillation to afford the desired product. The product may, if required, be further purified by such conventional techniques as recrystallization or the various chromatography techniques, notably column chromatography.

In Step B2 a compound of formula (VIII) is prepared by reacting the compound of formula (VII) with a reagent that is reactive with the amino group, to introduce the group of formula $R^6$-$(Y)_p$-C(=Y)-NH-.

The nature of the reagent to be employed will, of course, be dictated by the nature of the group which it is desired to introduce. However, in general, it may be a reactive derivative of a carboxylic acid of the type commonly used as an acylating agent such as an acid halide, an acid anhydride, a mixed acid anhydride, a reactive ester or a reactive amide. Alternatively, it may be: a chloroformate, such as methyl chloroformate or benzyl chloroformate; a thiochloroformate, such as ethyl chlorothioformate; a sulphonyl chloride, such as methanesulphonyl chloride or benzenesulphonyl chloride; an isocyanate; a thioisocyanate; or an imino ether. Alternatively, a carboxylic acid may be used as such, provided that it is activated, for example with dicyclohexylcarbodiimide.

When a halide, such as an acid halide, is employed as the reagent, it is usually preferred to carry out the reaction in the presence of an organic base, such as triethylamine, N,N-diethylaniline, pyridine, 4-dimethylaminopyridine or 1,8-diazabicyclo[5.4.0]undecene, as an acid-binding agent.

The reaction can take place over a wide range of temperatures, and the precise reaction temperature is not critical to the invention. In general, we find it convenient to carry out the reaction at a temperature from 0 °C to 80 °C, preferably from 0 °C to room temperature. The time required for the reaction may also vary widely, depending on many factors, notably the reaction temperature and the nature of the reagents. However, provided that the reaction is effected under the preferred conditions outlined above, a period of from 10 minutes to 10 hours will usually suffice.

After completion of the reaction, the reaction product can easily be recovered from the reaction mixture by conventional means. For example, the reaction mixture may be diluted with a water-immiscible organic solvent, and the insoluble materials may then be removed, if required, by filtration and washed with water, after which the solvent may be removed by distillation to afford the desired product. The product may, if required, be further purified by such conventional techniques as recrystallization or the various chromatography techniques, notably column chromatography.

The compound of formula (II), which is used as the starting material in the above sequences of reactions can advantageously be synthesized from 13-hydroxy-5-oxomilbemycin, which is represented by the general formula (IX), as illustrated in the following Reaction Scheme C:

Reaction Scheme C:

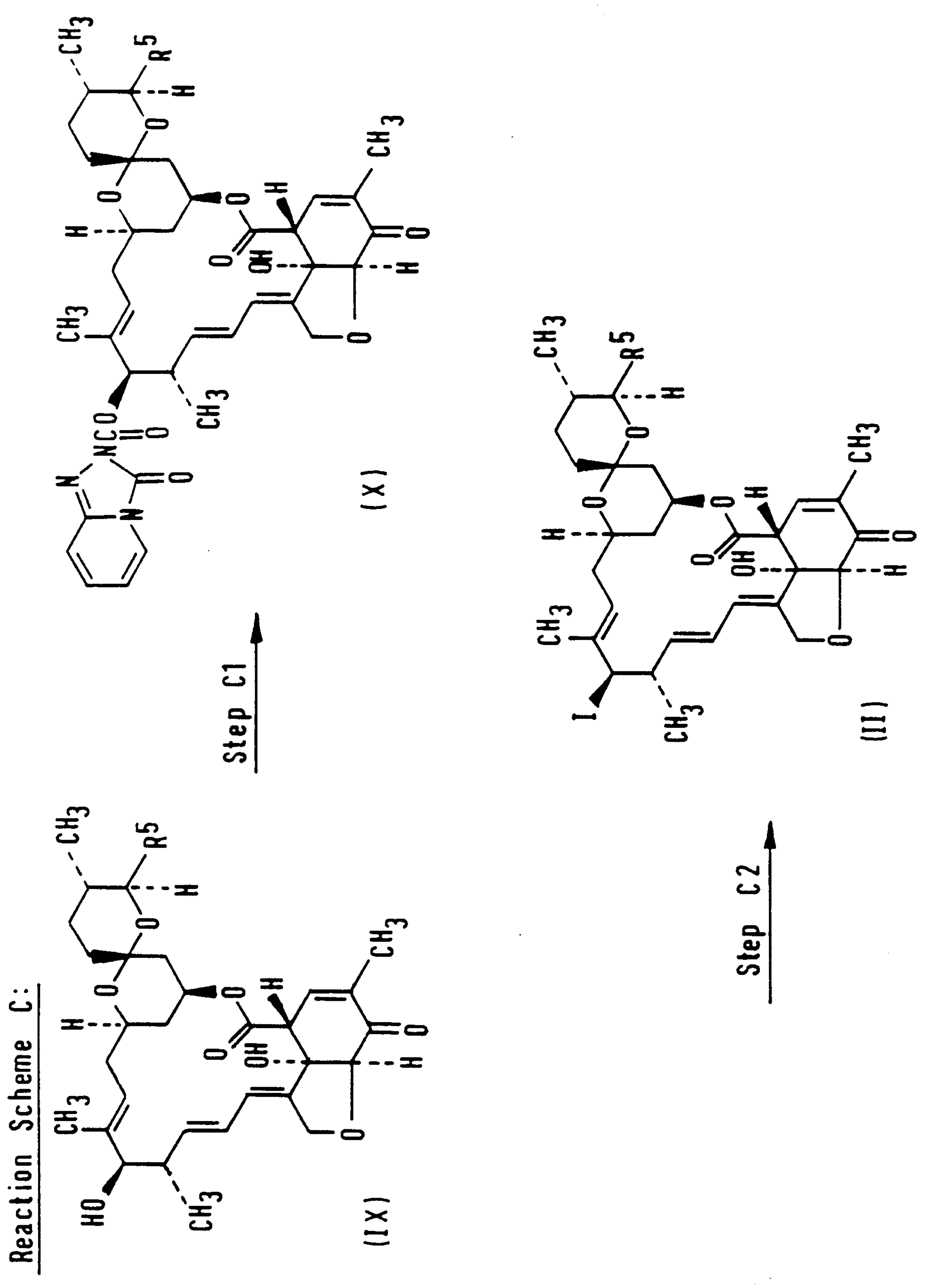


In the above formulae, $R^5$ is as defined above.

In Step C1 a compound of formula (X) is prepared by reacting the compound of formula (IX) with 2-chloroformyl-1,2,4-triazolo[4.3a]pyridin-3-one in the presence of an acid-binding agent.

There is no particular limitation on the nature of the acid-binding agent to be employed provided that it has the ability to eliminate any acid produced. For example, an organic amine, such as triethylamine, N,N-diethylaniline, pyridine, 4-dimethylaminopyridine or 1,8-diazabicyclo[5.4.0]undecene, may be used.

The reaction is also preferably effected in the presence of an inert solvent, the nature of which is not critical, provided that it has no adverse effect on the reaction or on the reagents involved. Examples of suitable solvents include: hydrocarbons, which may be aliphatic, aromatic or cycloaliphatic, such as hexane, benzene, toluene or xylene; halogenated hydrocarbons, especially halogenated aliphatic hydrocarbons, such as methylene chloride, 1,2-dichloroethane or chloroform; esters, such as ethyl acetate or propyl acetate; and ethers, such as diethyl ether, tetrahydrofuran, dioxane or dimethoxyethane.

The reaction can take place over a wide range of temperatures, and the precise reaction temperature is not critical to the invention. In general, we find it convenient to carry out the reaction at a temperature from 0°C to 50°C. The time required for the reaction may also vary widely, depending on many factors, notably the reaction temperature and the nature of the reagents. However, provided that the reaction is effected under the preferred conditions outlined above, a period of from 5 minutes to 2 hours will usually suffice.

After completion of the reaction, the reaction product can easily be recovered from the reaction mixture by conventional means. For example, the reaction mixture may be diluted with a water-immiscible organic solvent, the insoluble materials may then be removed, if required, by filtration and washed, for example successively with an aqueous solution of potassium iodide, an acid and water, after which the solvent may be removed by distillation to afford the desired product.

In Step C2 13-iodomilbemycin, which is represented by formula (II), is prepared by reacting the compound of formula (X) with zinc iodide.

This reaction is usually carried out in a solvent. There is no particular restriction on the nature of the solvent to be employed, provided that it has no adverse effect on the reaction or on the reagents involved and that it is capable of dissolving the reagents, at least to some extent. Examples of suitable solvents include: hydrocarbons, which may be aliphatic, aromatic or cycloaliphatic, such as hexane, benzene, toluene or xylene; halogenated hydrocarbons, especially halogenated aliphatic hydrocarbons, such as methylene chloride, 1,2-dichloroethane or chloroform; esters, such as ethyl acetate or propyl acetate; and ethers, such as diethyl ether, tetrahydrofuran, dioxane or dimethoxyethane.

The reaction can take place over a wide range of temperatures, and the precise reaction temperature is not critical to the invention. In general, we find it convenient to carry out the reaction at a temperature from 0°C to room temperature. The time required for the reaction may also vary widely, depending on many factors, notably the reaction temperature and the nature of the reagents. However, provided that the reaction is effected under the preferred conditions outlined above, a period of from 10 minutes to 2 hours will usually suffice.

After completion of the reaction, the reaction product can easily be recovered from the reaction mixture by conventional means. For example, the insoluble materials may be removed by filtration and the filtrate washed with water, after which the solvent may be removed by distillation to afford the desired product. The product may, if required, be further purified by such conventional techniques as recrystallization or the various chromatography techniques, notably column chromatography.

The compound of formula (IX), which is, therefore, the ultimate starting material for the above sequence of reactions, can be prepared from the natural or semisynthetic milbemycins or avermectins by the method disclosed in Japanese Patent Application Kokai No. Sho 61-103884.

The milbemycins and analogueous natural products are generally obtained as mixtures at various ratios of related compounds, and they may be reacted after being separated into the various fractions or they may be used in the above reactions as mixtures, whether the natural mixture or an artificially produced mixture. Therefore, the compound used in each step of the above reactions may be either a single compound or a mixture of compounds. Accordingly, the compound of formula (I) may be prepared as a single compound or as a mixture of compounds, and, if prepared as a mixture of compounds, may be used as such or may be separated into the individual compounds prior to use.

The compounds of the invention have a strong acaricidal activity against, for example, adults, imagos and eggs of Tetranychus, Panonychus (e.g. Panonychus ulmi and Panonychus citri), Aculopa pelekassi and rust mites, which are parasitic to fruit trees, vegetables and flowers. They are also active against Ixodidae, Dermanyssidae and Sarcoptidae, which are parasitic to animals. Surprisingly, they have a strong activity even against acarids which are resistant to the known acaricides, which have recently started to become a great problem. Further, they are active against: ectoparasites, such as Oestrus, Lucilia, Hypoderma, Gautrophilus, lice and fleas, which are parasitic to animals and birds, particularly livestock and poultry; domestic insects, such as cockroaches and houseflies; and various harmful insects in agricultural and horticultural areas, such as aphids and larval Lepidoptera. They are also effective against Meloidogyne, Bursaphelenchus and Rhizoglyphus in the soil, and against insects of the orders Coleoptera, Homoptera, Heteroptera, Diptera, Thysanoptera, Orthoptera, Anoplura, Siphonaptera, Mallophage, Thysanura, Isoptera,

Psocoptera, and Hymenoptera.

The compounds of the invention equally can be used to control other plant-damaging insects, particularly insects that damage plants by eating them. The compounds can be used to protect both ornamental plants and productive plants, particularly cotton (e.g. against Soodoptera littoralis and Heliothis virescens), as well as vegetable crops (e.g. against Leptinotarsa decemlineata and Myzus persicae) and rice crops (e.g. against Chilo suppressalis and Laodelphax).

Accordingly, the compounds of the invention can be used to treat all manner of plants (as well as the seeds from which such plants are grown and the environment, whether for growth or storage, containing such plants) to protect them from insects such as those exemplified above. Such plants include cereals (e.g. maize or rice), vegetables (e.g. potatoes or soybeans), fruits and other plants (e.g. cotton).

The compounds of the invention can similarly be used to protect animals from a variety of ectoparasites, by applying the compounds to the animals or to the animals' environment, e.g. livestock housing, animal boxes, abattoirs, pasture land and other grasslands, as well as to any other places liable to be infested. The compounds may also be applied to external parts of the animals, preferably before they are infested.

Moreover, the compounds of the invention are effective against various parasitical helminths. These parasites can attack livestock, poultry and pet animals (such as pigs, sheep, goats, cows, horses, dogs, cats and fowl) and can cause grave economic damage. Among the helminths, the nematodes in particular often cause serious infection. Typical genera of nematodes which are parasitic on these animals and against which the compounds of the invention are effective include:

Haemonchus, Trichostrongylus, Ostertagia, Nematodirus, Cooperia, Ascaris, Bunostomum, Oesophagostomum, Chabertia, Trichuris, Strongylus, Trichonema, Dictyocaulus, Capillaria, Heterakis, Toxocara, Ascaridia, Oxyuris, Ancylostoma, Uncinaria, Toxascaris and Parascaris.

Certain parasitical species of the genera Nematodirus, Cooperia and Oesophagostomum attack the intestines, while certain species of the genera Haemonchus and Ostertagia parasitize the stomach, and parasites belonging to the genus Dictyocaulus are found in the lungs. Parasites belonging to the families Filariidae and Setariidae are found in internal tissues and organs, for example, the heart, the blood vessels, the subcutaneous tissues and the lymphatic vessels. The compounds of the invention are active against all of these parasites.

The compounds of the invention are also effective against parasites which infect humans. Typical of the parasites which may most commonly be found in the digestive tracts of human beings are parasites of the genera:

Ancylostoma, Necator, Ascaris, Strongyloides, Trichinella, Capillaria, Trichuris and Enterobius.

The compounds are also active against parasites of the genera Wuchereria, Brugia, Onchocerca and Loa of the family Filariidae (which are found in blood, tissues and organs other than the digestive tract and are medically important), parasites of the genus Dracunculus of the family Dracunculidae and parasites of the genera Strongyloides and Trichinella, which in a particular state may parasitize outside the intestinal tract, although they are essentially intestinal parasites.

The form of the compositions of the invention and the nature of the carriers or diluents employed in them will vary depending upon the intended use of the composition. For example, where the compounds of the invention are to be employed as anthelmintics, they are preferably administered orally, parenterally or topically and the form of composition chosen will be appropriate to the intended route of administration.

For oral administration, the composition of the invention is preferably in the form of a liquid drink comprising a non-toxic solution, suspension or dispersion of the active compound in admixture with a suspending agent (such as bentonite), a wetting agent or other diluents, preferably in water or another non-toxic solvent. The drink, in general, also contains an anti-foaming agent. The active compound would normally be present in the drink in an amount of from 0.01 to 0.5% by weight, more preferably from 0.01 to 0.1% by weight.

Compositions for oral administration may also be in the form of dry solids, preferably in unit dosage form, such as capsules, pills or tablets containing the desired amount of the active compound. These compositions may be prepared by mixing the active compound uniformly with suitable diluents, fillers, disintegrators and/or binding agents, for example starch, lactose, talc, magnesium stearate and vegetable gum. The weight and contents of the preparation will vary widely, depending upon the nature of the animal to be treated, the degree of infection, the nature of the parasite and the body weight of the animal to be treated.

The compounds may also be administered as an additive to animal feedstuffs, in which case they may be dispersed uniformly in the feedstuffs, used as a top dressing or used in the form of pellets. The content of active compound in the feedstuff is preferably from 0.0001 to 0.02%, in order to achieve the desired

anthelmintic activity.

For parenteral administration, the compound of the invention is preferably dissolved or suspended in a liquid vehicle, preferably a vegetable oil, such as peanut oil or cottonseed oil. Where the compound is a salt of a compound of formula (I), the liquid vehicle may be water or another aqueous medium. Depending upon the animal to be treated, the injection may be subcutaneous or into the proventriculus, a muscle or the trachea. Such preparations would normally contain the active compound at a concentration of from 0.05 to 50% by weight.

The compounds of the invention may also be administered topically in admixture with a suitable carrier, such as dimethyl sulphoxide or a hydrocarbon solvent. Such preparations would be applied directly to the outside of the animal by spraying (e.g. by a hand spray or in spray races), by dipping (e.g. in a plunge dip), by a pour-on solution or by manual methods (e.g. hand-dressing).

The dose of active compound may be varied, depending upon the nature of the animal to be treated, and the nature and degree of parasitic infection. However, best results for oral administration are achieved when the dose is from 0.01 to 100 mg, more preferably from 0.5 to 50 mg, per 1 kg body weight. The compound may be administered in a single dose or in divided doses for a relatively short period, such as from 1 to 5 days.

Where the composition of the invention is intended for agricultural or horticultural use, a variety of forms and formulations is possible. For example, the composition may be formulated as dusts, coarse dusts, soluble powders, microgranules, fine microgranules, wettable powders, dilute emulsions, emulsifiable concentrates, aqueous or oily suspensions, dispersions or solutions (which may be directly sprayable or for dilution), aerosols or capsules in, for example, polymeric substances. The carrier employed may be natural or synthetic and organic or inorganic; it is generally employed to assist the active compound to reach the substrate to be treated, and to make it easier to store, transport or handle the active compound. Solid, liquid and gaseous carriers may be employed, chosen from carriers well known in the art for use with compositions of this type.

Such formulations may be prepared by conventional means, e.g. by intimate mixing and/or grinding of the active ingredient(s) with the carrier or diluent, e.g. solvent, solid carrier or, optionally, surface-active agent.

Suitable solvents include: aromatic hydrocarbons, preferably the $C_8$ to $C_{12}$ fractions from petroleum distillation, such as xylene mixtures or substituted naphthalenes; esters of phthalic acid, such as dibutyl or dioctyl phthalate; aliphatic hydrocarbons, such as cyclohexane or the paraffins; alcohols and glycols or esters thereof, such as ethanol, ethylene glycol, ethylene glycol monomethyl ether or ethylene glycol monoethyl ether; ketones, such as cyclohexanone; strongly polar solvents, such as N-methyl-2-pyrrolidone, dimethyl sulphoxide or N,N-dimethylformamide; optionally epoxidized vegetable oils, such as epoxidized coconut oil or soybean oil; and water.

Solid carriers, which may be used, for example, in dusts and dispersible powders, include natural mineral fillers, such as calcite, talc, kaolin, montmorillonite or attapulgite. In order to improve the physical properties of the composition, it is also possible to add highly dispersed silicic acid or highly dispersed absorbent polymers. Suitable granulated adsorptive carriers may be porous (such as pumice, ground brick, sepiolite or bentonite) or non-porous (such as calcite or sand). A wide variety of pregranulated materials, organic or inorganic, may also be used; examples include dolomite and ground plant residues.

Surface-active agents which may be used are well known in the art and may be non-ionic, cationic or anionic agents having good emulsifying, dispersing and wetting properties. Mixtures of such agents may also be used.

Compositions may also contain stabilizers, anti-foaming agents, viscosity regulators, binders or adhesives or any combination thereof, as well as fertilizers or other active substances to achieve special effects.

Pesticidal compositions will generally contain: from 0.01 to 99%, more preferably from 0.1 to 95%, by weight of the active compound; from 1 to 99.99% of a solid or liquid additive; and from 0 to 25%, more preferably from 0.1 to 25%, of a surface-active agent. Whereas commercial products are generally sold as concentrated compositions, they are generally diluted by the end-user to a concentration of from 0.001 to 0.0001% by weight (from 10 to 1 ppm).

The invention is further illustrated by the following Examples, which illustrate the preparation of the compounds of the present invention, and the subsequent Test Examples, which illustrate the biological activity of the compounds of the invention. In the following Examples, all Nuclear Magnetic Resonance Spectra were measured at 270 MHz, unless otherwise stated.

EXAMPLE 1

13-Cinnamyloxymilbemycin $A_4$

1(a) 13-Cinnamyloxy-5-oxomilbemycin $A_4$

0.333 g of 13-iodo-5-oxomilbemycin $A_4$ was dissolved in 2.50 ml of 1,2-dichloroethane, and 0.671 g of cinnamyl alcohol and 1.000 g of silver oxide were added to the resulting solution, after which the mixture was stirred at room temperature for 30 minutes. 30 ml of ethyl acetate were added to the reaction mixture, and the insoluble materials were removed by filtration using a Celite (trade mark) filter aid. The filtrate was then washed with a 10% w/v aqueous solution of sodium thiosulphate and with water, in that order, after which it was dried over anhydrous sodium sulphate. The solvent was then removed by evaporation under reduced pressure. The resulting residue was purified by column chromatography through silica gel, eluted with a 3 : 7 by volume mixture of ethyl acetate and cyclohexane, to afford 0.239 g of the title compound.

1(b) 13-Cinnamyloxymilbemycin $A_4$

0.119 g of 13-cinnamyloxy-5-oxomilbemycin $A_4$ [prepared as described in step (a) above] was dissolved in 4.2 ml of methanol, and 0.007 g of sodium borohydride was added to the resulting solution, whilst ice-cooling. The mixture was then stirred for 30 minutes, after which 25 ml of ethyl acetate were added the reaction mixture, and the mixture was washed twice with water. It was then dried over anhydrous sodium sulphate and the solvent was removed by evaporation under reduced pressure. The residue was purified by column chromatography through silica gel, eluted with a 3 : 7 by volume mixture of ethyl acetate and cyclohexane. The isomer substituted at the 15-position was then separated by reverse phase chromatography (through ODS; eluted with 85% v/v aqueous acetonitrile) to afford 0.040 g of the title compound. "ODS" is octadecylsilane.
Mass Spectrum m/z: 672 ($M^+$ -2).
Nuclear Magnetic Resonance Spectrum ($CDC\ell_3$), $\delta$ ppm:
1.89 (3H, singlet);
3.37 (1H, doublet, J = 9.9 Hz);
4.05 (1H, doublet, J = 6.2 Hz);
6.27 (1H, multiplet);
6.57 (1H, doublet, J = 14.6 Hz).

EXAMPLE 2

13-Cinnamyloxymilbemycin $A_4$5-oxime

0.119 g of 13-cinnamyloxy-5-oxomilbemycin $A_4$ [prepared as described in Example 1(a)] was dissolved in 1.5 ml of methanol, and 0.75 ml of water, 1.5 ml of dioxan and 0.12 g of hydroxylamine hydrochloride were added to the resulting solution, after which the mixture was stirred at 40°C for 3 hours. 20 ml of ethyl acetate were added to the reaction mixture, and the mixture was washed twice with water, and then dried over anhydrous sodium sulphate. The solvent was then removed by evaporation under reduced pressure. The resulting residue was purified by column chromatography through silica gel, eluted with a 3 : 7 by volume mixture of ethyl acetate and cyclohexane. The isomer substituted at the 15-position was then separated by reverse phase chromatography (through ODS; eluted with 85% v/v aqueous acetonitrile), to afford 0.04 g of the title compound.
Mass Spectrum m/z: 687 ($M^+$, $C_{41}H_{53}NO_8$).
Nuclear Magnetic Resonance Spectrum ($CDC\ell_3$), $\delta$ ppm:
1.94 (3H, singlet);
3.36 (1H, doublet, J = 9.5 Hz);
4.67 (1H, singlet);
6.28 (1H, multiplet);
6.56 (1H, doublet, J = 15.8 Hz).

EXAMPLE 3

13-[3-(3-Aminophenyl)propoxy]milbemycin $A_4$

3(a) 13-[3-(3-Nitrophenyl)propionyloxy]-5-oxomilbemycin $A_4$

0.33 g of 13-iodo-5-oxomilbemycin $A_4$ was dissolved in 2.0 ml of 1,2-dichloroethane, and 0.45 g of 3-(3-nitrophenyl)propanol and 0.34 g of mercuric iodide were added to the resulting solution, after which the mixture was stirred at room temperature for 5 hours. At the end of this time, 10 ml of ethyl acetate were added to the reaction mixture, and insolubles were removed by filtration. The filtrate was washed with a 20% w/v aqueous solution of potassium iodide (twice), with a 10% w/v aqueous solution of sodium thiosulphate and with water, in that order, after which it was dried over anhydrous sodium sulphate. The solvent was then removed by evaporation under reduced pressure. The resulting residue was purified by column chromatography through silica gel, eluted with a 25 : 75 by volume mixture of ethyl acetate and hexane, to afford 0.29 g of the title compound.

3(b) 13-[3-(3-Nitrophenyl)propoxy]milbemycin $A_4$

0.29 g of 13-[3-(3-nitrophenyl)propionyloxy]-5-oxo-milbemycin $A_4$ [prepared as described in step (a) above] was dissolved in 2 ml of methanol, and 0.015 g of sodium borohydride was added to the resulting solution, whilst ice-cooling, after which the mixture was stirred for 20 minutes. At the end of this time, 10 ml of ethyl acetate were added to the reaction mixture, and the mixture was washed twice with water and then dried over anhydrous sodium sulphate. The solvent was then removed by evaporation under reduced pressure. The residue was purified by column chromatography through silica gel, eluted with a 1 : 1 by volume mixture of ethyl acetate and hexane to afford 0.27 g of the title compound.

3(c) 13-[3-(3-Aminophenyl)propoxy]milbemycin $A_4$

0.27 g of 13-[3-(3-nitrophenyl)propoxy]milbemycin $A_4$ [prepared as described in step (b) above] was dissolved in 5 ml of 90% v/v aqueous acetic acid, and 0.12 g of zinc powder was added to the resulting solution, whilst ice-cooling, after which the mixture was stirred for 20 minutes. At the end of this time, 20 ml of ethyl acetate were added to the reaction mixture, and insolubles were removed by filtration. The filtrate was washed with water three times and then dried over anhydrous sodium sulphate. The solvent was then removed by evaporation under reduced pressure. The resulting residue was purified by column chromatography (through ODS; eluted with 75% v/v aqueous acetonitrile), to afford 0.17 g of the title compound.

Nuclear Magnetic Resonance Spectrum ($CDCl_3$), $\delta$ ppm:

1.87 (3H, singlet);
3.20 (1H, doublet, J = 9.8 Hz);
3.96 (1H, doublet, J = 6.3 Hz).

EXAMPLES 4 TO 11

The compounds of Examples 4 to 11 were prepared using the same procedures as described in Example 3.

Example 4

13-[3-(2-Aminophenyl)propoxy]milbemycin $A_4$

Nuclear Magnetic Resonance Spectrum ($CDCl_3$), $\delta$ ppm:

1.87 (3H, singlet);
3.25 (1H, doublet, J = 10.3 Hz);
3.96 (1H, doublet, J = 6.3 Hz).

Example 5

13-[3-(4-Aminophenyl)propoxy]milbemycin $A_4$

Nuclear Magnetic Resonance Spectrum ($CDCl_3$), δ ppm:
1.87 (3H, singlet);
3.19 (1H, doublet, J = 9.7 Hz);
3.96 (1H, doublet, J = 6.3 Hz).

Example 6

13-[3-(4-Aminophenyl)cinnamyloxy]milbemycin $A_4$

Nuclear Magnetic Resonance Spectrum ($CDCl_3$), δ ppm:
1.87 (3H, singlet);
3.34 (1H, doublet, J = 10.2 Hz);
3.95 (1H, doublet, J = 6.3 Hz);
6.06 (1H, doublet of triplets, J = 15.6 and 5.9 Hz);
6.45 (1H, doublet, J = 15.6 Hz).

Example 7

13-(3-Aminocinnamyloxy)milbemycin $A_4$

Nuclear Magnetic Resonance Spectrum ($CDCl_3$), δ ppm:
1.87 (3H, singlet);
3.34 (1H, doublet, J = 9.8 Hz);
3.96 (1H, doublet, J = 6.3 Hz);
6.19 (1H, doublet of triplets, J = 16.1 and 5.9 Hz);
6.47 (1H, doublet, J = 16.1 Hz).

Example 8

13-(2-Aminocinnamyloxy)milbemycin $A_4$

Nuclear Magnetic Resonance Spectrum ($CDCl_3$), δ ppm:
1.87 (3H, singlet);
3.36 (1H, doublet, J = 9.7 Hz);
3.96 (1H, doublet, J = 5.9 Hz);
6.13 (1H, doublet of triplets, J = 16.1 and 5.4 Hz);
6.61 (1H, doublet, J = 16.1 Hz).

Example 9

13-[3-(3-Aminophenyl)-2-methylprop-2(E)-enyloxylmilbemycin $A_4$

Nuclear Magnetic Resonance Spectrum ($CDCl_3$), δ ppm:
1.88 (3H, singlet);
1.88 (3H, singlet);
3.34 (1H, doublet, J = 9.7 Hz);
3.96 (1H, doublet, J = 6.3 Hz);
6.36 (1H, multiplet).

Example 10

13-[3-(3-Aminophenyl)-3-methylprop-2(E)-enyloxy]milbemycin $A_4$

Nuclear Magnetic Resonance Spectrum ($CDCl_3$), $\delta$ ppm:
1.87 (3H, singlet);
1.99 (3H, singlet);
3.33 (1H, doublet, J = 9.8 Hz);
3.96 (1H, doublet, J = 5.9 Hz);
5.86 (1H, multiplet).

Example 11

13-[3-(3-Aminophenyl)-3-methylprop-2(Z)-enyloxy]milbemycin $A_4$

Nuclear Magnetic Resonance Spectrum ($CDCl_3$), $\delta$ ppm:
1.87 (3H, singlet);
2.05 (3H, singlet);
3.16 (1H, doublet, J = 10.3 Hz);
3.95 (1H, doublet, J = 6.4 Hz);
5.60 (1H, multiplet).

EXAMPLE 12

13-[3-(4-Acetamidophenyl)propoxy]milbemycin $A_4$

0.035 g of 13-[3-(4-aminophenyl)propoxy]milbemycin $A_4$ (prepared as described in Example 5) was dissolved in 2.0 ml of methylene chloride, and 0.005 g of pyridine and 0.006 g of acetic anhydride were added to the resulting solution, after which the mixture was stirred at room temperature for 12 hours. At the end of this time, 10 ml of ethyl acetate were added to the reaction mixture, and the resulting mixture was washed with 0.1N aqueous hydrochloric acid, with water, with a 4% w/v aqueous solution of sodium hydrogencarbonate and with water, in that order, after which it was dried over anhydrous sodium sulphate. The solvent was then removed by evaporation under reduced pressure. The resulting residue was purified by column chromatography (through ODS; eluted with 80% v/v aqueous acetonitrile), to afford 0.035 g of the title compound.
Nuclear Magnetic Resonance Spectrum ($CDCl_3$), $\delta$ ppm:
1.87 (3H, singlet);
2.17 (3H, singlet);
3.19 (1H, doublet, J = 9.7 Hz);
3.96 (1H, doublet, J = 6.3 Hz).

EXAMPLES 13 TO 15

The compounds of Examples 13 to 15 were prepared by treating the amines prepared as described in Examples 6 to 8, respectively, by the same procedures as described in Example 12.

Example 13

13-(4-Acetamidocinnamyloxy)milbemycin $A_4$

Nuclear Magnetic Resonance Spectrum ($CDCl_3$), $\delta$ ppm:
1.88 (3H, singlet);
2.18 (3H, singlet);
3.34 (1H, doublet, J = 9.7 Hz);
3.96 (1H, doublet, J = 5.9 Hz);
6.19 (1H, doublet of triplets, J = 16.1 and 5.9 Hz);
6.52 (1H, doublet, J = 16.1 Hz).

Example 14

13-(3-Acetamidocinnamyloxy)milbemycin $A_4$

Nuclear Magnetic Resonance Spectrum ($CDCl_3$), δ ppm:
1.87 (3H, singlet);
2.18 (3H, singlet);
3.34 (1H, doublet, J = 9.7 Hz);
3.96 (1H, doublet, J = 6.3 Hz);
6.26 (1H, doublet of triplets, J = 16.1 and 5.9 Hz);
6.53 (1H, doublet, J = 16.1 Hz).

Example 15

13-(2-Acetamidocinnamyloxy)milbemycin $A_4$

Nuclear Magnetic Resonance Spectrum ($CDCl_3$), δ ppm:
1.88 (3H, singlet);
2.20 (3H, singlet);
3.37 (1H, doublet, J = 9.7 Hz);
3.96 (1H, doublet, J = 6.3 Hz);
6.17 (1H, doublet of triplets, J = 15.6 and 5.4 Hz);
6.66 (1H, doublet, J = 15.6 Hz).

EXAMPLE 16

13-{3-[3-(3-Methylureido)phenyl]propoxy}milbemycin $A_4$

0.069 g of 13-[3-(3-aminophenyl)propoxy]milbemycin $A_4$ (prepared as described in Example 3) was dissolved in 2.0 ml of tetrahydrofuran, and 0.009 g of methyl isocyanate was added to the resulting solution, after which the mixture was stirred for 4 days. At the end of this time, the reaction mixture was diluted with 20 ml of ethyl acetate, washed with water, and dried over anhydrous sodium sulphate. The solvent was then removed by evaporation under reduced pressure. The resulting residue was purified by column chromatography (through ODS; eluted with 80% v/v aqueous acetonitrile), to afford 0.051 g of the title compound.
Nuclear Magnetic Resonance Spectrum ($CDCl_3$), δ ppm:
1.87 (3H, singlet);
2.84 (3H, singlet);
3.20 (1H, doublet, J = 10.3 Hz);
3.96 (1H, doublet, J = 6.3 Hz).

EXAMPLES 17 TO 20

The compounds of Examples 17 to 20 were prepared by the same procedures as described in Example 16 above.

Example 17

13-{3-[2-(3-Methylureido)phenyl]propoxy}milbemycin $A_4$

Nuclear Magnetic Resonance Spectrum ($CDCl_3$), δ ppm:
1.88 (3H, singlet);
2.82 (3H, singlet);
3.25 (1H, doublet, J = 9.7 Hz);
3.97 (1H, doublet, J = 6.3 Hz).

Example 18

13-[3-(3-Methylureido)cinnamyloxy]milbemycin $A_4$

Nuclear Magnetic Resonance Spectrum ($CDCl_3$), $\delta$ ppm:
1.88 (3H, singlet);
3.34 (1H, doublet, J = 9.8 Hz);
3.96 (1H, doublet, J = 6.4 Hz);
6.26 (1H, doublet of triplets, J = 15.6 and 5.4 Hz);
6.54 (1H, doublet, J = 15.6 Hz).

Example 19

13-[3-(3-Phenylureido)cinnamyloxy]milbemycin $A_4$

Mass Spectrum m/z: 689 ($M^+$ -119).
Nuclear Magnetic Resonance Spectrum ($CDCl_3$), $\delta$ ppm:
1.87 (3H, singlet);
3.33 (1H, doublet, J = 9.8 Hz);
3.96 (1H, doublet, J = 6.4 Hz);
6.25 (1H, doublet of triplets, J = 15.6 and 5.9 Hz);
6.53 (1H, doublet, J = 15.6 Hz).

Example 20

13-[3-(3-Methylthioureido)cinnamyloxy]milbemycin $A_4$

Nuclear Magnetic Resonance Spectrum ($CDCl_3$), $\delta$ ppm:
1.88 (3H, singlet);
3.15 (3H, singlet);
3.34 (1H, doublet, J = 9.8 Hz);
3.96 (1H, doublet, J = 6.2 Hz);
6.00 (1H, broad singlet);
6.29 (1H, doublet of triplets, J = 16.1 and 5.4 Hz);
6.56 (1H, doublet, J = 16.1 Hz).

Example 21

13-(3-Ethoxycarbonylaminocinnamyloxy)milbemycin $A_4$

0.120 g of 13-(3-aminocinnamyloxy)milbemycin $A_4$ (prepared as described in Example 7) was dissolved in 1.0 ml of methylene chloride, and 0.014 ml of pyridine and 0.016 ml of ethyl chloroformate were added to the resulting solution, after which the mixture was stirred at room temperature for 25 minutes. At the end of this time, 10 ml of ethyl acetate was added to the reaction mixture, and the resulting mixture was washed with 0.1N aqueous hydrochloric acid, with water, with a 4% w/v aqueous solution of sodium hydrogencarbonate and with water, in that order, after which it was dried over anhydrous sodium sulphate. The solvent was then removed by evaporation under reduced pressure. The resulting residue was purified by column chromatography (through ODS; eluted with 80% v/v aqueous acetonitrile), to afford 0.035 g of the title compound.

Nuclear Magnetic Resonance Spectrum ($CDCl_3$), $\delta$ ppm:
1.87 (3H, singlet);
3.34 (1H, doublet, J = 9.8 Hz);
3.96 (1H, doublet, J = 6.8 Hz);
4.23 (2H, singlet);
6.26 (1H, doublet of triplets, J = 16.1 and 5.4 Hz);
6.26 (1H, doublet, J = 16.1 Hz).

EXAMPLES 22 TO 26

The compounds of Examples 22 to 26 were prepared by the same procedures as described in Example 21, above.

Example 22

13-(3-Cyanoacetamidocinnamyloxy)milbemycin $A_4$

Nuclear Magnetic Resonance Spectrum ($CDCl_3$), δ ppm:
1.87 (3H, singlet);
3.34 (1H, doublet, J = 9.8 Hz);
3.56 (2H, singlet);
3.96 (1H, doublet, J = 5.9 Hz);
6.28 (1H, doublet of triplets, J = 16.1 and 5.9 Hz);
6.56 (1H, doublet, J = 16.1 Hz).

Example 23

13-(3-Methoxalylaminocinnamyloxy)milbemycin $A_4$

Nuclear Magnetic Resonance Spectrum ($CDCl_3$), δ ppm:
1.88 (3H, singlet);
3.35 (1H, doublet, J = 9.8 Hz);
3.97 (1H, doublet, J = 7.8 Hz);
3.98 (2H, singlet);
6.29 (1H, doublet of triplets, J = 15.6 and 5.9 Hz);
6.57 (1H, doublet, J = 15.6 Hz).

Example 24

13-[3-(4-Cyanobenzoylamino)cinnamyloxy]milbemycin $A_4$

Nuclear Magnetic Resonance Spectrum ($CDCl_3$), δ ppm:
1.87 (3H, singlet);
3.35 (1H, doublet, J = 9.8 Hz);
3.96 (1H, doublet, J = 5.9 Hz);
6.28 (1H, doublet of triplets, J = 16.1 and 5.9 Hz);
6.58 (1H, doublet, J = 16.1 Hz).

Example 25

13-(3-Methanesulphonylaminocinnamyloxy)milbemycin $A_4$

Nuclear Magnetic Resonance Spectrum ($CDCl_3$), δ ppm:
1.88 (3H, singlet);
3.01 (3H, singlet);
3.34 (1H, doublet, J = 9.8 Hz);
3.96 (1H, doublet, J = 6.4 Hz);
6.29 (1H, doublet of triplets, J = 15.6 and 5.9 Hz);
6.56 (1H, doublet, J = 15.6 Hz).

Example 26

13-[3-N-(Methanesulphonyl)methylaminocinnamyloxy]milbemycin $A_4$

Nuclear Magnetic Resonance Spectrum ($CDCl_3$), δ ppm:
1.88 (3H, singlet);

2.85 (3H, singlet);
3.33 (3H, singlet);
3.96 (1H, doublet, J = 6.4 Hz);
6.29 (1H, doublet of triplets, J = 16.1 and 5.9 Hz);
6.57 (1H, doublet, J = 16.1 Hz).

EXAMPLE 27

13-(3-Methylaminocinnamyloxy)milbemycin $A_4$

250 mg of 13-(3-aminocinnamyloxy)milbemycin $A_4$ (obtained by substantially the same method as that described in Example 7) were dissolved in 0.5 ml of methanol. 15.0 mg of paraformaldehyde and 28.5 mg of sodium cyanoborohydride were then added to the resulting solution, and then the mixture was stirred at room temperature for 8 hours. At the end of this time, the reaction mixture was dissolved in 30 ml of ethyl acetate and washed with water, with a 4% w/v aqueous solution of sodium hydrogencarbonate and with water, in that order. The solution was then dried over anhydrous sodium sulphate, after which it was evaporated to dryness. The residue was purified by reverse phase chromatography (through ODS; 90% v/v aqueous acetonitrile), to afford 80 mg of the title compound.
Mass Spectrum m/z : 703 ($M^+$, $C_{42}H_{57}NO_8$).
Nuclear Magnetic Resonance Spectrum ($CDC\ell_3$), $\delta$ ppm:
1.87 (3H, singlet);
2.84 (3H, singlet);
3.25 (1H, doublet, J = 9.8 Hz);
3.96 (1H, doublet, J = 5.9 Hz);
6.15 - 6.27 (1H, multiplet);
6.75 (1H, doublet, J = 7.8 Hz).

EXAMPLE 28

13-[3-(3,3-Dimethylcarbazoylamino)cinnamyloxy]milbemycin $A_4$

119 mg of 13-(3-aminocinnamyloxy)milbemycin $A_4$ (obtained by substantially the same method as that described in Example 7) were dissolved in 0.9 ml of methylene chloride. 44.4 mg of 2-chloroformyl-1,2,4-triazolo[4.3-a]pyridin-2-one and 0.019 ml of pyridine were then added to the resulting solution, and then the mixture was stirred at room temperature for 1 hour. At the end of this time, 0.010 ml of 1,1-dimethylhydrazine was added, and then the whole mixture was stirred at room temperature for a further 1 hour. The reaction mixture was allowed to stand overnight at room temperature, after which it was diluted with 30 ml of ethyl acetate. The solution was then washed with water, with a 4% w/v aqueous solution of sodium hydrogencarbonate, and with water, in that order. The solution was then dried over anhydrous sodium sulphate, after which it was evaporated to dryness. The residue was purified by reverse phase chromatography (through ODS; 80% v/v aqueous acetonitrile), to afford 80 mg of the title compound.
Mass Spectrum m/z : 689 ($M^+$-86).
Nuclear Magnetic Resonance Spectrum ($CDC\ell_3$), $\delta$ ppm:
1.87 (3H, singlet);
2.62 (6H, singlet);
3.34 (1H, doublet, J = 9.8 Hz);
3.96 (1H, doublet, J = 6.4 Hz).

EXAMPLE 29

13-(2-Phenylcyclopropylmethoxy)milbemycin $A_4$

The procedure described in Example 3(a) was repeated, except that 2-phenylcyclopropylmethanol was used in place of 3-(3-nitrophenyl)propanol, and then the reaction product was worked up following the method described in Example 3(b), to give the title compound.
Mass Spectrum m/z : 688 ($M^+$, $C_{42}H_{56}O_8$).
Nuclear Magnetic Resonance Spectrum ($CDC\ell_3$), $\delta$ ppm:
1.87 (3H, singlet);

3.10 - 3.45 (2H, multiplet);
3.25 (1H, doublet, J = 6.8 Hz);
3.95 (1H, doublet, J = 6.4 Hz).

## TEST EXAMPLES

The anthelmintic activity against Nippostongylus brasiliensis, a nematode parasitic to rats, was examined with groups each containing 3 Wistar strain rats of body weight in the range from 40 to 60 g.

The rats were infested percutaneously with about 100 larvae of the nematode for each rat. Solutions containing the test compound at various concentrations were administered orally 3 days after infection. Each solution was prepared by dissolving 1.0 mg of the test compound in 0.1 ml of dimethylformamide, and then adding 10 ml of polyethylene glycol (PEG 400) to the solution. The solution was then adjusted by the addition of PEG 400 to achieve a concentration of 0.250 or 0.125 mg/kg.

The rats were killed 4 days after infection, and the number of parasites in the small intestine was counted. The results obtained are summarized in Table 1.

In the Table, the anthelmintic activity was calculated by the following formula:-

$$\text{Anthelmintic activity (\%)} = 100 \times \frac{\text{Number of parasites in untreated group} - \text{Number of parasites in treated group}}{\text{Number of parasites in untreated infected group}}$$

Table 1

| Effect of the compounds administered orally | | |
|---|---|---|
| | Anthelmintic activity (%)* | |
| | 0.250** | 0.125** |
| 1) Compound of Example 7 | - | 87.8 |
| 2) Compound of Example 8 | - | 69.1 |
| 3) Compound of Example 13 | - | 76.1 |
| 4) Compound of Example 14 | - | 91.6 |
| 5) Compound of Example 15 | - | 88.1 |
| 6) Compound of Example 18 | - | 88.7 |
| 7) Compound of Example 20 | - | 76.9 |
| 8) Compound of Example 21 | - | 83.2 |
| 9) Compound of Example 22 | - | 98.3 |
| 10) Compound of Example 23 | - | 83.1 |
| 11) Compound of Example 25 | - | 99.3 |
| 12) Compound of Example 26 | - | 99.8 |
| 13) Compound of Example 27 | - | 100.0 |
| 14) Compound of Example 28 | - | 99.0 |
| 15) 13-Methoxy-milbemycin $A_4$*** | 44.0 | 49.5 |
| 16) Milbemycin $A_4$ | 24.8 | - |

** Dose : mg/kg
*** Compound disclosed in US Patent No. 4 696 945.

In the above Table, a dash means that the compound was not tested at the particular concentration.

**Claims**

**Claims for the following Contracting States : GB, DE, FR, IT, CH, DK, BE, NL, SE, LU, AT**

1. Compounds of formula (I):

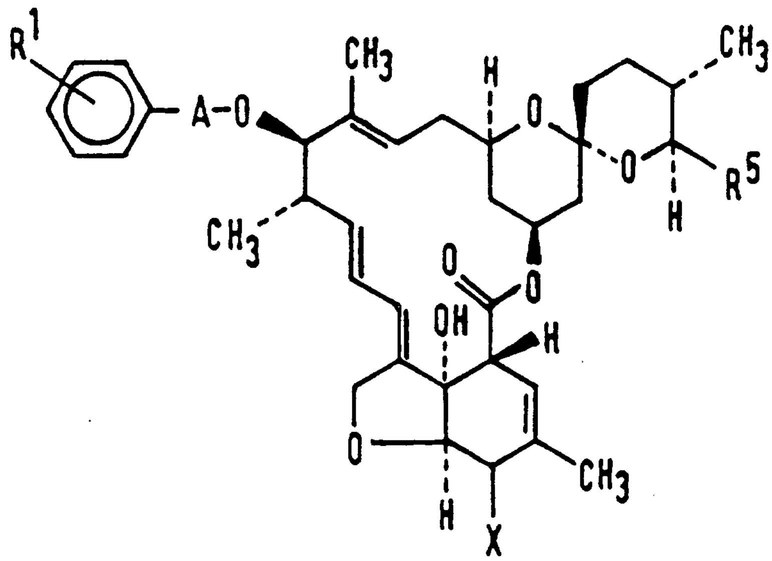

(I)

in which:

$R^1$ represents: a hydrogen atom; a halogen atom; a cyano group; a nitro group; an alkyl group which has from 1 to 4 carbon atoms and which is unsubstituted or is substituted by at least one of substituents (a); an alkoxy group having from 1 to 4 carbon atoms; an alkoxyalkoxy group having a total of from 2 to 6 carbon atoms; or a group having one of the following formulae:

$-(CH_2)_nNHR^9$
$-(CH_2)_nNR^9COR^6$
$-(CH_2)_nNR^9COCOR^6$
$-(CH_2)_nNR^9COCOOR^7$
$-(CH_2)_nNR^9CHR^6NHCOR^6$
$-(CH_2)_nNR^9CHR^6NHCONHR^6$
$-(CH_2)_nNR^9CHR^6NHCOOR^7$
$-(CH_2)_nNR^9C(=Y)YR^6$
$-(CH_2)_nNR^9C(=Y)NR^6R^6$
$-(CH_2)_nNR^9C(=Y)NR^6NR^6R^6$
$-(CH_2)_nNR^9C(=Y)NR^6NHZ$
$-(CH_2)_nNR^9C(=NR^{11})NHR^{11}$
$-(CH_2)_nNR^9C(=NR^{11})R^6$
$-(CH_2)_nNR^9SO_mR^6$
$-CONHR^6$

or

$-COOR^7$

in which:

m is 1 or 2;

n is 0, 1 or 2;

$R^6$ represents a hydrogen atom; an alkyl group having from 1 to 8 carbon atoms; a substituted alkyl group which has from 1 to 8 carbon atoms and which is substituted by at least one of substituents (b); an aliphatic hydrocarbon group having from 2 to 8 carbon atoms and having one or two carbon-carbon double or triple bonds; a cycloalkyl group having from 2 to 8 carbon atoms; a substituted cycloalkyl group which has from 2 to 8 carbon atoms and which is substituted by at least one of substituents (c); an

aryl group which has from 6 to 14 ring carbon atoms and which is unsubstituted or which is substituted by at least one of substituents (c); an aryloxy group which has from 6 to 14 ring carbon atoms and which is unsubstituted or which is substituted by at least one of substituents (c); or an arylthio group which has from 6 to 14 ring carbon atoms and which is unsubstituted or which is substituted by at least one of substituents (c);

$R^7$ represents an alkyl group having from 1 to 4 carbon atoms, a cycloalkyl group having from 3 to 7 carbon atoms, or an aralkyl group in which an alkyl group having from 1 to 4 carbon atoms is substituted by from 1 to 3 aryl groups which have from 6 to 10 ring carbon atoms and which are unsubstituted or substituted by at least one of substituents (c);

$R^9$ represents a hydrogen atom or an alkyl group having from 1 to 4 carbon atoms;

$R^{11}$ represents any of the groups or atoms defined above for $R^6$, or it represents a cyano group, a nitro group, a group of formula $-COOR^7$ (in which $R^7$ is as defined above), or a group of formula $-COR^6$ - (in which $R^6$ is as defined above);

Y represents an oxygen atom or a sulphur atom; and, where there are two or more groups represented by Y, these are the same or different from each other;

Z represents a group of formula $-COOR^7$ (in which $R^7$ is as defined above), a group of formula $-COR^6$ (in which $R^6$ is as defined above) or a group of formula $-SO_2R^6$ (in which $R^6$ is as defined above);

A represents a group having one of the following formulae:

$-CHR^2-CHR^3-CHR^4-$
$-CR^2=CR^3-CHR^4-$

or

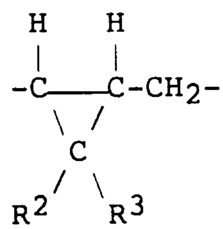

in which $R^2$, $R^3$ and $R^4$ are the same or different and each represents a hydrogen atom, a halogen atom, an alkyl group having from 1 to 4 carbon atoms, or an alkoxy group having from 1 to 4 carbon atoms;

$R^5$ represents a methyl group, an ethyl group, an isopropyl group or a sec-butyl group; and

X represents: a hydroxy group; an alkanoyloxy group which has from 1 to 5 carbon atoms, and which is unsubstituted or is substituted by at least one of substituents (d); or a hydroxyimino group;

substituents (a):

halogen atoms, alkoxy groups having from 1 to 4 carbon atoms, alkylthio groups having from 1 to 4 carbon atoms, and alkanoyloxy groups having from 1 to 5 carbon atoms;

substituents (b):

cycloalkyl groups having from 3 to 8 carbon atoms; alkoxy groups having from 1 to 4 carbon atoms; alkylthio groups having from 1 to 4 carbon atoms; cyanoalkylthio groups having from 2 to 5 carbon atoms; alkoxycarbonyl groups having from 2 to 5 carbon atoms; halogen atoms; cyano groups; nitro groups; amino groups; aryl groups which have from 6 to 14 ring carbon atoms and which are unsubstituted or are substituted by at least one of substituents (c); a pyridyl, thienyl, furyl, pyrrolyl, imidazolyl, triazolyl, tetrazolyl, thiazolyl, oxazolyl, indolyl group, or such a group substituted by at least one of substituents (c); aryloxy groups which have from 6 to 14 ring carbon atoms and which are unsubstituted or are substituted by at least one of substituents (c); and arylthio groups which have from 6 to 14 ring carbon atoms and which are unsubstituted or are substituted by at least one of substituents (c);

substituents (c):

alkyl groups having from 1 to 4 carbon atoms, alkoxy groups having from 1 to 4 carbon atoms, alkylthio groups having from 1 to 4 carbon atoms, alkanoyloxy groups having from 1 to 5 carbon atoms, alkoxycarbonyl groups having from 2 to 5 carbon atoms, halogen atoms, cyano groups, nitro

groups, amino groups, alkylamino groups in which the alkyl part has from 1 to 4 carbon atoms, dialkylamino groups in which each alkyl part is the same or different and each represents an alkyl group having from 1 to 4 carbon atoms, a carbamoyl group, an alkylcarbamoyl group in which the alkyl part has from 1 to 4 carbon atoms, a dialkylcarbamoyl group in which each alkyl part has from 1 to 4 carbon atoms, and alkanoylamino groups having from 1 to 5 carbon atoms;

substituents (d):

halogen atoms, alkoxy groups having from 1 to 4 carbon atoms, alkoxycarbonyl groups having from 2 to 5 carbon atoms, and carboxy groups;

and salts and esters thereof.

**2.** Compounds according to Claim 1, in which $R^1$ represents: a hydrogen atom, an alkyl group having from 1 to 3 carbon atoms; an alkoxy group having from 1 to 3 carbon atoms; the fluorine or chlorine atoms; or the nitro or amino groups.

**3.** Compounds according to Claim 1, in which $R^1$ represents a group of formula $-NR^{9a}COR^{6a}$

in which:

$R^{9a}$ represents a hydrogen atom or a methyl group; and

$R^{6a}$ represents: a hydrogen atom; an alkyl group having from 1 to 4 carbon atoms; a cycloalkyl group having from 3 to 5 carbon atoms; a substituted alkyl group having from 1 to 3 carbon atoms, and substituted with a halogen atom, a cyano group, an alkoxy group having from 1 to 3 carbon atoms, an alkylthio group having from 1 to 3 carbon atoms, a cyanomethylthio group or a phenoxy group; an alkenyl group having from 2 to 4 carbon atoms; a phenyl group; or a phenyl group substituted with an alkyl group having from 1 to 3 carbon atoms, an alkoxy group having from 1 to 3 carbon atoms, a halogen atom or a nitro group.

**4.** Compounds according to Claim 1, in which $R^1$ represents a group of formula $-NR^{9a}COCOR^{6b}$

in which:

$R^{9a}$ represents a hydrogen atom or a methyl group; and

$R^{6b}$ represents: a hydrogen atom; an alkyl group having from 1 to 4 carbon atoms; a cycloalkyl group having from 3 to 5 carbon atoms; an alkenyl group having from 2 to 4 carbon atoms; a phenyl group; or a phenyl group substituted with an alkyl group having from 1 to 3 carbon atoms, an alkoxy group having from 1 to 3 carbon atoms, a halogen atom or a nitro group.

**5.** Compounds according to Claim 1, in which $R^1$ represents a group of formula $-NR^{9a}C(=Y)YR^{6c}$

in which:

$R^{9a}$ represents a hydrogen atom or a methyl group;

Y represents an oxygen atom; and

$R^{6c}$ represents: an alkyl group having from 1 to 4 carbon atoms; an alkyl group having from 1 to 4 carbon atoms, and substituted with a halogen atom or an alkoxy group having from 1 to 3 carbon atoms; a vinyl group; an allyl group; a benzyl group; a methoxybenzyl group; nitrobensyl group; or a phenyl group.

**6.** Compounds according to Claim 1, in which $R^1$ represents a group of formula $-NR^{9a}C(=Y)NR^{6d}R^{6e}$

in which:

$R^{9a}$ represents a hydrogen atom or a methyl group;

Y represents an oxygen atom or a sulphur atom; and

$R^{6d}$ and $R^{6e}$ are the same or different and each represents: a hydrogen atom; an alkyl group having from 1 to 4 carbon atoms; a cycloalkyl group having from 3 to 6 carbon atoms; a phenyl group; or a phenyl group substituted with an alkyl group having from 1 to 3 carbon atoms, an alkoxy group having from 1 to 3 carbon atoms, a halogen atom or a nitro group.

**7.** Compounds according to Claim 1, in which $R^1$ represents a group of formula

$$-NR^{9a}C(=Y)NR^{6f}NR^{6g}R^{6h}$$

in which:

$R^{9a}$ represents a hydrogen atom or a methyl group;

Y represents an oxygen atom or a sulphur atom; and

$R^{6f}$, $R^{6g}$ and $R^{6h}$ are the same or different and each represents: a hydrogen atom; an alkyl group having from 1 to 4 carbon atoms; a cycloalkyl group having from 3 to 6 carbon atoms; a phenyl group; or a phenyl group substituted with an alkyl group having from 1 to 3 carbon atoms, an alkoxy group having from 1 to 3 carbon atoms, a halogen atom or a nitro group.

**8.** Compounds according to Claim 1, in which $R^1$ represents a group of formula $-NR^{9a}C(=Y)NR^{6j}NHZ$

in which:

$R^{9a}$ represents a hydrogen atom or a methyl group;

Y represents an oxygen atom or a sulphur atom;

$R^{6j}$ represents an alkyl group having from 1 to 4 carbon atoms; or a cycloalkyl group having from 3 to 6 carbon atoms;

Z represents a group of formula $-COOR^{7a}$ (in which: $R^{7a}$ represents an alkyl group having from 1 to 4 carbon atoms, a cycloalkyl group having from 3 to 6 carbon atoms or a benzyl group); a group of formula $-COR^{6k}$ (in which $R^{6k}$ represents: an alkyl group having from 1 to 4 carbon atoms; a cycloalkyl group having from 3 to 6 carbon atoms a phenyl group; or a phenyl group substituted with an alkyl group having from 1 to 3 carbon atoms, an alkoxy group having from 1 to 3 carbon atoms, a halogen atom or a nitro group), or a group of formula $-SO_2R^{6m}$ (in which $R^{6m}$ represents: an alkyl group having from 1 to 4 carbon atoms; a phenyl group; or a phenyl group substituted with an alkyl group having from 1 to 3 carbon atoms, an alkoxy group having from 1 to 3 carbon atoms, a halogen atom or a nitro group).

**9.** Compounds according to Claim 1, in which $R^1$ represents a group of formula $-NR^{9a}C(=NR^{11a})NHR^{11b}$

in which:

$R^{9a}$ represents a hydrogen atom or a methyl group;

$R^{11a}$ and $R^{11b}$ are the same or different and each represents: a hydrogen atom; an alkyl group having from 1 to 4 carbon atoms; a phenyl group; a phenyl group substituted with an alkyl group having from 1 to 3 carbon atoms, an alkoxy group having from 1 to 3 carbon atoms, a halogen atom or a nitro group; a group of formula $-COOR^{7b}$ (in which $R^{7b}$ represents: an alkyl group having from 1 to 4 carbon atoms; a cycloalkyl group having from 3 to 6 carbon atoms; or a benzyl group); or a group of formula $-COR^{6n}$ (in which $R^{6n}$ represents: an alkyl group having from 1 to 4 carbon atoms; a cycloalkyl group having from 3 to 6 carbon atoms; a phenyl group; or a phenyl group substituted with an alkyl group having from 1 to 3 carbon atoms, an alkoxy group having from 1 to 3 carbon atoms, a halogen atom or a nitro group).

**10.** Compounds according to Claim 1, in which $R^1$ represents a group of formula $-NR^{9a}C(=NR^{11c})R^{6p}$

in which:

$R^{9a}$ represents a hydrogen atom or a methyl group;

$R^{11c}$ represents: a hydrogen atom; an alkyl group having from 1 to 4 carbon atoms; a phenyl group; a phenyl group substituted with an alkyl group having from 1 to 3 carbon atoms, an alkoxy group having from 1 to 3 carbon atoms, a halogen atom or a nitro group; a group of formula $-COOR^{7c}$ (in which $R^{7c}$ represents: an alkyl group having from 1 to 4 carbon atoms; a cycloalkyl group having from 3 to 6 carbon atoms; or a benzyl group); or a group of formula $-COR^{6q}$ (in which $R^{6q}$ represents: an alkyl group having from 1 to 4 carbon atoms; a cycloalkyl group having from 3 to 6 carbon atoms; a phenyl group; or a phenyl group substituted with an alkyl group having from 1 to 3 carbon atoms, an alkoxy group having from 1 to 3 carbon atoms, a halogen atom or a nitro group);

$R^{6p}$ represents: an alkyl group having from 1 to 4 carbon atoms; a cycloalkyl group having from 3 to 6 carbon atoms; a phenyl group; or a phenyl group substituted with an alkyl group having from 1 to 3 carbon atoms, an alkoxy group having from 1 to 3 carbon atoms, a halogen atom or a nitro group).

**11.** Compounds according to Claim 1, in which $R^1$ represents a group of formula $-NR^{9a}SO_mR^{6r}$

in which:

$R^{9a}$ represents a hydrogen atom or a methyl group;

$R^{6r}$ represents: an alkyl group having from 1 to 4 carbon atoms; an alkyl group substituted with a cyano group; a phenyl group; or a phenyl group substituted with an alkyl group having from 1 to 3 carbon atoms, an alkoxy group having from 1 to 3 carbon atoms, a halogen atom or a nitro group; and

$\underline{m}$ is 1 or 2.

**12.** Compounds according to any one of Claims 1 to 11, in which A represents a group of formula:

$$-CHR^2-CHR^3-CHR^4-$$

in which $R^2$, $R^3$ and $R^4$ are the same or different and each represents a hydrogen atom or a methyl group.

**13.** Compounds according to any one of Claims 1 to 11, in which A represents a group of formula:

$$-CR^2=CR^3-CHR^4-$$

in which $R^2$, $R^3$ and $R^4$ are the same or different and each represents a hydrogen atom or a methyl group.

**14.** Compounds according to any one of the preceding Claims, in which the substituent $R^1$ is at the meta or the ortho position.

**15.** Compounds according to Claim 1, in which A represents a group of formula:

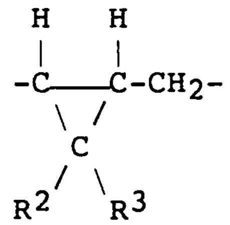

in which $R^2$ and $R^3$ are the same or different and each represents a hydrogen atom, a chlorine atom or a methyl group.

**17.** Compounds according to any one of Claims 1 to 16, in which $R^5$ represents an ethyl group.

**18.** A compound according to any one of Claims 1 to 16, which is a mixture of a compound in which $R^5$ represents an ethyl group and the corresponding compound in which $R^5$ represents a methyl group.

**19.** Compounds according to any one of the preceding Claims, in which $R^3$ and $R^4$ each represent hydrogen atoms.

**20.** Compounds according to any one of the preceding Claims, in which X represents a hydroxy group.

**21.** 13-(3-Cyanoacetamidocinnamyloxy)milbemycin $A_4$ and pharmaceutically acceptable salts thereof.

**22.** 13-(3-Methanesulphonylaminocinnamyloxy)milbemycin $A_4$ and pharmaceutically acceptable salts thereof.

**23.** 13-[3-N-(Methanesulphonyl)methylaminocinnamyloxy]milbemycin $A_4$ and pharmaceutically acceptable salts thereof.

**24.** An anthelmintic, acaricidal and insecticidal composition comprising at least one compound according to any one of the preceding Claims in admixture with a pharmaceutically, agriculturally, veterinarily or horticulturally acceptable carrier or diluent.

**25.** A composition according to Claim 24, in which said compound is:
13-(3-aminocinnamyloxy)milbemycin $A_4$
13-(2-aminocinnamyloxy)milbemycin $A_4$
13-(3-acetamidocinnamyloxy)milbemycin $A_4$
13-[3-(3-methylthioureido)cinnamyloxy]milbemycin $A_4$
13-(3-cyanoacetamidocinnamyloxy)milbemycin $A_4$
13-(3-methanesulphonylaminocinnamyloxy)milbemycin $A_4$
13-[3-N-(methanesulphonyl)methylaminocinnamyloxy]milbemycin $A_4$
13-(3-methylaminocinnamyloxy)milbemycin $A_4$ and
13-[3-(3,3-dimethylcarbazoylamino)cinnamyloxy]milbemycin $A_4$,
or a pharmaceutically acceptable salt thereof.

**26.** A compound according to any one of Claims 1 to 23 for use in therapy.

**27.** A use of a compound according to any one of Claims 1 to 23 for the manufacture of a medicament for the treatment of animals parasitized by helminths, acarids or insects. plants or to seeds of said plants or to a locus including said animals, plants or seeds, in which the active compound is at least one compound according to any one of Claims 1 to 23.

**28.** A method according to Claim 26 or 27, in which said compound is:
13-(3-aminocinnamyloxy)milbemycin $A_4$
13-(2-aminocinnamyloxy)milbemycin $A_4$
13-(3-acetamidocinnamyloxy)milbemycin $A_4$
13-[3-(3-methylthioureido)cinnamyloxy]milbemycin $A_4$
13-(3-cyanoacetamidocinnamyloxy)milbemycin $A_4$
13-(3-methanesulphonylaminocinnamyloxy)milbemycin $A_4$
13-[3-N-(methanesulphonyl)methylaminocinnamyloxy]milbemycin $A_4$
13-(3-methylaminocinnamyloxy)milbemycin $A_4$ and
13-[3-(3,3-dimethylcarbazoylamino)cinnamyloxy]milbemycin $A_4$,
or a pharmaceutically acceptable salt thereof.

**29.** The use of a compound according to any one of Claims 1 to 23 in therapy.

**30.** The use of a compound according to any one of Claims 1 to 23 for the manufacture of a medicament for the treatment of animals parasitized by helminths, acarids or insects.

**31.** A process for preparing a compound according to any one of Claims 1 to 23, which comprises subjecting a compound of formula (II) in either order to steps (a) and (b):

(II)

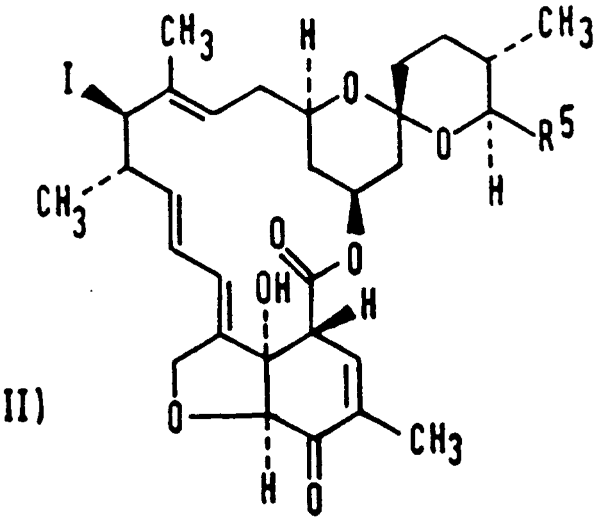

in which $R^5$ is as defined above,

(a) reaction with an alcohol of formula (IIa):

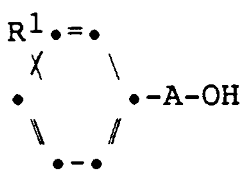

(IIa)

in which $R^1$ is as defined in Claim 1; and

(b) reaction either

($b^i$) with a reducing agent to reduce the oxygen atom at the 5-position to a hydroxy group, followed, if desired by reaction with an acylating agent, to give a compound of formula (I) in which X represents an alkanoyloxy group which has from 1 to 5 carbon atoms, and which is unsubstituted or which is substituted by at least one of substituents (d); or

($b^{ii}$) with hydroxylamine or with a salt thereof, to give a compound of formula (I) in which X represents a hydroxyimino group;

and then, if required, subjecting the product to one or both of steps (c) and (d):

(c) converting a group represented by $R^1$ to any other group so represented; and

(d) salifying or esterifying the product.

**Claims for the following Contracting States : ES, GR**

**1.** A process for preparing compounds of formula (I):

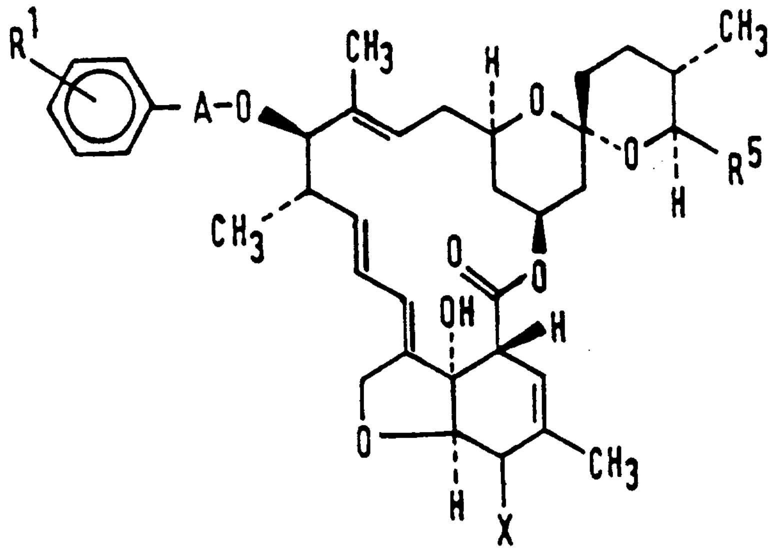

(I)

in which:
$R^1$ represents: a hydrogen atom; a halogen atom; a cyano group; a nitro group; an alkyl group which has from 1 to 4 carbon atoms and which is unsubstituted or is substituted by at least one of substituents (a); an alkoxy group having from 1 to 4 carbon atoms; an alkoxyalkoxy group having a total of from 2 to 6 carbon atoms; or a group having one of the following formulae:

$-(CH_2)_nNHR^9$
$-(CH_2)_nNR^9COR^6$
$-(CH_2)_nNR^9COCOR^6$
$-(CH_2)_nNR^9COCOOR^7$
$-(CH_2)_nNR^9CHR^6NHCOR^6$
$-(CH_2)_nNR^9CHR^6NHCONHR^6$
$-(CH_2)_nNR^9CHR^6NHCOOR^7$
$-(CH_2)_nNR^9C(=Y)YR^6$
$-(CH_2)_nNR^9C(=Y)NR^6R^6$
$-(CH_2)_nNR^9C(=Y)NR^6NR^6R^6$
$-(CH_2)_nNR^9C(=Y)NR^6NHZ$
$-(CH_2)_nNR^9C(=NR^{11})NHR^{11}$
$-(CH_2)_nNR^9C(=NR^{11})R^6$
$-(CH_2)_nNR^9SO_mR^6$
$-CONHR^6$

or

$-COOR^7$

in which:

$m$ is 1 or 2;

$n$ is 0, 1 or 2;

$R^6$ represents a hydrogen atom; an alkyl group having from 1 to 8 carbon atoms; a substituted alkyl group which has from 1 to 8 carbon atoms and which is substituted by at least one of substituents (b); an aliphatic hydrocarbon group having from 2 to 8 carbon atoms and having one or two carbon-carbon double or triple bonds; a cycloalkyl group having from 2 to 8 carbon atoms; a substituted cycloalkyl group which has from 2 to 8 carbon atoms and which is substituted by at least one of substituents (c); an aryl group which has from 6 to 14 ring carbon atoms and which is unsubstituted or which is substituted

by at least one of substituents (c); an aryloxy group which has from 6 to 14 ring carbon atoms and which is unsubstituted or which is substituted by at least one of substituents (c); or an arylthio group which has from 6 to 14 ring carbon atoms and which is unsubstituted or which is substituted by at least one of substituents (c);

$R^7$ represents an alkyl group having from 1 to 4 carbon atoms, a cycloalkyl group having from 3 to 7 carbon atoms, or an aralkyl group in which an alkyl group having from 1 to 4 carbon atoms is substituted by from 1 to 3 aryl groups which have from 6 to 10 ring carbon atoms and which are unsubstituted or substituted by at least one of substituents (c);

$R^9$ represents a hydrogen atom or an alkyl group having from 1 to 4 carbon atoms;

$R^{11}$ represents any of the groups or atoms defined above for $R^6$, or it represents a cyano group, a nitro group, a group of formula $-COOR^7$ (in which $R^7$ is as defined above), or a group of formula $-COR^6$ - (in which $R^6$ is as defined above);

Y represents an oxygen atom or a sulphur atom; and, where there are two or more groups represented by Y, these are the same or different from each other;

Z represents a group of formula $-COOR^7$ (in which $R^7$ is as defined above), a group of formula $-COR^6$ (in which $R^6$ is as defined above) or a group of formula $-SO_2R^6$ (in which $R^6$ is as defined above);

A represents a group having one of the following formulae:

$-CHR^2-CHR^3-CHR^4-$

$-CR^2=CR^3-CHR^4-$

or

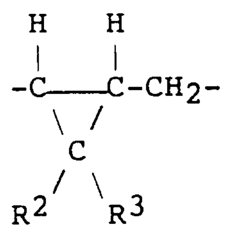

in which $R^2$, $R^3$ and $R^4$ are the same or different and each represents a hydrogen atom, a halogen atom, an alkyl group having from 1 to 4 carbon atoms, or an alkoxy group having from 1 to 4 carbon atoms;

$R^5$ represents a methyl group, an ethyl group, an isopropyl group or a sec-butyl group; and

X represents: a hydroxy group; an alkanoyloxy group which has from 1 to 5 carbon atoms, and which is unsubstituted or is substituted by at least one of substituents (d); or a hydroxyimino group;

substituents (a):

halogen atoms, alkoxy groups having from 1 to 4 carbon atoms, alkylthio groups having from 1 to 4 carbon atoms, and alkanoyloxy groups having from 1 to 5 carbon atoms;

substituents (b):

cycloalkyl groups having from 3 to 8 carbon atoms; alkoxy groups having from 1 to 4 carbon atoms; alkylthio groups having from 1 to 4 carbon atoms; cyanoalkylthio groups having from 2 to 5 carbon atoms; alkoxycarbonyl groups having from 2 to 5 carbon atoms; halogen atoms; cyano groups; nitro groups; amino groups; aryl groups which have from 6 to 14 ring carbon atoms and which are unsubstituted or are substituted by at least one of substituents (c); a pyridyl, thienyl, furyl, pyrrolyl, imidazolyl, triazolyl, tetrazolyl, thiazolyl, oxazolyl, indolyl group, or such a group substituted by at least one of substituents (c); aryloxy groups which have from 6 to 14 ring carbon atoms and which are unsubstituted or are substituted by at least one of substituents (c); and arylthio groups which have from 6 to 14 ring carbon atoms and which are unsubstituted or are substituted by at least one of substituents (c);

substituents (c):

alkyl groups having from 1 to 4 carbon atoms, alkoxy groups having from 1 to 4 carbon atoms, alkylthio groups having from 1 to 4 carbon atoms, alkanoyloxy groups having from 1 to 5 carbon atoms, alkoxycarbonyl groups having from 2 to 5 carbon atoms, halogen atoms, cyano groups, nitro

groups, amino groups, alkylamino groups in which the alkyl part has from 1 to 4 carbon atoms, dialkylamino groups in which each alkyl part is the same or different and each represents an alkyl group having from 1 to 4 carbon atoms, a carbamoyl group, an alkylcarbamoyl group in which the alkyl part has from 1 to 4 carbon atoms, a dialkylcarbamoyl group in which each alkyl part has from 1 to 4 carbon atoms, and alkanoylamino groups having from 1 to 5 carbon atoms;

substituents (d):

halogen atoms, alkoxy groups having from 1 to 4 carbon atoms, alkoxycarbonyl groups having from 2 to 5 carbon atoms, and carboxy groups;

and salts and esters thereof, which comprises subjecting a compound of formula (II) in either order to steps (a) and (b):

(II)

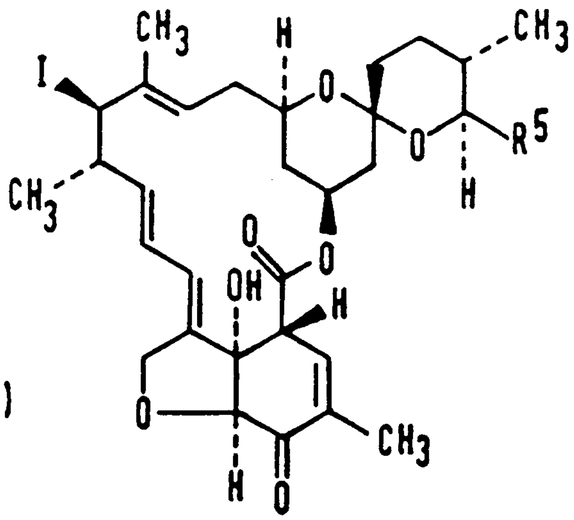

in which $R^5$ is as defined above,

(a) reaction with an alcohol of formula (IIa):

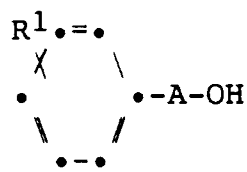

(IIa)

in which $R^1$ is as defined above; and

(b) reaction either

($b^i$) with a reducing agent to reduce the oxygen atom at the 5-position to a hydroxy group, followed, if desired by reaction with an acylating agent, to give a compound of formula (I) in which X represents an alkanoyloxy group which has from 1 to 5 carbon atoms, and which is unsubstituted or which is substituted by at least one of substituents (d); or

($b^{ii}$) with hydroxylamine or with a salt thereof, to give a compound of formula (I) in which X represents a hydroxyimino group;

and then, if required, subjecting the product to one or both of steps (c) and (d):

(c) converting a group represented by $R^1$ to any other group so represented; and

(d) salifying or esterifying the product.

**2.** A process according to Claim 1, in which $R^1$ represents: a hydrogen atom, an alkyl group having from 1 to 3 carbon atoms; an alkoxy group having from 1 to 3 carbon atoms; the fluorine or chlorine atoms; or the nitro or amino groups.

**3.** A process according to Claim 1, in which $R^1$ represents a group of formula $-NR^{9a}COR^{6a}$

in which:

$R^{9a}$ represents a hydrogen atom or a methyl group; and

$R^{6a}$ represents: a hydrogen atom; an alkyl group having from 1 to 4 carbon atoms; a cycloalkyl group having from 3 to 5 carbon atoms; a substituted alkyl group having from 1 to 3 carbon atoms, and substituted with a halogen atom, a cyano group, an alkoxy group having from 1 to 3 carbon atoms, an alkylthio group having from 1 to 3 carbon atoms, a cyanomethylthio group or a phenoxy group; an alkenyl group having from 2 to 4 carbon atoms; a phenyl group; a phenyl group substituted with an alkyl group having from 1 to 3 carbon atoms, an alkoxy group having from 1 to 3 carbon atoms, a halogen atom or a nitro group.

**4.** A process according to Claim 1, in which $R^1$ represents a group of formula $-NR^{9a}COCOR^{6b}$

in which:

$R^{9a}$ represents a hydrogen atom or a methyl group; and

$R^{6a}$ represents: a hydrogen atom; an alkyl group having from 1 to 4 carbon atoms; a cycloalkyl group having from 3 to 5 carbon atoms; an alkenyl group having from 2 to 4 carbon atoms; a phenyl group; or a phenyl group substituted with an alkyl group having from 1 to 3 carbon atoms, an alkoxy group having from 1 to 3 carbon atoms, a halogen atom or a nitro group.

**5.** A process according to Claim 1, in which $R^1$ represents a group of formula $-NR^{9a}C(=Y)YR^{6c}$

in which:

$R^{9a}$ represents a hydrogen atom or a methyl group;

Y represents an oxygen atom; and

$R^{6c}$ represents: an alkyl group having from 1 to 4 carbon atoms; an alkyl group having from 1 to 4 carbon atoms, and substituted with a halogen atom or an alkoxy group having from 1 to 3 carbon atoms; a vinyl group; an allyl group; a benzyl group; a methoxybenzyl group; nitrobenzyl group;

or a phenyl group.

**6.** A process according to Claim 1, in which $R^1$ represents a group of formula $-NR^{9a}C(=Y)NR^{6d}R^{6e}$

in which:

$R^{9a}$ represents a hydrogen atom or a methyl group;

Y represents an oxygen atom or a sulfur atom; and

$R^{6d}$ and $R^{6e}$ are the same or different and each represents: a hydrogen atom; an alkyl group having from 1 to 4 carbon atoms; a cycloalkyl group having from 3 to 6 carbon atoms; a phenyl group; or a phenyl group substituted with an alkyl group having from 1 to 3 carbon atoms, an alkoxy group having from 1 to 3 carbon atoms, a halogen atom or a nitro group.

**7.** A process according to Claim 1, in which $R^1$ represents a group of formula

$$-NR^{9a}C(=Y)NR^{6f}NR^{6g}R^{6h}$$

in which:

$R^{9a}$ represents a hydrogen atom or a methyl group;

Y represents an oxygen atom or a sulfur atom; and

$R^{6f}$, $R^{6g}$ and $R^{6h}$ are the same or different and each represents: a hydrogen atom; an alkyl group having from 1 to 4 carbon atoms; a cycloalkyl group having from 3 to 6 carbon atoms; a phenyl group; or a phenyl group substituted with an alkyl group having from 1 to 3 carbon atoms, an alkoxy group having from 1 to 3 carbon atoms, a halogen atom or a nitro group.

**8.** A process according to Claim 1, in which $R^1$ represents a group of formula $-NR^{9a}C(=Y)NR^{6j}NHZ$

in which:

$R^{9a}$ represents a hydrogen atom or a methyl group;

Y represents an oxygen atom or a sulfur atom;

$R^{6j}$ represents an alkyl group having from 1 to 4 carbon atoms; or a cycloalkyl group having from 3 to 6 carbon atoms;

Z represents a group of formula $-COOR^{7a}$ (in which: $R^{7a}$ represents an alkyl group having from 1 to 4 carbon atoms, a cycloalkyl group having from 3 to 6 carbon atoms or a benzyl group); a group of formula $-COR^{6k}$ (in which $R^{6k}$ represents: an alkyl group having from 1 to 4 carbon atoms; a cycloalkyl group having from 3 to 6 carbon atoms a phenyl group; or a phenyl group substituted with an alkyl group having from 1 to 3 carbon atoms, an alkoxy group having from 1 to 3 carbon atoms, a halogen atom or a nitro group), or a group of formula $-SO_2R^{6m}$ (in which $R^{6m}$ represents: an alkyl group having from 1 to 4 carbon atoms; a phenyl group; or a phenyl group substituted with an alkyl group haveing from 1 to 3 carbon atoms, an alkoxy group having from 1 to 3 carbon atoms, a halogen atom or a nitro group).

**9.** A process according to Claim 1, in which $R^1$ represents a group of formula $-NR^{9a}C(=NR^{11a})NHR^{11b}$

in which:

$R^{9a}$ represents a hydrogen atom or a methyl group;

$R^{11a}$ and $R^{11b}$ are the same or different and each represents: a hydrogen atom; an alkyl group having from 1 to 4 carbon atoms; a phenyl group; a phenyl group substituted with an alkyl group having from 1 to 3 carbon atoms, an alkoxy group having from 1 to 3 carbon atoms, a halogen atom or a nitro group; a group of formula $-COOR^{7b}$ (in which $R^{7b}$ represents: an alkyl group having from 1 to 4 carbon atoms; a cycloalkyl group having from 3 to 6 carbon atoms; or a benzyl group); or a group of formula $-COR^{6n}$ (in which $R^{6n}$ represents: an alkyl group having from 1 to 4 carbon atoms; a cycloalkyl group having from 3 to 6 carbon atoms; a phenyl group; or a phenyl group substituted with an alkyl group having from 1 to 3 carbon atoms, an alkoxy group having from 1 to 3 carbon atoms, a halogen atom or a nitro group).

**10.** A process according to Claim 1, in which $R^1$ represents a group of formula $-NR^{9a}C(=NR^{11c})R^{6p}$

in which:

$R^{9a}$ represents a hydrogen atom or a methyl group;

$R^{11c}$ represents: a hydrogen atom; an alkyl group having from 1 to 4 carbon atoms; a phenyl group; a phenyl group substituted with an alkyl group having from 1 to 3 carbon atoms, an alkoxy group having from 1 to 3 carbon atoms, a halogen atom or a nitro group; a group of formula $-COOR^{7c}$ (in which $R^{7c}$ represents: an alkyl group having from 1 to 4 carbon atoms; a cycloalkyl group having from 3 to 6 carbon atoms; or a benzyl group); or a group of formula $-COR^{6q}$ (in which $R^{6q}$ represents: an alkyl group having from 1 to 4 carbon atoms; a cycloalkyl group having from 3 to 6 carbon atoms; a phenyl group; or a phenyl group substituted with an alkyl group having from 1 to 3 carbon atoms, an alkoxy group having from 1 to 3 carbon atoms, a halogen atom or a nitro group);

$R^{6p}$ represents: an alkyl group having from 1 to 4 carbon atoms; a cycloalkyl group having from 3 to 6 carbon atoms; a phenyl group; or a phenyl group substituted with an alkyl group having from 1 to 3 carbon atoms, an alkoxy group having from 1 to 3 carbon atoms, a halogen atom or a nitro group).

**11.** A process according to Claim 1, in which $R^1$ represents a group of formula $-NR^{9a}SO_mR^{6r}$

in which:

$R^{9a}$ represents a hydrogen atom or a methyl group;

$R^{6r}$ represents: an alkyl group having from 1 to 4 carbon atoms; an alkyl group substituted with a cyano group; a phenyl group; or a phenyl group substituted with an alkyl group having from 1 to 3 carbon atoms, an alkoxy group having from 1 to 3 carbon atoms, a halogen atom or a nitro group; and

m is 1 or 2.

**12.** A process according to any one of Claims 1 to 11, in which A represents a group of formula:

$-CHR^2-CHR^3-CHR^4-$

in which $R^2$, $R^3$ and $R^4$ are the same or different and each represents a hydrogen atom or a methyl group.

**13.** A process according to any one of Claims 1 to 11, in which A represents a group of formula:

$-CR^2=CR^3-CHR^4-$

in which $R^2$, $R^3$ and $R^4$ are the same or different and each represents a hydrogen atom or a methyl group.

**14.** A process according to any one of the preceding Claims, in which the substituent $R^1$ is at the meta or the ortho position.

**15.** A process according to Claim 1, in which A represents a group of formula:

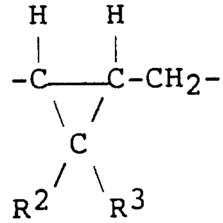

in which $R^2$ and $R^3$ are the same or different and each represents a hydrogen atom, a chlorine atom or a methyl group.

**17.** A process according to any one of Claims 1 to 16, in which $R^5$ represents an ethyl group.

**18.** A process according to any one of Claims 1 to 16, in which the starting material is a mixture of a compound in which $R^5$ represents an ethyl group and the corresponding compound in which $R^5$ represents a methyl group, to prepare a mixture of compounds of formula (I), in one of which $R^5$ represents an ethyl group and in the other of which $R^5$ represents a methyl group,.

**19.** A process according to any one of the preceding Claims, in which $R^3$ and $R^4$ each represent hydrogen atoms.

**20.** A process according to any one of the preceding Claims, in which X represents a hydroxy group.

**21.** A process according to Claim 1, in which the compound prepared is:
13-(3-aminocinnamyloxy)milbemycin $A_4$
13-(2-aminocinnamyloxy)milbemycin $A_4$
13-(3-acetamidocinnamyloxy)milbemycin $A_4$
13-[3-(3-methylthioureido)cinnamyloxy]milbemycin $A_4$
13-(3-cyanoacetamidocinnamyloxy)milbemycin $A_4$
13-(3-methanesulphonylaminocinnamyloxy)milbemycin $A_4$
13-[3-N-(methanesulphonyl)methylaminocinnamyloxy]milbemycin $A_4$
13-(3-methylaminocinnamyloxy)milbemycin $A_4$ and
13-[3-(3,3-dimethylcarbazoylamino)cinnamyloxy]milbemycin $A_4$,
or a pharmaceutically acceptable salt thereof.

**22.** A process according to Claim 1, in which the compound prepared is:
13-(3-cyanoacetamidocinnamyloxy)milbemycin $A_4$;
13-(3-methanesulphonylaminocinnamyloxy)milbemycin $A_4$;
13-[3-N-(methanesulphonyl)methylaminocinnamyloxy]milbemycin $A_4$;
or a pharmaceutically acceptable salt thereof.

**23.** The use of a compound of formula (I) or a salt or ester thereof as defined in any one of Claims 1 to 23 for the manufacture of a medicament for the treatment of animals parasitized by helminths, acarids or insects.

**24.** The use according to Claim 23, in which said compound is:
13-(3-aminocinnamyloxy)milbemycin $A_4$
13-(2-aminocinnamyloxy)milbemycin $A_4$
13-(3-acetamidocinnamyloxy)milbemycin $A_4$
13-[3-(3-methylthioureido)cinnamyloxy]milbemycin $A_4$
13-(3-cyanoacetamidocinnamyloxy)milbemycin $A_4$
13-(3-methanesulphonylaminocinnamyloxy)milbemycin $A_4$
13-[3-N-(methanesulphonyl)methylaminocinnamyloxy]milbemycin $A_4$
13-(3-methylaminocinnamyloxy)milbemycin $A_4$ and
13-[3-(3,3-dimethylcarbazoylamino)cinnamyloxy]milbemycin $A_4$,
or a pharmaceutically acceptable salt thereof.

**Patentansprüche**
**Patentansprüche für folgende Vertragsstaaten : GB, DE, FR, IT, CH, DK, BE, NL, SE, LU, AT**

**1.** Verbindungen der Formel (I)

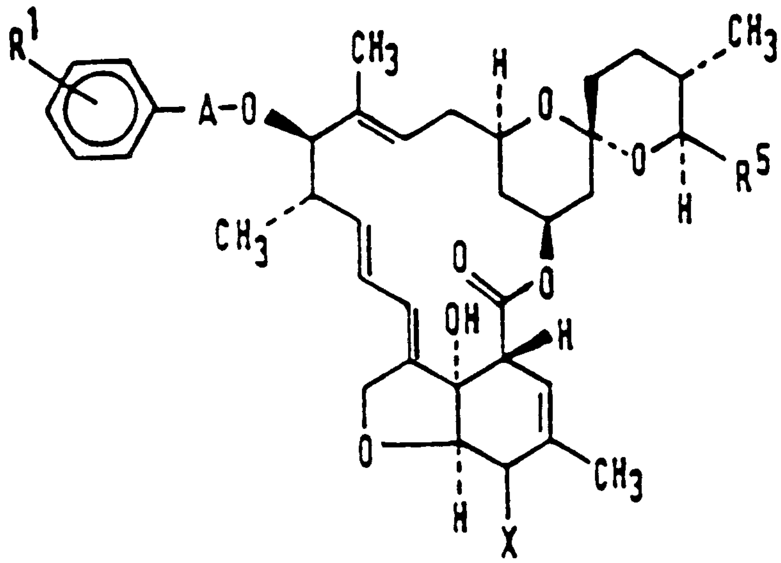

(I)

worin:
$R^1$ bedeutet: eine Wasserstoffatom, ein Halogenatom, eine Cyangruppe, eine Nitrogruppe, eine Alkylgruppe mit 1 bis 4 Kohlenstoffatomen, die unsubstituiert ist oder mit mindestens einem der Substituenten (a) substituiert ist, eine Alkoxygruppe mit 1 bis 4 Kohlenstoffatomen, eine Alkoxyalkoxygruppe mit insgesamt 2 bis 6 Kohlenstoffatomen oder eine Gruppe einer der folgenden Formeln:

$-(CH_2)_nNHR^9$
$-(CH_2)_nNR^9COR^6$
$-(CH_2)_nNR^9COCOR^6$
$-(CH_2)_nNR^9COCOOR^7$
$-(CH_2)_nNR^9CHR^6NHCOR^6$
$-(CH_2)_nNR^9CHR^6NHCONHR^6$
$-(CH_2)_nNR^9CHR^6NHCOOR^7$
$-(CH_2)_nNR^9C(=Y)YR^6$
$-(CH_2)_nNR^9C(=Y)NR^6R^6$
$-(CH_2)_nNR^9C(=Y)NR^6NR^6R^6$
$-(CH_2)_nNR^9C(=Y)NR^6NHZ$
$-(CH_2)_nNR^9C(=NR^{11})NHR^{11}$
$-(CH_2)_nNR^9C(=NR^{11})R^6$
$-(CH_2)_nNR^9SO_mR^6$
$-CONHR^6$

oder

$-COOR^7$

worin:
m 1 oder 2 ist,
n 0, 1 oder 2 ist,
$R^6$ Wasserstoffatom eine Alkylgruppe mit 1 bis 8 Kohlen stoffatomen, eine substituierte Alkylgruppe mit 1 bis 8 Kohlenstoffatomen, die mit mindestens einem der Substituenten (b) substituiert ist, eine aliphatische Kohlenwasserstoffgruppe mit 2 bis 8 Kohlenstoffatomen, die eine oder zwei Kohlenstoff-Kohlenstoff-Doppelbindungen oder -Dreifachbindungen hat, eine Cycloalkylgruppe mit 2 bis 8 Kohlenstoffatomen, eine substituierte Cycloalkylgruppe mit 2 bis 8 Kohlenstoffatomen, die mit mindestens einem der Substituenten (c) substituiert ist, eine Arylgruppe mit 6 bis 14 Ringkohlenstoffatomen, die unsubstituiert ist, oder die mit mindestens einem der Substituenten (c) substituiert ist, eine Aryloxygruppe mit 6 bis 14 Ringkohlenstoffatomen, die unsubstituiert ist, oder die mit mindestens einem der Substituenten (c) substituiert ist oder eine Arylthiogruppe mit 6 bis 14 Ringkohlenstoffatomen, die unsubstituiert ist oder die mit mindestens einem der Substituenten (c) substituiert ist, bedeutet,

$R^7$ eine Alkylgruppe mit 1 bis 4 Kohlenstoffatomen, eine Cycloalkylgruppe mit 3 bis 7 Kohlenstoffatomen oder eine Aralkylgrrnppe, deren Alkylgruppe 1 bis 4 Kohlenstoffatome hat und mit 1 bis 3 Arylgruppen substituiert ist, welche 6 bis 10 Ringkohlenstoffatome haben und welche unsubstituiert sind oder mit mindestens einem Substituenten (c) substituiert sind,

$R^9$ ein Wasserstoffatom oder eine Alkylgruppe mit 1 bis 4 Kohlenstoffatomen bedeutet,

$R^{11}$ irgendeine der vorstehend für $R^6$ definierten Gruppen oder Atome darstellt oder eine Cyangruppe, eine Nitrogruppe, eine Gruppe der Formel $-COOR^7$ (wobei $R^7$ wie oben definiert ist) oder eine Gruppe der Formel $-COR^6$ (wobei $R^6$ wie oben definiert ist) bedeutet,

Y ein Sauerstoffatom oder ein Schwefelatom bedeutet und, wenn zwei oder mehr durch Y dargestellte Gruppen vorhanden sind, diese gleich oder verschieden voneinander sind,

Z eine Gruppe der Formel $-COOR^7$ (wobei $R^7$ wie oben definiert ist), eine Gruppe der Formel $-COR^6$ (wobei $R^6$ wie oben definiert ist) oder eine Gruppe der Formel $-SO_2R^6$ (wobei $R^6$ wie oben definiert ist) bedeutet,

A eine Gruppe einer der nachstehenden Formeln bedeutet:

$-CHR^2-CHR^3-CHR^4-$

$-CR^2=CR^3-CHR^4-$

oder

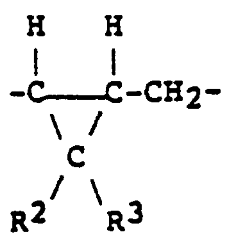

worin $R^2$, $R^3$ und $R^4$ gleich oder verschieden sind und jeweils ein Wasserstoffatom, ein Halogenatom, eine Alkylgruppe mit 1 bis 4 Kohlenstoffatomen oder eine Alkoxygruppe mit 1 bis 4 Kohlenstoffatomen bedeuten,

$R^5$ eine Methylgruppe, eine Ethylgruppe, eine Isopropylgruppe oder eine sec.-Butylgruppe bedeutet und

X eine Hydroxygruppe, eine Alkanoyloxygruppe mit 1 bis 5 Kohlenstoffatomen, die unsubstituiert ist oder mit mindestens einem der Substituenten (d) substituiert ist oder eine Hydroxyiminogruppe bedeutet;

Substituenten (a):

Halogenatome, Alkoxygruppen mit 1 bis 4 Kohlenstoffatomen, Alkylthiogruppen mit 1 bis 4 Kohlenstoffatomen und Alkanoyloxygruppen mit 1 bis 5 Kohlenstoffatomen,

Substituenten (b):

Cycloalkylgruppen mit 3 bis 8 Kohlenstoffatomen, Alkoxygruppen mit 1 bis 4 Kohlenstoffatomen, Alkylthiogruppen mit 1 bis 4 Kohlenstoffatomen, Cyanalkylthiogruppen mit 2 bis 5 Kohlenstoffatomen, Alkoxycarbonylgruppen mit 2 bis 5 Kohlenstoffatomen, Halogenatome, Cyangruppen, Nitrogruppen, Aminogruppen, Arylgruppen mit 6 bis 14 Ringkohlenstoffatomen die unsubstituiert sind oder mit mindestens einem der Substituenten (c) substituiert sind, eine Pyridyl-, Thienyl-, Furyl-, Pyrrolyl-, Imidazolyl-, Triazolyl-, Tetrazolyl-, Thiazolyl-, Oxazolyl-, Indolyl-Gruppe oder eine derartige Gruppe, die mit mindestens einem der Substituenten (c) substituiert ist, Aryloxygruppen mit 6 bis 14 Ringkohlenstoffatomen, die unsubstituiert sind oder mit mindestens einem der Substituenten (c) substituiert sind und Arylthiogruppen mit 6 bis 14 Ringkohlenstoffatomen, die unsubstituiert sind oder mit mindestens einem der Substituenten (c) substituiert sind,

Substituenten (c):

Alkylgruppen mit 1 bis 4 Kohlenstoffatomen, Alkoxygruppen mit 1 bis 4 Kohlenstoffatomen, Alkylthiogruppen mit 1 bis 4 Kohlenstoffatomen, Alkanoyloxygruppen mit 1 bis 5 Kohlenstoffatomen, Alkoxycarbonylgruppen mit 2 bis 5 Kohlenstoffatomen, Halogenatome, Cyangruppen, Nitrogruppen, Aminogruppen, Alkylaminogruppen, deren Alkylteil 1 bis 4 Kohlenstoffatome hat, Dialkylaminogruppen, in denen jeder Alkylteil gleich oder verschieden ist und jeweils eine Alkylgruppe mit 1 bis 4 Kohlenstoffatomen bedeutet, eine Carbamoylgruppe, eine Alkylcarbamoylgruppe, in der der Alkylteil 1 bis 4 Kohlenstoffatome hat, eine Dialkylcarbamoylgruppe, in der jeder Alkylteil 1 bis 4 Kohlenstoffatome hat und Alkanoylaminogruppen mit 1 bis 5 Kohlenstoffatomen,

Substituenten (d):

Halogenatome, Alkoxygruppen mit 1 bis 4 Kohlenstoffatomen, Alkoxycarbonylgruppen mit 2 bis 5 Kohlenstoffatomen und Carboxygruppen,

und Salze und Ester dieser Verbindungen.

**2.** Verbindungen nach Anspruch 1, worin $R^1$ bedeutet: ein Wasserstoffatom, eine Alkylgruppe mit 1 bis 3 Kohlenstoffatomen, eine Alkoxygruppe mit 1 bis 3 Kohlenstoffatomen, ein Fluor- oder Chloratom oder die Nitro- oder Aminogruppe.

**3.** Verbindungen nach Anspruch 1, worin $R^1$ eine Gruppe der Formel $-NR^{9a}COR^{6a}$ bedeutet, in der:

$R^{9a}$ ein Wasserstoffatom oder eine Methylgruppe bedeutet und

$R^{6a}$ bedeutet: ein Wasserstoffatom, eine Alkylgruppe mit 1 bis 4 Kohlenstoffatomen, eine Cycloalkylgruppe mit 3 bis 5 Kohlenstoffatomen, eine substituierte Alkylgruppe mit 1 bis 3 Kohlenstoffatomen, die mit einem Halogenatom, einer Cyangruppe, einer Alkoxygruppe mit 1 bis 3 Kohlenstoffatomen, einer Alkylthiogruppe mit 1 bis 3 Kohlenstoffatomen, einer Cyanmethylthiogruppe oder einer Phenoxygruppe substituiert ist, eine Alkenylgruppe mit 2 bis 4 Kohlenstoffatomen, eine Phenylgruppe oder Phenylgruppe, die mit einer Alkylgruppe mit 1 bis 3 Kohlenstoffatomen, einer Alkoxygruppe mit 1 bis 3 Kohlenstoffatomen, einem Halogenatom oder einer Nitrogruppe substituiert ist.

**4.** Verbindungen nach Anspruch 1, worin $R^1$ eine Gruppe der Formel $-NR^{9a}COCOR^{6b}$ bedeutet, in der:

$R^{9a}$ ein Wasserstoffatom oder eine Methylgruppe bedeutet und

$R^{6b}$ bedeutet: ein Wasserstoffatom, eine Alkylgruppe mit 1 bis 4 Kohlenstoffatomen, eine Cycloalkylgruppe mit 3 bis 5 Kohlenstoffatomen, eine Alkenylgruppe mit 2 bis 4 Kohlenstoffatomen, eine Phenylgruppe oder eine Phenylgruppe, die mit einer Alkylgruppe mit 1 bis 3 Kohlenstoffatomen, einer Alkoxygruppe mit 1 bis 3 Kohlenstoffatomen, einem Halogenatom oder einer Nitrogruppe substituiert ist.

**5.** Verbindungen nach Anspruch 1, worin $R^1$ eine Gruppe der Formel $-NR^{9a}C(=Y)YR^{6c}$ bedeutet, worin:

$R^{9a}$ ein Wasserstoffatom oder eine Methylgruppe bedeutet,

Y ein Sauerstoffatom bedeutet und

$R^{6c}$ bedeutet: eine Alkylgruppe mit 1 bis 4 Kohlenstoffatomen, eine Alkylgruppe mit 1 bis 4 Kohlenstoffatomen, die mit einem Halogenatom oder einer Alkoxygruppe mit 1 bis 3 Kohlenstoffatomen substituiert ist, eine Vinylgruppe, eine Allylgruppe, eine Benzylgruppe, eine Methoxybenzylgruppe, eine Nitrobenzylgruppe oder eine Phenylgruppe.

**6.** Verbindungen nach Anspruch 1, worin $R^1$ eine Gruppe der Formel $-NR^{9a}C(=y)NR^{6d}R^{6e}$ bedeutet, worin:

$R^{9a}$ ein Wasserstoffatom oder eine Methylgruppe bedeutet,

Y ein Sauerstoffatom oder ein Schwefelatom bedeutet und

$R^{6d}$ und $R^{6e}$ gleich oder verschieden sind und jeweils ein Wasserstoffatom, eine Alkylgruppe mit 1 bis 4 Kohlenstoffatomen, eine Cycloalkylgruppe mit 3 bis 6 Kohlenstoffatomen, eine Phenylgruppe oder eine Phenylgruppe, die mit einer Alkylgruppe mit 1 bis 3 Kohlenstoffatomen, einer Alkoxygruppe mit 1 bis 3 Kohlenstoffatomen, einem Halogenatom oder einer Nitrogruppe substituiert ist, bedeuten.

**7.** Verbindungen nach Anspruch 1, worin $R^1$ eine Gruppe der Formel $-NR^{9a}C(=Y)NR^{6f}NR^{6g}R^{6h}$ bedeutet worin:

$R^{9a}$ ein Wasserstoffatom oder eine Methylgruppe bedeutet,

Y ein Sauerstoffatom oder ein Schwefelatom bedeutet und

$R^{6f}$, $R^{6g}$ und $R^{6h}$ gleich oder verschieden sind und jeweils ein Wasserstoffatom, eine Alkylgruppe mit 1 bis 4 Kohlenstoffatomen, eine Cycloalkylgruppe mit 3 bis 6 Kohlenstoffatomen, eine Phenylgruppe oder eine Phenylgruppe die mit einer Alkylgruppe mit 1 bis 3 Kohlenstoffatomen, einer Alkoxygruppe mit 1 bis 3 Kohlenstoffatomen, einem Halogenatom oder einer Nitrogruppe substituiert ist, darstellen.

**8.** Verbindungen nach Anspruch 1, worin $R^1$ eine Gruppe der Formel $-NR^{9a}C(=Y)NR^{6j}NHZ$ bedeutet, worin:

$R^{9a}$ ein Wasserstoffatom oder eine Methylgruppe bedeutet,

Y ein Sauerstoffatom oder ein Schwefelatom bedeutet,

$R^{6j}$ eine Alkylgruppe mit 1 bis 4 Kohlenstoffatomen oder eine Cycloalkylgruppe mit 3 bis 6 Kohlenstoffatomen bedeutet,

Z eine Gruppe der Formel $-COOR^{7a}$ (in der $R^{7a}$ eine Alkylgruppe mit 1 bis 4 Kohlenstoffatomen, eine Cycloalkylgruppe mit 3 bis 6 Kohlenstoffatomen oder eine Benzylgruppe darstellt), eine Gruppe der Formel $-COR^{6k}$ (in der $R^{6k}$ bedeutet: eine Alkylgruppe mit 1 bis 4 Kohlenstoffatomen, eine Cycloalkylgruppe mit 3 bis 6 Kohlenstoffatomen, eine Phenylgruppe oder eine Phenylgruppe, die mit einer Alkylgruppe mit 1 bis 3 Kohlenstoffatomen, einer Alkoxygruppe mit 1 bis 3 Kohlenstoffatomen, einem Halogenatom oder einer Nitrogruppe substituiert ist), oder eine Gruppe der Formel $-SO_2R^{6m}$ (in der $R^{6m}$ bedeutet: eine Alkylgruppe mit 1 bis 4 Kohlenstoffatomen, eine Phenylgruppe oder Phenylgruppe, die mit einer Alkylgruppe mit 1 bis 3 Kohlenstoffatomen, einer Alkoxygruppe mit 1 bis 3 Kohlenstoffatomen, einem Halogenatom oder einer Nitrogruppe substituiert ist), bedeutet.

**9.** Verbindungen nach Anspruch 1, worin $R^1$ eine Gruppe der Formel $-NR^{9a}C(=NR^{11a})NHR^{11b}$ bedeutet, worin:

$R^{9a}$ ein Wasserstoffatom oder eine Methylgruppe bedeutet,

$R^{11a}$ und $R^{11b}$ gleich oder verschieden sind und jeweils bedeuten: ein Wasserstoffatom, eine Alkylgruppe mit 1 bis 4 Kohlenstoffatomen, eine Phenylgruppe, eine Phenylgruppe, die mit einer Alkylgruppe mit 1 bis 3 Kohlenstoffatomen, einer Alkoxygruppe mit 1 bis 3 Kohlenstoffatomen, einem Halogenatom oder einer Nitrogruppe substituiert ist, eine Gruppe der Formel $-COOR^{7b}$ (in der $R^{7b}$ eine Alkylgruppe mit 1 bis 4 Kohlenstoffatomen, eine Cycloalkylgruppe mit 3 bis 6 Kohlenstoffatomen oder eine Benzylgruppe bedeutet) oder eine Gruppe der Formel $-COR^{6n}$ (in der $R^{6n}$ eine Alkylgruppe mit 1 bis 4 Kohlenstoffatomen, eine Cycloalkylgruppe mit 3 bis 6 Kohlenstoffatomen, eine Phenylgruppe oder eine Phenylgruppe, die mit einer Alkylgruppe mit 1 bis 3 Kohlenstoffatomen, einer Alkoxygruppe mit 1 bis 3 Kohlenstoffatomen, einem Halogenatom oder einer Nitrogruppe substituiert ist, bedeutet).

**10.** Verbindungen nach Anspruch 1, worin $R^1$ eine Gruppe der Formel $-NR^{9a}C(=NR^{11c})R^{6p}$ bedeutet, worin:

$R^{9a}$ ein Wasserstoffatom oder eine Methylgruppe bedeutet,

$R^{11c}$ bedeutet: ein Wasserstoffatom, eine Alkylgruppe mit 1 bis 4 Kohienstoffatomen, eine Phenylgruppe, eine Phenylgruppe, die mit einer Alkylgruppe mit 1 bis 3 Kohlenstoffatomen, einer Alkoxygruppe mit 1 bis 3 Kohlenstoffatomen, einem Halogenatom oder einer Nitrogruppe substituiert ist, eine Gruppe der Formel $-COOR^{7c}$ (in der $R^{7c}$ eine Alkylgruppe mit 1 bis 4 Kohlenstoffatomen, eine Cycloalkylgruppe mit 3 bis 6 Kohlenstoffatomen oder eine Benzylgruppe bedeutet) oder eine Gruppe der Formel $-COR^{6q}$ (in der $p^{6q}$ eine Alkylgruppe mit 1 bis 4 Kohlenstoffatomen, eine Cycloalkylgruppe mit 3 bis 6 Kohlenstoffatomen, eine Phenylgruppe oder eine Phenylgruppe bedeutet, die mit einer Alkylgruppe mit 1 bis 3 Kohlenstoffatomen, einer Alkoxygruppe mit 1 bis 3 Kohlenstoffatomen, einem Halogenatom oder einer Nitrogruppe substituiert ist),

$R^{6p}$ bedeutet: eine Alkylgruppe mit 1 bis 4 Kohlenstoffatomen, eine Cycloalkylgruppe mit 3 bis 6 Kohlenstoffatomen, eine Phenylgruppe oder eine Phenylgruppe, die mit einer Alkylgruppe mit 1 bis 3 Kohlenstoffatomen, einer Alkoxygruppe mit 1 bis 3 Kohlenstoffatomen, einem Halogenatom oder einer Nitrogruppe substituiert ist.

**11.** Verbindungen nach Anspruch 1, worin $R^1$ eine Gruppe der Formel $-NR^{9a}SO_mR^{6r}$ bedeutet, worin:

$R^{9a}$ ein Wasserstoffatom oder eine Methylgruppe bedeutet,

$R^{6r}$ eine Alkylgruppe mit 1 bis 4 Kohlenstoffatomen, eine mit einer Cyangruppe substituierte Alkylgruppe, eine Phenylgruppe oder eine Phenylgruppe, die mit einer Alkylgruppe mit 1 bis 3 Kohlenstoffatomen, einer Alkoxygruppe mit 1 bis 3 Kohlenstoffatomen, einem Halogenatom oder einer Nitrogruppe substituiert ist, bedeutet und

m für 1 oder 2 steht.

**12.** Verbindungen nach einem der Ansprüche 1 bis 11, worin A eine Gruppe der Formel

$$-CHR^2-CHR^3-CHR^4-$$

bedeutet, in der $R^2$, $R^3$ und $R^4$ gleich oder verschieden sind und jeweils ein Wasserstoffatom oder eine Methylgruppe bedeuten.

**13.** Verbindungen nach einem der Ansprüche 1 bis 11, worin A eine Gruppe der Formel

$$-CR^2=CR^3-CHR^4-$$

bedeutet, worin $R^2$, $R^3$ und $R^4$ gleich oder verschieden sind und jeweils ein Wasserstoffatom oder eine Methylgruppe bedeuten.

**14.** Verbindungen nach einem der vorhergehenden Patentansprüche, worin der Substituent $R^1$ in meta- oder ortho-Stellung ist.

**15.** Verbindungen nach Anspruch 1, worin A eine Gruppe der Formel

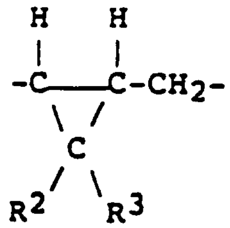

bedeutet, in der $R^2$ und $R^3$ gleich oder verschieden sind und jeweils ein Wasserstoffatom, ein Chloratom oder eine Methylgruppe bedeuten.

**17.** Verbindungen nach einem der Ansprüche 1 bis 16, worin $R^5$ eine Ethylgruppe bedeutet.

**18.** Verbindungen nach einem der Ansprüche 1 bis 16, die ein Gemisch aus einer Verbindung, in der $R^5$ eine Ethylgruppe bedeutet, und der entsprechenden Verbindung, in der $R^5$ eine Methylgruppe bedeutet, darstellt.

**19.** Verbindungen nach einem der vorhergehenden Patentansprüche, worin $R^3$ und $R^4$ jeweils Wasserstoffatome bedeuten.

**20.** Verbindungen nach einem der vorhergehenden Patentansprüche, worin X eine Hydroxygruppe bedeutet.

**21.** 13-(3-Cyanoacetamidocinnamyloxy)milbemycin $A_4$ und dessen pharmazeutisch geeignete Salze.

**22.** 13-(3-Methansulfonylaminocinnamyloxy)milbemycin $A_4$ und dessen pharmazeutisch geeignete Salze.

**23.** 13-[3-N-(Methansulfonyl)methylaminocinnamyloxy]-milbemycin $A_4$ und dessen pharmazeutisch geeignete Salze.

**24.** Anthelmintisch, acarizid und insektizid wirksame Zusammensetzung, enthaltend mindestens eine Verbindung nach einem der vorhergehenden Patentansprüche im Gemisch mit einem in der Pharmazie, Landwirtschaft, Veterinärmedizin oder Gartenbau geeigneten Trägermaterial oder Verdünnungsmittel.

**25.** Zusammensetzung nach Anspruch 24, wobei diese Verbindung
13-(3-Aminocinnamyloxy)milbemycin $A_4$
13-(2-Aminocinnamyloxy)milbemycin $A_4$
13-(3-Acetamidocinnamyloxy)milbemycin $A_4$
13-[3-(3-Methylthioureido)cinnamyloxy]milbemycin $A_4$
13-(3-Cyanoacetamidocinnamyloxy)milbemycin $A_4$
13-(3-Methansulfonylaminocinnamyloxy)milbemycin $A_4$
13-[3-N-(Methansulfonyl)-methylaminocinnamyloxy]milbemycin $A_4$
13-(3-Methylaminocinnamyloxy)milbemycin $A_4$ bzw.
13-[3-(3,3-Dimethylcarbazoylamino)cinnamyloxy]milbemycin $A_4$
oder ein pharmazeutisch geeignetes Salz davon ist.

**26.** Verbindung nach einem der Ansprüche 1 bis 23 zur therapeutischen Verwendung.

**27.** Verwendung einer Verbindung nach einem der Ansprüche 1 bis 23 zur Herstellung eines Arzneimittels zur Behandlung von Tieren, die an Parasitenbefall durch Würmer, Milben oder Insekten leiden.

**28.** Verfahren nach Anspruch 26 oder 27, wobei diese Verbindung
13-(3-Aminocinnamyloxy)milbemycin $A_4$
13-(2-Aminocinnamyloxy)milbemycin $A_4$
13-(3-Acetamidocinnamyloxy)milbemycin $A_4$
13-[3-(3-Methylthioureido)cinnamyloxy]milbemycin $A_4$
13-(3-Cyanoacetamidocinnamyloxy)milbemycin $A_4$
13-(3-Methansulfonylaminocinnamyloxy)milbemycin $A_4$
13-[3-N-(Methansulfonyl)-methylaminocinnamyloxy]milbemycin $A_4$
13-(3-Methylaminocinnamyloxy)milbemycin $A_4$ bzw.
13-[3-(3,3-Dimethylcarbazoylamino)cinnamyloxy]milbemycin $A_4$
oder ein pharmazeutisch geeignetes Salz davon ist.

**29.** Verwendung einer Verbindung nach einem der Ansprüche 1 bis 23 für therapeutische Zwecke.

**30.** Verwendung einer Verbindung nach einem der Ansprüche 1 bis 23 zur Herstellung eines Arzneimittels zur Behandlung von Tieren, die an Parasitenbefall durch Würmer, Milben oder Insekten leiden.

**31.** Verfahren zur Herstellung einer Verbindung nach einem der Ansprüche 1 bis 23, das darin besteht, daß eine Verbindung der Formel (II) in beliebiger Reihenfolge den Stufen (a) und (b) unterworfen wird:

(II)

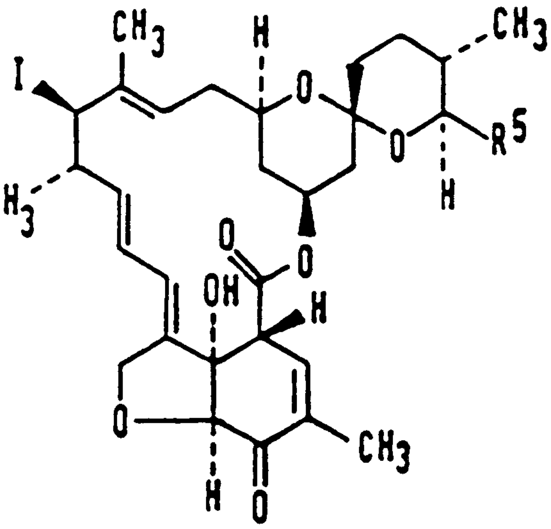

worin $R^5$ wie oben definiert ist,

(a) der Umsetzung mit einem Alkohol der Formel (IIa):

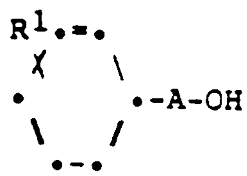

(IIa)

worin $R^1$ wie in Anspruch 1 definiert ist, und

(b) entweder der Umsetzung ($b^i$) mit einem Reduktionsmittel unter Reduktion des Sauerstoffatoms in 5-Stellung zu einer Hydroxygruppe wonach gewunschtenfalls die Umsetzung mit einem Acylierungsmittel folgt unter Bildung einer Verbindung der Formel (I), in der X eine Alkanoyloxygruppe mit 1 bis 5 Kohlenstoffatomen bedeutet, die unsubstituiert ist oder die mit mindestens einem der Substituenten (d) substituiert ist, oder

($b^{ii}$) der Umsetzung mit Hydroxylamin oder einem Salz davon unter Bildung einer Verbindung der Formel (I), in der X eine Hydroxyiminogruppe bedeutet,

wonach gewünschtenfalls das erhaltene Produkt einer oder beiden Stufen (c) und (d) unterworfen wird:

(c) Umwandeln einer durch $R^1$ dargestellten Gruppe in irgendeine andere durch $R^1$ dargestellte Gruppe, und

(d) Überführen des Produkts in ein Salz oder einen Ester.

**Patentansprüche für folgende Vertragsstaaten : ES, GR**

**1.** Verfahren zur Herstellung von Verbindungen der Formel (I)

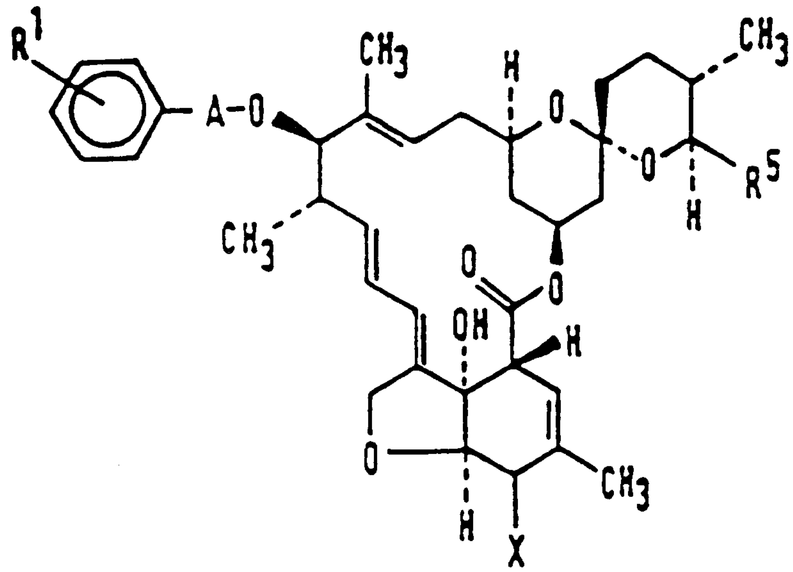

(I)

worin:
$R^1$ bedeutet: eine Wasserstoffatom, ein Halogenatom, eine Cyangruppe, eine Nitrogruppe, eine Alkylgruppe mit 1 bis 4 Kohlenstoffatomen, die unsubstituiert ist oder mit mindestens einem der Substituenten (a) substituiert ist, eine Alkoxygruppe mit 1 bis 4 Kohlenstoffatomen, eine Alkoxyalkoxygruppe mit insgesamt 2 bis 6 Kohlenstoffatomen oder eine Gruppe einer der folgenden Formeln:

$-(CH_2)_nNHR^9$
$-(CH_2)_nNR^9COR^6$
$-(CH_2)_nNR^9COCOR^6$
$-(CH_2)_nNR^9COCOOR^7$
$-(CH_2)_nNR^9CHR^6NHCOR^6$
$-(CH_2)_nNR^9CHR^6NHCONHR^6$
$-(CH_2)_nNR^9CHR^6NHCOOR^7$
$-(CH_2)_nNR^9C(=Y)YR^6$
$-(CH_2)_nNR^9C(=Y)NR^6R^6$
$-(CH_2)_nNR^9C(=Y)NR^6NR^6R^6$
$-(CH_2)_nNR^9C(=Y)NR^6NHZ$
$-(CH_2)_nNR^9C(=NR^{11})NHR^{11}$
$-(CH_2)_nNR^9C(=NR^{11})R^6$
$-(CH_2)_nNR^9SO_mR^6$
$-CONHR^6$

oder

$-COOR^7$

worin:
m 1 oder 2 ist,
n 0, 1 oder 2 ist,
$R^6$ Wasserstoffatom eine Alkylgruppe mit 1 bis 8 Kohlenstoffatomen, eine substituierte Alkylgruppe mit 1 bis 8 Kohlenstoffatomen, die mit mindestens einem der Substituenten (b) substituiert ist, eine aliphatische Kohlenwasserstoffgruppe mit 2 bis 8 Kohlenstoffatomen, die eine oder zwei Kohlenstoff-Kohlenstoff-Doppelbindungen oder -Dreifachbindungen hat, eine Cycloalkylgruppe mit 2 bis 8 Kohlenstoffatomen, eine substituierte Cycloalkylgruppe mit 2 bis 8 Kohlenstoffatomen, die mit mindestens

einem der Substituenten (c) substituiert ist, eine Arylgruppe mit 6 bis 14 Ringkohlenstoffatomen, die unsubstituiert ist, oder die mit mindestens einem der Substituenten (c) substituiert ist, eine Aryloxygruppe mit 6 bis 14 Ringkohlenstoffatomen, die unsubstituiert ist, oder die mit mindestens einem der Substituenten (c) substituiert ist oder eine Arylthiogruppe mit 6 bis 14 Ringkohlenstoffatomen, die unsubstituiert ist oder die mit mindestens einem der Substituenten (c) substituiert ist, bedeutet,

$R^7$ eine Alkylgruppe mit 1 bis 4 Kohlenstoffatomen, eine Cycloalkylgruppe mit 3 bis 7 Kohlenstoffatomen oder eine Alalkylgruppe, deren Alkylgruppe 1 bis 4 Kohlenstoffatome hat und mit 1 bis 3 Arylgruppen substituiert ist, welche 6 bis 10 Ringkohlenstoffatome haben und welche unsubstituiert sind oder mit mindestens einem Substituenten (c) substituiert sind,

$R^9$ ein Wasserstoffatom oder eine Alkylgruppe mit 1 bis 4 Kohlenstoffatomen bedeutet,

$R^{11}$ irgendeine der vorstehend für $R^6$ definierten Gruppen oder Atome darstellt oder eine Cyangruppe, eine Nitrogruppe, eine Gruppe der Formel $-COOR^7$ (wobei $R^7$ wie oben definiert ist) oder eine Gruppe der Formel $-COR^6$ (wobei $R^6$ wie oben definiert ist) bedeutet,

Y ein Sauerstoffatom oder ein Schwefelatom bedeutet und, wenn zwei oder mehr durch Y dargestellte Gruppen vorhanden sind, diese gleich oder verschieden voneinander sind,

Z eine Gruppe der Formel $-COOR^7$ (wobei $R^7$ wie oben definiert ist), eine Gruppe der Formel $-COR^6$ (wobei $R^6$ wie oben definiert ist) oder eine Gruppe der Formel $-SO_2R^6$ (wobei $R^6$ wie oben definiert ist) bedeutet,

A eine Gruppe einer der nachstehenden Formeln bedeutet:

$-CHR^2-CHR^3-CHR^4-$

$-CR^2=CR^3-CHR^4-$

oder

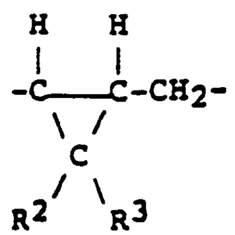

worin $R^2$, $R^3$ und $R^4$ gleich oder verschieden sind und jeweils ein Wasserstoffatom, ein Halogenatom, eine Alkylgruppe mit 1 bis 4 Kohlenstoffatomen oder eine Alkoxygruppe mit 1 bis 4 Kohlenstoffatomen bedeuten,

$R^5$ eine Methylgruppe, eine Ethylgruppe, eine Isopropylgruppe oder eine sec.-Butylgruppe bedeutet und X eine Hydroxygruppe, eine Alkanoyloxygruppe mit 1 bis 5 Kohlenstoffatomen, die unsubstituiert ist oder mit mindestens einem der Substituenten (d) substituiert ist oder eine Hydroxyiminogruppe bedeutet;

Substituenten (a):

Halogenatome, Alkoxygruppen mit 1 bis 4 Kohlenstoffatomen, Alkylthiogruppen mit 1 bis 4 Kohlenstoffatomen und Alkanoyloxygruppen mit 1 bis 5 Kohlenstoffatomen,

Substituenten (b):

Cycloalkylgruppen mit 3 bis 8 Kohlenstoffatomen, Alkoxygruppen mit 1 bis 4 Kohlenstoffatomen, Alkylthiogruppen mit 1 bis 4 Kohlenstoffatomen, Cyanalkylthiogruppen mit 2 bis 5 Kohlenstoffatomen, Alkoxycarbonylgruppen mit 2 bis 5 Kohlenstoffatomen, Halogenatome, Cyangruppen, Nitrogruppen, Aminogruppen, Arylgruppen mit 6 bis 14 Ringkohlenstoffatomen die unsubstituiert sind oder mit mindestens einem der Substituenten (c) substituiert sind, eine Pyridyl-, Thienyl-, Furyl-, Pyrrolyl-, Imidazolyl-, Triazolyl-, Tetrazolyl-, Thiazolyl-, Oxazolyl-, Indolyl-Gruppe oder eine derartige Gruppe, die mit mindestens einem der Substituenten (c) substituiert ist, Aryloxygruppen mit 6 bis 14 Ringkohlenstoffatomen, die unsubstituiert sind oder mit mindestens einem der Substituenten (c) substituiert sind und Arylthiogruppen mit 6 bis 14 Ringkohlenstoffatomen, die unsubstituiert sind oder mit mindestens einem der Substituenten (c) substituiert sind,

Substituenten (c):

Alkylgruppen mit 1 bis 4 Kohlenstoffatomen, Alkoxygruppen mit 1 bis 4 Kohlenstoffatomen, Alkylthiogruppen mit 1 bis 4 Kohlenstoffatomen, Alkanoyloxygruppen mit 1 bis 5 Kohlenstoffatomen, Alkoxycarbonylgruppen mit 2 bis 5 Kohlenstoffatomen, Halogenatome, Cyangruppen, Nitrogruppen, Aminogruppen, Alkylaminogruppen, deren Alkylteil 1 bis 4 Kohlenstoffatome hat, Dialkylaminogruppen, in denen jeder Alkylteil gleich oder verschieden ist und jeweils eine Alkylgruppe mit 1 bis 4 Kohlenstoffatomen bedeutet, eine Carbamoylgruppe, eine Alkylcarbamoylgruppe, in der der Alkylteil 1 bis 4 Kohlenstoffatome hat, eine Dialkylcarbamoylgruppe, in der jeder Alkylteil 1 bis 4 Kohlenstoffatome hat und Alkanoylaminogruppen mit 1 bis 5 Kohlenstoffatomen,

Substituenten (d):

Halogenatome, Alkoxygruppen mit 1 bis 4 Kohlenstoffatomen, Alkoxycarbonylgruppen mit 2 bis 5 Kohlenstoffatomen und Carboxygruppen,

und von Salzen und Estern dieser Verbindungen,

das darin besteht, daß eine Verbindung der Formel (II) in beliebiger Reihenfolge den Stufen (a) und (b) unterworfen wird:

(II)

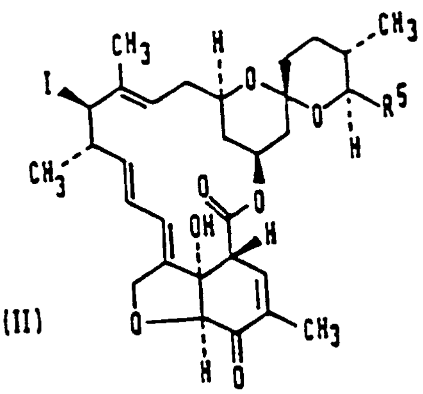

worin $R^5$ wie oben definiert ist,

(a) der Umsetzung mit einem Alkohol der Formel (IIa):

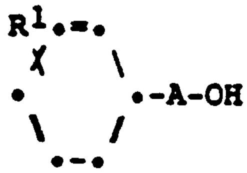

(IIa)

worin $R^1$ wie oben definiert ist, und

(b) entweder der Umsetzung ($b^i$) mit einem Reduktionsmittel unter Reduktion des Sauerstoffatoms in 5-Stellung zu einer Hydroxygruppe, wonach gewünschtenfalls die Umsetzung mit einem Acylierungsmittel folgt, unter Bildung einer Verbindung der Formel (I), in der X eine Alkanoyloxygruppe mit 1 bis 5 Kohlenstoffatomen bedeutet, die unsubstituiert ist oder die mit mindestens einem der Substituenten (d) substituiert ist, oder

($b^{ii}$) der Umsetzung mit Hydroxylamin oder einem Salz davon unter Bildung einer Verbindung der Formel (I), in der X eine Hydroxyiminogruppe bedeutet,

wonach gewilnschtenfalls das erhaltene Produkt einer oder beiden Stufen (c) und (d) unterworfen wird:

(c) Umwandeln einer durch $R^1$ dargestellten Gruppe in irgendeine andere durch $R^1$ dargestellte Gruppe, und

(d) Überführen des Produkts in ein Salz oder einen Ester.

**2.** Verfahren nach Anspruch 1, wobei $R^1$ bedeutet: ein Wasserstoffatom, eine Alkylgruppe mit 1 bis 3 Kohlenstoffatomen, eine Alkoxygruppe mit 1 bis 3 Kohlenstoffatomen, ein Fluor- oder Chloratom oder die Nitro- oder Aminogruppe.

**3.** Verfahren nach Anspruch 1, wobei $R^1$ eine Gruppe der Formel $-NR^{9a}COR^{6a}$ bedeutet, in der:

$R^{9a}$ ein Wasserstoffatom oder eine Methylgruppe bedeutet und

$R^{6a}$ bedeutet: ein Wasserstoffatom, eine Alkylgruppe mit 1 bis 4 Kohlenstoffatomen, eine Cycloalkylgruppe mit 3 bis 5 Kohlenstoffatomen, eine substituierte Alkylgruppe mit 1 bis 3 Kohlenstoffatomen, die mit einem Halogenatom, einer Cyangruppe, einer Alkoxygruppe mit 1 bis 3 Kohlenstoffatomen, einer

Alkylthiogruppe mit 1 bis 3 Kohlenstoffatomen, einer Cyanmethylthiogruppe oder einer Phenoxygruppe substituiert ist, eine Alkenylgruppe mit 2 bis 4 Kohlenstoffatomen, eine Phenylgruppe oder Phenylgruppe, die mit einer Alkylgruppe mit 1 bis 3 Kohlenstoffatomen, einer Alkoxygruppe mit 1 bis 3 Kohlenstoffatomen, einem Halogenatom oder einer Nitrogruppe substituiert ist.

**4.** Verfahren nach Anspruch 1, wobei $R^1$ eine Gruppe der Formel $-NR^{9a}COCOR^{6b}$ bedeutet, in der:
$R^{9a}$ ein Wasserstoffatom oder eine Methylgruppe bedeutet und
$R^{6b}$ bedeutet: ein Wasserstoffatom, eine Alkylgruppe mit 1 bis 4 Kohlenstoffatomen, eine Cycloalkylgruppe mit 3 bis 5 Kohlenstoffatomen, eine Alkenylgruppe mit 2 bis 4 Kohlenstoffatomen, eine Phenylgruppe oder eine Phenylgruppe, die mit einer Alkylgruppe mit 1 bis 3 Kohlenstoffatomen, einer Alkoxygruppe mit 1 bis 3 Kohlenstoffatomen, einem Kalogenatom oder einer Nitrogruppe substituiert ist.

**5.** Verfahren nach Anspruch 1, wobei $R^1$ eine Gruppe der Formel $-NR^{9a}C(=Y)YR^{6c}$ bedeutet, worin:
$R^{9a}$ ein Wasserstoffatom oder eine Methylgruppe bedeutet,
Y ein Sauerstoffatom bedeutet und
$R^{6c}$ bedeutet: eine Alkylgruppe mit 1 bis 4 Kohlenstoffatomen, eine Alkylgruppe mit 1 bis 4 Kohlenstoffatomen, die mit einem Halogenatom oder einer Alkoxygruppe mit 1 bis 3 Kohlenstoffatomen substituiert ist, eine Vinylgruppe, eine Allylgruppe, eine Benzylgruppe, eine Methoxybenzylgruppe, eine Nitrobenzylgruppe oder eine Phenylgruppe.

**6.** Verfahren nach Anspruch 1, wobei $R^1$ eine Gruppe der Formel $-NR^{9a}C(=Y)NR^{6d}R^{6e}$ bedeutet, worin:
$R^{9a}$ ein Wasserstoffatom oder eine Methylgruppe bedeutet,
Y ein Sauerstoffatom oder ein Schwefelatom bedeutet und
$R^{6d}$ und $R^{6e}$ gleich oder verschieden sind und jeweils ein Wasserstoffatom, eine Alkylgruppe mit 1 bis 4 Kohlenstoffatomen, eine Cycloalkylgruppe mit 3 bis 6 Kohlenstoffatomen, eine Phenylgruppe oder eine phenylgruppe, die mit einer Alkylgruppe mit 1 bis 3 Kohlenstoffatomen, einer Alkoxygruppe mit 1 bis 3 Kohlenstoffatomen, einem Halogenatom oder einer Nitrogruppe substituiert ist, bedeuten.

**7.** Verfahren nach Anspruch 1, wobei $R^1$ eine Gruppe der Formel $-NR^{9a}C(=Y)NR^{6f}NR^{6g}R^{6h}$ bedeutet, worin:
$R^{9a}$ ein Wasserstoffatom oder eine Methylgruppe bedeutet,
Y ein Sauerstoffatom oder ein Schwefelatom bedeutet und
$R^{6f}$, $R^{6g}$ und $R^{6h}$ gleich oder verschieden sind und jeweils ein Wasserstoffatom, eine Alkylgruppe mit 1 bis 4 Kohlenstoffatomen, eine Cycloalkylgruppe mit 3 bis 6 Kohlenstoffatomen, eine Phenylgruppe oder eine Phenylgruppe die mit einer Alkylgruppe mit 1 bis 3 Kohlenstoffatomen, einer Alkoxygruppe mit 1 bis 3 Kohlenstoffatomen, einem Halogenatom oder einer Nitrogruppe substituiert ist, darstellen.

**8.** Verfahren nach Anspruch 1, wobei $R^1$ eine Gruppe der Formel $-NR^{9a}C(=y)NR^{6j}NHZ$ bedeutet, worin:
$R^{9a}$ ein Wasserstoffatom oder eine Methylgruppe bedeutet,
Y ein sauerstoffatom oder ein Schwefelatom bedeutet,
$R^{6j}$ eine Alkylgruppe mit 1 bis 4 Kohlenstoffatomen oder eine Cycloalkylgruppe mit 3 bis 6 Kohlenstoffatomen bedeutet,
Z eine Gruppe der Formel $-COOR^{7a}$ (in der $R^{7a}$ eine Alkylgruppe mit 1 bis 4 Kohlenstoffatomen, eine Cycloalkylgruppe mit 3 bis 6 Kohlenstoffatomen oder eine Benzylgruppe darstellt), eine Gruppe der Formel $-COR^{6k}$ (in der $R^{6k}$ bedeutet: eine Alkylgruppe mit 1 bis 4 Kohlenstoffatomen, eine Cycloalkylgruppe mit 3 bis 6 Kohlenstoffatomen, eine Phenylgruppe oder eine Phenylgruppe, die mit einer Alkylgruppe mit 1 bis 3 Kohlenstoffatomen, einer Alkoxygruppe mit 1 bis 3 Kohlenstoffatomen, einem Halogenatom oder einer Nitrogruppe substituiert ist), oder eine Gruppe der Formel $-SO_2R^{6m}$ (in der $R^{6m}$ bedeutet: eine Alkylgruppe mit 1 bis 4 Kohlenstoffatomen, eine Phenylgruppe oder Phenylgruppe, die mit einer Alkylgruppe mit 1 bis 3 Kohlenstoffatomen, einer Alkoxygruppe mit 1 bis 3 Kohlenstoffatomen, einem Halogenatom oder einer Nitrogruppe substituiert ist), bedeutet.

**9.** Verfahren nach Anspruch 1, wobei $R^1$ eine Gruppe der Formel $-NR^{9a}C(=NR^{11a})NHR^{11b}$ bedeutet, worin:
$R^{9a}$ ein Wasserstoffatom oder eine Methylgruppe bedeutet,
$R^{11a}$ und $R^{11b}$ gleich oder verschieden sind und jeweils bedeuten: ein Wasserstoffatom, eine Alkylgruppe mit 1 bis 4 Kohlenstoffatomen, eine Phenylgruppe, eine Phenylgruppe, die mit einer Alkylgruppe mit 1 bis 3 Kohlenstoffatomen, einer Alkoxygruppe mit 1 bis 3 Kohlenstoffatomen, einem Halogenatom oder einer Nitrogruppe substituiert ist, eine Gruppe der Formel $-COOR^{7b}$ (in der $R^{7b}$ eine Alkylgruppe mit 1 bis 4 Kohlenstoffatomen, eine Cycloalkylgruppe mit 3 bis 6 Kohlenstoffatomen oder eine Benzylgruppe

bedeutet) oder eine Gruppe der Formel $-COR^{6n}$ (in der $R^{6n}$ eine Alkylgruppe mit 1 bis 4 Kohlenstoffatomen, eine Cycloalkylgruppe mit 3 bis 6 Kohlenstoffatomen, eine Phenylgruppe oder eine Phenylgruppe, die mit einer Alkylgruppe mit 1 bis 3 Kohlenstoffatomen, einer Alkoxygruppe mit 1 bis 3 Kohlenstoffatomen, einem Halogenatom oder einer Nitrogruppe substituiert ist, bedeutet).

**10.** Verfahren nach Anspruch 1, wobei $R^1$ eine Gruppe der Formel $-NR^{9a}C(=NR^{11c})R^{6p}$ bedeutet, worin:

$R^{9a}$ ein Wasserstoffatom oder eine Methylgruppe bedeutet,

$R^{11c}$ bedeutet: ein Wasserstoffatom, eine Alkylgruppe mit 1 bis 4 Kohlenstoffatomen, eine Phenylgruppe, eine Phenylgruppe, die mit einer Alkylgruppe mit 1 bis 3 Kohlenstoffatomen, einer Alkoxygruppe mit 1 bis 3 Kohlenstoffatomen, einem Halogenatom oder einer Nitrogruppe substituiert ist, eine Gruppe der Formel $-COOR^{7c}$ (in der $R^{7c}$ eine Alkylgruppe mit 1 bis 4 Kohlenstoffatomen, eine Cycloalkylgruppe mit 3 bis 6 Kohlenstoffatomen oder eine Benzylgruppe bedeutet) oder eine Gruppe der Formel $-COR^{6q}$ (in der $R^{6q}$ eine Alkylgruppe mit 1 bis 4 Kohlenstoffatomen, eine Cycloalkylgruppe mit 3 bis 6 Kohlenstoffatomen, eine Phenylgruppe oder eine Phenylgruppe bedeutet, die mit einer Alkylgruppe mit 1 bis 3 Kohlenstoffatomen, einer Alkoxygruppe mit 1 bis 3 Kohlenstoffatomen, einem Halogenatom oder einer Nitrogruppe substituiert ist),

$R^{6p}$ bedeutet: eine Alkylgruppe mit 1 bis 4 Kohlenstoffatomen, eine Cycloalkylgruppe mit 3 bis 6 Kohlenstoffatomen, eine Phenylgruppe oder eine Phenylgruppe, die mit einer Alkylgruppe mit 1 bis 3 Kohlenstoffatomen, einer Alkoxygruppe mit 1 bis 3 Kohlenstoffatomen, einem Halogenatom oder einer Nitrogruppe substituiert ist.

**11.** Verfahren nach Anspruch 1, wobei $R^1$ eine Gruppe der Formel $-NR^{9a}SO_mR^{6r}$ bedeutet, worin:

$R^{9a}$ ein Wasserstoffatom oder eine Methylgruppe bedeutet,

$R^{6r}$ eine Alkylgruppe mit 1 bis 4 Kohlenstoffatomen, eine mit einer Cyangruppe substituierte Alkylgruppe, eine Phenylgruppe oder eine Phenylgruppe, die mit einer Alkylgruppe mit 1 bis 3 Kohlenstoffatomen, einer Alkoxygruppe mit 1 bis 3 Kohlenstoffatomen, einem Halogenatom oder einer Nitrogruppe substituiert ist, bedeutet und

m für 1 oder 2 steht.

**12.** Verfahren nach einem der Ansprüche 1 bis 11, wobei A eine Gruppe der Formel

$-CHR^2-CHR^3-CHR^4-$

bedeutet, in der $R^2$, $R^3$ und $R^4$ gleich oder verschieden sind und jeweils ein Wasserstoffatom oder eine Methylgruppe bedeuten.

**13.** Verfahren nach einem der Ansprüche 1 bis 11, wobei A eine Gruppe der Formel

$-CR^2=CR^3-CHR^4-$

bedeutet, worin $R^2$, $R^3$ und $R^4$ gleich oder verschieden sind und jeweils ein Wasserstoffatom oder eine Methylgruppe bedeuten.

**14.** Verfahren nach einem der vorhergehenden Patentansprüche, wobei der Substituent $R^1$ in meta- oder ortho-Stellung ist.

**15.** Verfahren nach Anspruch 1, wobei A eine Gruppe der Formel

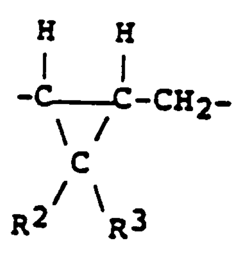

bedeutet, in der $R^2$ und $R^3$ gleich oder verschieden sind und jeweils ein Wasserstoffatom, ein Chloratom oder eine Methylgruppe bedeuten.

**17.** Verfahren nach einem der Ansprüche 1 bis 16, wobei $R^5$ eine Ethylgruppe bedeutet.

**18.** Verfahren nach einem der Ansprüche 1 bis 16, wobei das Ausgangsmaterial ein Gemisch aus einer Verbindung, in der $R^5$ eine Ethylgruppe bedeutet, und der entsprechenden Verbindung, in der $R^5$ eine

Methylgruppe bedeutet, darstellt, wobei ein Gemisch aus Verbindungen der Formel (I) hergestellt wird, in deren einer $R^5$ eine Ethylgruppe und in deren anderer $R^5$ eine Methylgruppe ist.

**19.** Verfahren nach einem der vorhergehenden Patentansprüche, wobei $R^3$ und $R^4$ jeweils Wasserstoffatome bedeuten.

**20.** Verfahren nach einem der vorhergehenden Patentansprüche, wobei X eine Hydroxygruppe bedeutet.

**21.** Verfahren nach Anspruch 1, wobei die hergestellte Verbindung
13-(3-Aminocinnamyloxy)milbemycin $A_4$
13-(2-Aminocinnamyloxy)milbemycin $A_4$
13-(3-Acetamidocinnamyloxy)milbemycin $A_4$
13-[3-(3-Methylthioureido)cinnamyloxy]milbemycin $A_4$
13-(3-Cyanoacetamidocinnamyloxy)milbemycin $A_4$
13-(3-Methansulfonylaminocinnamyloxy)milbemycin $A_4$
13-[3-N-(Methansulfonyl)methylaminocinnamyloxy]milbemycin $A_4$
13-(3-Methylaminocinnamyloxy)milbemycin $A_4$ bzw.
13-[3-(3,3-Dimethylcarbazoylamino)cinnamyloxy]milbemycin $A_4$
oder ein pharmazeutisch geeignetes Salz davon ist.

**22.** Verfahren nach Anspruch 1, wobei die hergestellte Verbindung
13-(3-Cyanoacetamidocinnamyloxy)milbemycin $A_4$
13-(3-Methansulfonylaminocinnamyloxy)milbemycin $A_4$
13-[3-N-(Methansulfonyl)methylaminocinnamyloxy]milbemycin $A_4$
oder ein pharmazeutisch geeignetes Salz davon ist.

**23.** Verwendung einer Verbindung der Formel (I) oder eines Salzes oder Esters davon gemäß der Definition in einem der Ansprüche 1 bis 22 zur Herstellung eines Arzneimittels zur Behandlung von Tieren, die an Parasitenbefall durch Würmer, Milben oder Insekten leiden.

**24.** Verwendung nach Anspruch 23, wobei diese Verbindung
13-(3-Aminocinnamyloxy)milbemycin $A_4$
13-(2-Aminocinnamyloxy)milbemycin $A_4$
13-(3-Acetamidocinnamyloxy)milbemycin $A_4$
13-[3-(3-Methylthioureido)cinnamyloxy]milbemycin $A_4$
13-(3-Cyanoacetamidocinnamyloxy)milbemycin $A_4$
13-(3-Methansulfonylaminocinnamyloxy)milbemycin $A_4$
13-[3-N-(Methansulfonyl)methylaminocinnamyloxy]milbemycin $A_4$
13-(3-Methylaminocinnamyloxy)milbemycin $A_4$ bzw.
13-[3-(3,3-Dimethylcarbazoylamino)cinnamyloxy]milbemycin $A_4$
oder ein pharmazeutisch geeignetes Salz davon ist.

## Revendications

**Revendications pour les Etats contractants suivants : GB, DE, FR, IT, CH, DK, BE, NL, SE, LU, AT**

**1.** Composés de formule (I) :

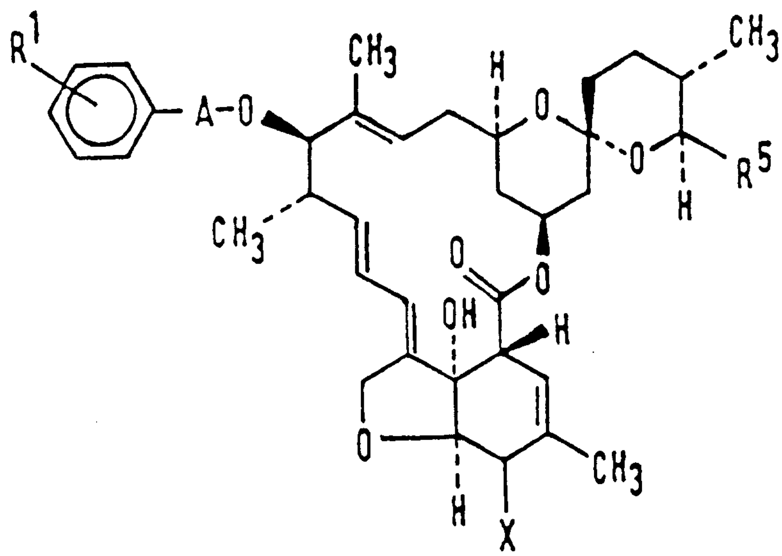

(I)

dans laquelle :

$R^1$ représente : un atome d'hydrogène ; un atome d'halogène ; un groupe cyano ; un groupe nitro ; un groupe alkyle qui a 1 à 4 atomes de carbone et qui n'est pas substitué ou est substitué par au moins l'un des substituants (a) ; un groupe alcoxy ayant 1 à 4 atomes de carbone ; un groupe alcoxyalcoxy ayant un total de 2 à 6 atomes de carbone ; ou un groupe répondant à l'une des formules suivantes :

$-(CH_2)_nNHR^9$
$-(CH_2)_nNR^9COR^6$
$-(CH_2)_nNR^9COCOR^6$
$-(CH_2)_nNR^9COCOOR^7$
$-(CH_2)_nNR^9CHR^6NHCOR^6$
$-(CH_2)_nNR^9CHR^6NHCONHR^6$
$-(CH_2)_nNR^9CHR^6NHCOOOR^7$
$-(CH_2)_nNR^9C(=Y)YR^6$
$-(CH_2)_nNR^9C(=Y)NR^6R^6$
$-(CH_2)_nNR^9C(=Y)NR^6NR^6R^6$
$-(CH_2)_nNR^9C(=Y)NR^6NHZ$
$-(CH_2)_nNR^9C(=NR^{11})NHR^{11}$
$-(CH_2)_nNR^9C(=NR^{11})R^6$
$-(CH_2)_nNR^9SO_mR^6$
$-CONHR^6$

ou

$-COOR^7$

dans lesquelles :

m est 1 ou 2 ;

n est 0, 1 ou 2 ;

$R^6$ représente un atome d'hydrogène ; un groupe alkyle ayant 1 à 8 atomes de carbone ; un groupe alkyle substitué qui a 1 à 8 atomes de carbone et qui est substitué par au moins l'un des substituants (b) ; un groupe hydrocarboné aliphatique ayant 2 à 8 atomes de carbone et ayant une ou deux doubles ou triples liaisons carbone-carbone ; un groupe cycloalkyle ayant 2 à 8 atomes de carbone ; un groupe

cycloalkyle substitué qui a 2 à 8 atomes de carbone et qui est substitué par au moins l'un des substituants (c) ; un groupe aryle qui a 6 à 14 atomes de carbone cycliques et qui n'est pas substitué ou qui est substitué par au moins l'un des substituants (c) ; un groupe aryloxy qui a 6 à 14 atomes de carbone cycliques et qui n'est pas substitué ou qui est substitué par au moins l'un des substituants (c) ; ou un groupe arylthio qui a 6 à 14 atomes de carbone cycliques et qui n'est pas substitué ou qui est substitué par au moins l'un des substituants (c) ;

$R^7$ représente un groupe alkyle ayant 1 à 4 atomes de carbone, un groupe cycloalkyle ayant 3 à 7 atomes de carbone, ou un groupe aralkyle dans lequel un groupe alkyle ayant 1 à 4 atomes de carbone est substitué par 1 à 3 groupes aryle qui ont 6 à 10 atomes de carbone cycliques et qui ne sont pas substitués ou sont substitués par au moins l'un des substituants (c) ;

$R^9$ représente un atome d'hydrogène ou un groupe alkyle ayant 1 à 4 atomes de carbone ;

$R^{11}$ représente l un quelconque des groupes ou atomes définis ci-dessus pour $R^6$, ou bien il représente un groupe cyano, un groupe nitro, un groupe de formule $-COOR^7$ (où $R^7$ est tel que défini ci-dessus), ou un groupe de formule $-COR^6$ (où $R^6$ est tel que défini ci-dessus) ;

Y représente un atome d'oxygène ou un atome de soufre ; et lorsqu'il y a deux ou plusieurs groupes représentés par Y, ceux-ci sont identiques ou différents entre eux ;

Z représente un groupe de formule $-COOR^7$ (où $R^7$ est tel que défini ci-dessus), un groupe de formule $-COR^6$ (où $R^6$ est tel que défini ci-dessus) ou un groupe de formule $-SO_2R^6$ (où $R^6$ est tel que défini ci-dessus) ;

A représente un groupe répondant à l'une des formules suivantes :

$-CHR^2-CHR^3-CHR^4-$

$-CR^2=CR^3-CHR^4-$

ou

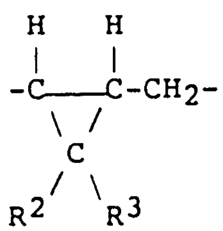

dans lesquelles $R^2$, $R^3$ et $R^4$ sont identiques ou différents et représentent chacun un atome d'hydrogène, un atome d'halogène, un groupe alkyle ayant 1 à 4 atomes de carbone ou un groupe alcoxy ayant 1 à 4 atomes de carbone ;

$R^5$ représente un groupe méthyle, un groupe éthyle, un groupe isopropyle ou un groupe *sec*-butyle ; et

X représente : un groupe hydroxyle ; un groupe alcanoyloxy qui a 1 à 5 atomes de carbone et qui n'est pas substitué ou est substitué par au moins l'un des substituants (d) ; ou un groupe hydroxyimino ;

substituants (a) :

atomes d'halogènes, groupes alcoxy ayant 1 à 4 atomes de carbone, groupes alkylthio ayant 1 à 4 atomes de carbone et groupes alcanoyloxy ayant 1 à 5 atomes de carbone ;

substituants (b) :

groupes cycloalkyle ayant 3 à 8 atomes de carbone ; groupes alcoxy ayant 1 à 4 atomes de carbone ; groupes alkylthio ayant 1 à 4 atomes de carbone ; groupes cyanoalkylthio ayant 2 à 5 atomes de carbone ; groupes alcoxycarbonyle ayant 2 à 5 atomes de carbone ; atomes d'halogènes ; groupes cyano ; groupes nitro ; groupes amino ; groupes aryle qui ont 6 à 14 atomes de carbone cycliques et qui ne sont pas substitués ou sont substitués par au moins l'un des substituants (c) ; un groupe pyridyle, thiényle, furyle, pyrrolyle, imidazolyle, triazolyle, tétrazolyle, thiazolyle, oxazolyle, indolyle, ou un tel groupe substitué par au moins l'un des substituants (c) ; groupes aryloxy qui ont 6 à 14 atomes de carbone cycliques et qui ne sont pas substitués ou sont substitués par au moins l'un des substituants (c) ; et groupes arylthio qui ont 6 à 14 atomes de carbone cycliques et qui ne sont pas substitués ou sont substitués par au moins l'un des substituants (c) ;

substituants (c) :

groupes alkyle ayant 1 à 4 atomes de carbone, groupes alcoxy ayant 1 à 4 atomes de carbone,

groupes alkylthio ayant 1 à 4 atomes de carbone, groupes alcanoyloxy ayant 1 à 5 atomes de carbone, groupes alcoxycarbonyle ayant 2 à 5 atomes de carbone, atomes d'halogènes, groupes cyano, groupes nitro, groupes amino, groupes alkylamino dans lesquels la portion alkyle compte 1 à 4 atomes de carbone, groupes dialkylamino dans lesquels les portions alkyle sont identiques ou différentes et chacune représente un groupe alkyle ayant 1 à 4 atomes de carbone, un groupe carbamoyle, un groupe alkylcarbamoyle dans lequel la portion alkyle compte 1 à 4 atomes de carbone, un groupe dialkylcarbamoyle dans lequel chaque portion alkyle compte 1 à 4 atomes de carbone, et groupes alcanoylamino ayant 1 à 5 atomes de carbone ;

substituants (d) :

atomes d'halogènes, groupes alcoxy ayant 1 à 4 atomes de carbone, groupes alcoxycarbonyle ayant 2 à 5 atomes de carbone et groupes carboxyle ;

et leurs sels et esters.

**2.** Composés selon la revendication 1, dans lesquels

$R^1$ représente : un atome d'hydrogène, un groupe alkyle ayant 1 à 3 atomes de carbone ; un groupe alcoxy ayant 1 à 3 atomes de carbone ; les atomes de fluor ou de chlore ; ou les groupes nitro ou amino.

**3.** Composés selon la revendication 1, dans lesquels

$R^1$ représente un groupe de formule $-NR^{9a}COR^{6a}$ où :

$R^{9a}$ représente un atome d'hydrogène ou un groupe méthyle ; et

$R^{6a}$ représente : un atome d'hydrogène ; un groupe alkyle ayant 1 à 4 atomes de carbone ; un groupe cycloalkyle ayant 3 à 5 atomes de carbone ; un groupe alkyle substitué ayant 1 à 3 atomes de carbone et substitué par un atome d'halogène, un groupe cyano, un groupe alcoxy ayant 1 à 3 atomes de carbone, un groupe alkylthio ayant 1 à 3 atomes de carbone, un groupe cyanométhylthio ou un groupe phénoxy ; un groupe alcényle ayant 2 à 4 atomes de carbone ; un groupe phényle ; ou un groupe phényle substitué par un groupe alkyle ayant 1 à 3 atomes de carbone, un groupe alcoxy ayant 1 à 3 atomes de carbone, un atome d'halogène ou un groupe nitro.

**4.** Composés selon la revendication 1, dans lesquels

$R^1$ représente un groupe de formule $-NR^{9a}COCOR^{6b}$ ou :

$R^{9a}$ représente un atome d'hydrogène ou un groupe méthyle ; et

$R^{6b}$ représente ; un atome d'hydrogène ; un groupe alkyle ayant 1 à 4 atomes de carbone ; un groupe cycloalkyle ayant 3 à 5 atomes de carbone ; un groupe alcényle ayant 2 à 4 atomes de carbone ; un groupe phényle ; ou un groupe phényle substitué par un groupe alkyle ayant 1 à 3 atomes de carbone, un groupe alcoxy ayant 1 à 3 atomes de carbone, un atome d'halogène ou un groupe nitro.

**5.** Composés selon la revendication 1, dans lesquels

$R^1$ représente un groupe de formule $-NR^{9a}C(=Y)YR^{6c}$ où :

$R^{9a}$ représente un atome d'hydrogène ou un groupe méthyle ;

Y représente un atome d'oxygène ; et

$R^{6c}$ représente : un groupe alkyle ayant 1 à 4 atomes de carbone ; un groupe alkyle ayant 1 à 4 atomes de carbone et substitué par un atome d'halogène ou un groupe alcoxy ayant 1 à 3 atomes de carbone ; un groupe vinyle ; un groupe allyle ; un groupe benzyle ; un groupe méthoxybenzyle ; un groupe nitrobenzyle ; ou un groupe phényle.

**6.** Composés selon la revendication 1, dans lesquels

$R^1$ représente un groupe de formule $-NR^{9a}(=Y)NR^{6d}R^{6e}$ où :

$R^{9a}$ représente un atome d'hydrogène ou un groupe méthyle ;

Y représente un atome d'oxygène ou un atome de soufre ; et

$R^{6d}$ et $R^{6e}$ sont identiques ou différents et représentent chacun : un atome d'hydrogène ; un groupe alkyle ayant 1 à 4 atomes de carbone ; un groupe cycloalkyle ayant 3 à 6 atomes de carbone ; un groupe phényle ; ou un groupe phényle substitué par un groupe alkyle ayant 1 à 3 atomes de carbone, un groupe alcoxy ayant 1 à 3 atomes de carbone, un atome d'halogène ou un groupe nitro.

**7.** Composés selon la revendication 1, dans lesquels

$R^1$ représente un groupe de formule $-NR^{9a}C(=Y)NR^{6f}NR^{6g}R^{6h}$ où :

$R^{9a}$ représente un atome d'hydrogène ou un groupe méthyle ;

Y représente un atome d'oxygène ou un atome de soufre ; et

$R^{6f}$, $R^{6g}$ et $R^{6h}$ sont identiques ou différents et représentent chacun : un atome d'hydrogène ; un groupe alkyle ayant 1 à 4 atomes de carbone ; un groupe cycloalkyle ayant 3 à 6 atomes de carbone ; un groupe phényle ; ou un groupe phényle substitué par un groupe alkyle ayant 1 à 3 atomes de carbone, un groupe alcoxy ayant 1 à 3 atomes de carbone, un atome d'halogène ou un groupe nitro.

**8.** Composés selon la revendication 1, dans lesquels
$R^1$ représente un groupe de formule $-NR^{9a}C(=Y)NR^{6j}NHZ$ où :
$R^{9a}$ représente un atome d'hydrogène ou un groupe méthyle ;
Y représente un atome d'oxygène ou un atome de soufre ;
$R^{6j}$ représente un groupe alkyle ayant 1 à 4 atomes de carbone ; ou un groupe cycloalkyle ayant 3 à 6 atomes de carbone ;
Z représente un groupe de formule $-COOR^{7a}$ (où $R^{7a}$ représente : un groupe alkyle ayant 1 à 4 atomes de carbone, un groupe cycloalkyle ayant 3 à 6 atomes de carbone ou un groupe benzyle) ; un groupe de formule $-COR^{6k}$ (où $R^{6k}$ représente : un groupe alkyle ayant 1 à 4 atomes de carbone ; un groupe cycloalkyle ayant 3 à 6 atomes de carbone ; un groupe phényle ; ou un groupe phényle substitué par un groupe alkyle ayant 1 à 3 atomes de carbone, un groupe alcoxy ayant 1 à 3 atomes de carbone, un atome d'halogène ou un groupe nitro), ou un groupe de formule $-SO_2R^{6m}$ (où $R^{6m}$ représente : un groupe alkyle ayant 1 à 4 atomes de carbone ; un groupe phényle ; ou un groupe phényle substitué par un groupe alkyle ayant 1 à 3 atomes de carbone, un groupe alcoxy ayant 1 à 3 atomes de carbone, un atome d'halogène ou un groupe nitro).
**9.** Composés selon la revendication 1, dans lesquels
$R^1$ représente un groupe de formule $NR^9{}_aC(=NR^{11a})NHR^{11b}$ ou :
$R^{9a}$ représente un atome d'hydrogène ou un groupe méthyle ;
$R^{11a}$ et $R^{11b}$ sont identiques ou différents et représentent chacun : un atome d'hydrogène ; un groupe alkyle ayant 1 à 4 atomes de carbone ; un groupe phényle ; un groupe phényle substitué par un groupe alkyle ayant 1 à 3 atomes de carbone, un groupe alcoxy ayant 1 à 3 atomes de carbone, un atome d'halogène ou un groupe nitro ; un groupe de formule $-COOR^{7b}$ (où $R^{7b}$ représente : un groupe alkyle ayant 1 à 4 atomes de carbone ; un groupe cycloalkyle ayant 3 à 6 atomes de carbone ; ou un groupe benzyle) ; ou un groupe de formule $-COR^{6n}$ (où $R^{6n}$ représente : un groupe alkyle ayant 1 à 4 atomes de carbone ; un groupe cycloalkyle ayant 3 à 6 atomes de carbone ; un groupe phényle ; ou un groupe phényle substitué par un groupe alkyle ayant 1 à 3 atomes de carbone, un groupe alcoxy ayant 1 à 3 atomes de carbone, un atome d'halogène ou un groupe nitro).
**10.** Composés selon la revendication 1, dans lesquels
$R^1$ représente un groupe de formule $-NR^{9a}C(=NR^{11c})R^{6P}$ où :
$R^{9a}$ représente un atome d'hydrogène ou un groupe méthyle ;
$R^{11c}$ représente : un atome d'hydrogène ; un groupe alkyle ayant 1 à 4 atomes de carbone ; un groupe phényle ; un groupe phényle substitué par un groupe alkyle ayant 1 à 3 atomes de carbone, un groupe alcoxy ayant 1 à 3 atomes de carbone, un atome d'halogène ou un groupe nitro ; un groupe de formule $-COOR^{7c}$ (où $R^{7c}$ représente : un groupe alkyle ayant 1 à 4 atomes de carbone ; un groupe cycloalkyle ayant 3 à 6 atomes de carbone ; ou un groupe benzyle) ; ou un groupe de formule $-COR^{6q}$ (où $R^{6q}$ représente : un groupe alkyle ayant 1 à 4 atomes de carbone ; un groupe cycloalkyle ayant 3 à 6 atomes de carbone ; un groupe phényle ; ou un groupe phényle substitué par un groupe alkyle ayant 1 à 3 atomes de carbone, un groupe alcoxy ayant 1 à 3 atomes de carbone, un atome d'halogène ou un groupe nitro) ;
$R^{6p}$ représente : un groupe alkyle ayant 1 à 4 atomes de carbone ; un groupe cycloalkyle ayant 3 à 6 atomes de carbone ; un groupe phényle ; ou un groupe phényle substitué par un groupe alkyle ayant 1 à 3 atomes de carbone, un groupe alcoxy ayant 1 à 3 atomes de carbone, un atome d'halogène ou un groupe nitro.
**11.** Composés selon la revendication 1, dans lesquels
$R^1$ représente un groupe de formule $-NR^{9a}SO_mR^{6r}$ où :
$R^{9a}$ représente un atome d'hydrogène ou un groupe méthyle ;
$R^{6r}$ représente : un groupe alkyle ayant 1 à 4 atomes de carbone ; un groupe alkyle substitué par un groupe cyano ; un groupe phényle ; ou un groupe phényle substitué par un groupe alkyle ayant 1 à 3 atomes de carbone, un groupe alcoxy ayant 1 à 3 atomes de carbone, un atome d'halogène ou un groupe nitro ; et
m est 1 ou 2.
**12.** Composés selon l'une quelconque des revendications 1 à 11, dans lesquels A représente un groupe de formule :

$$-CHR^2-CHR^3-CHR^4-$$

où $R^2$, $R^3$ et $R^4$ sont identiques ou différents et représentent chacun un atome d'hydrogène ou un groupe méthyle.

**13.** Composés selon l'une quelconque des revendications 1 à 11, dans lesquels A représente un groupe de formule :

$-CR^2=CR^3-CHR^4-$

où $R^2$, $R^3$ et $R^4$ sont identiques ou différents et représentent chacun un atome d'hydrogène ou un groupe méthyle.

**14.** Composés selon l'une quelconque des revendications précédentes, dans lesquels le substituant $R^1$ est en position *méta* ou *ortho*.

**15.** Composés selon la revendication 1, dans lesquels A représente un groupe de formule :

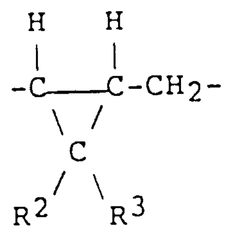

où $R^2$ et $R^3$ sont identiques ou différents et représentent chacun un atome d'hydrogène, un atome de chlore ou un groupe méthyle.

**17.** Composés selon l'une quelconque des revendications 1 à 16, dans lesquels $R^5$ représente un groupe éthyle.

**18.** Composé selon l'une quelconque des revendications 1 à 16, qui est un mélange d'un composé dans lequel $R^5$ représente un groupe éthyle et du composé correspondant dans lequel $R^5$ représente un groupe méthyle.

**19.** Composés selon l'une quelconque des revendications précédentes, dans lesquels chacun de $R^3$ et $R^4$ représente un atome d'hydrogène.

**20.** Composés selon l'une quelconque des revendications précédentes, dans lesquels X représente un groupe hydroxyle.

**21.** 13-(3-cyanoacétamidocinnamyloxy)milbémycine $A_4$ et ses sels pharmaceutiquement acceptables.

**22.** 13-(3-méthanesulfonylaminocinnamyloxymilbémycine $A_4$ et ses sels pharmaceutiquement acceptables.

**23.** 13-[3-*N*-(méthanesulfonyl)méthylaminocinnamyloxy]milbémycine $A_4$ et ses sels pharmaceutiquement acceptables.

**24.** Composition anthelminthique, acaricide et insecticide comprenant au moins un composé selon l'une quelconque des revendications précédentes en mélange avec un support ou diluant acceptable pour l'usage pharmaceutique, agricole, vétérinaire ou horticole.

**25.** Composition selon la revendication 24, dans laquelle ledit composé est :
la 13-(3-aminocinnamyloxy)milbémycine $A_4$
la 13-(2-aminocinnamyloxy)milbémycine $A_4$
la 13-(3-acétamidocinnamyloxy)milbémycine $A_4$
la 13-[3-(3-méthylthiouréido)cinnamyloxy]milbémycine $A_4$
la 13-(3-cyanoacétamidocinnamyloxy)milbémycine $A_4$
la 13-(3-méthanesulfonylaminocinnamyloxy)milbémycine $A_4$
la 13-[3-*N*-(méthanesulfonyl)méthylaminocinnamyloxy]milbémycine $A_4$
la 13-(3-méthylaminocinnamyloxy)milbémycine $A_4$ et
la 13-[3-(3,3-diméthylcarbazoylamino)cinnamyloxy]milbémycine $A_4$
ou un de leurs sels pharmaceutiquement acceptables.

**26.** Composé selon l'une quelconque des revendications 1 à 23, pour son utilisation en thérapie.

**27.** Utilisation d'un composé selon l'une quelconque des revendications 1 à 23, pour la fabrication d'un médicament destiné au traitement d'animaux parasités par des helminthes, des acariens ou des insectes.

**28.** Procédé selon la revendications 26 ou 27, dans lequel ledit compose est :
la 13-(3-aminocinnamyloxy)milbémycine $A_4$
la 13-(2-aminocinnamyloxy)milbémycine $A_4$
la 13-(3-acétamidocinnamyloxy)milbémycine $A_4$

la 13-[3-(3-méthylthiouréido)cinnamyloxy]milbémycine $A_4$
la 13-(3-cyanoacétamidocinnamyloxy)milbémycine $A_4$
la 13-(3-méthanesulfonylaminocinnamyloxy)milbémycine $A_4$
la 13-[3-*N*-(méthanesulfonyl)méthylaminocinnamyloxy]milbémycine $A_4$
la 13-(3-méthylaminocinnamyloxy)milbémycine $A_4$ et
la 13-[3-(3,3-diméthylcarbazoylamino)cinnamyloxy]milbémycine $A_4$
ou un de leurs sels pharmaceutiquement acceptables.

**29.** Utilisation d'un composé selon l'une quelconque des revendications 1 à 23 en thérapie.

**30.** Utilisation d'un composé selon l'une quelconque des revendications 1 à 23 pour la fabrication d'un médicament destiné au traitement d'animaux parasités par des helminthes, des acariens ou des insectes.

**31.** Procédé pour préparer un composé selon l'une quelconque des revendications 1 à 23, qui consiste à soumettre un composé de formule (II) dans un ordre quelconque aux étapes (a) et (b) :

(II)

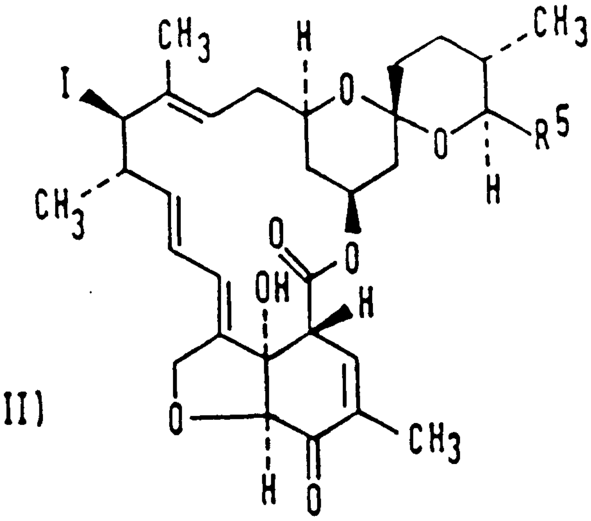

où $R^5$ est tel que défini ci-dessus,
(a) réaction avec un alcool de formule (IIa) :

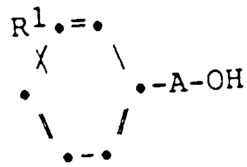

(IIa)

où $R^1$ est tel que défini dans la revendication 1 ; et
(b) réaction soit
($b^i$) avec un agent réducteur pour réduire l'atome d'oxygène à la position 5 en un groupe hydroxyle, suivie, facultativement, d'une réaction avec un agent acylant, pour former un composé de formule (I) dans lequel X représente un groupe alcanoyloxy qui compte 1 à 5 atomes de carbone et qui n'est pas substitué ou qui est substitué par au moins l'un des substituants (d) ; soit
($b^{ii}$) avec l'hydroxylamine ou un de ses sels, pour former un composé de formule (I) dans lequel X représente un groupe hydroxyimino ;
puis, facultativement, à soumettre le produit à l'une des étapes (c) et (d) ou aux deux ;
(c) conversion d'un groupe représenté par $R^1$ en un quelconque autre groupe ainsi représenté ; et
(d) salification ou estérification du produit.

**Revendications pour les Etats contractants suivants : ES, GR**

**1.** Procédé pour préparer des composés de formule (I) :

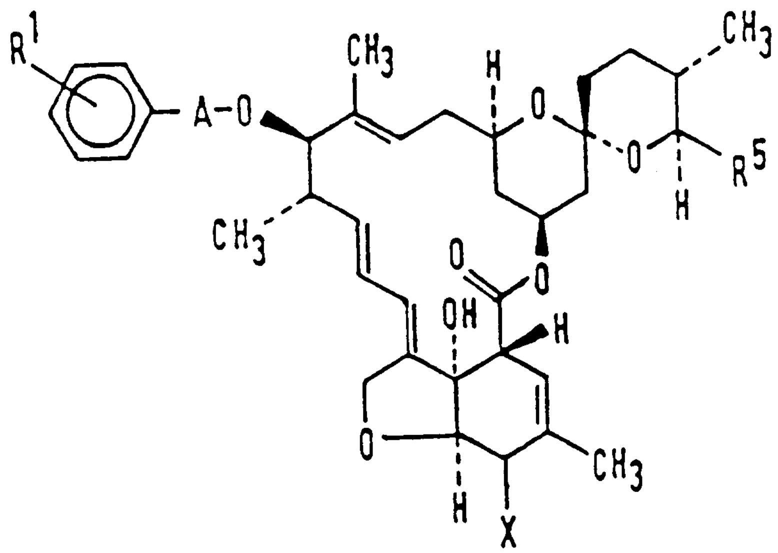

(I)

dans laquelle :

$R^1$ représente : un atome d'hydrogène ; un atome d'halogène ; un groupe cyano ; un groupe nitro ; un groupe alkyle qui a 1 à 4 atomes de carbone et qui n'est pas substitué ou est substitué par au moins l'un des substituants (a) ; un groupe alcoxy ayant 1 à 4 atomes de carbone ; un groupe alcoxyalcoxy ayant un total de 2 à 6 atomes de carbone ; ou un groupe répondant à l'une des formules suivantes :

$-(CH_2)_nNHR^9$
$-(CH_2)_nNR^9COR^6$
$-(CH_2)_nNR^9COCOR^6$
$-(CH_2)_nNR^9COCOOR^7$
$-(CH_2)_nNR^9CHR^6NHCOR^6$
$-(CH_2)_nNR^9CHR^6NHCONHR^6$
$-(CH_2)_nNR^9CHR^6NHCOOR^7$
$-(CH_2)_nNR^9C(=Y)YR^6$
$-(CH_2)_nNR^9C(=Y)NR^6R^6$
$-(CH_2)_nNR^9C(=Y)NR^6NR^6R^6$
$-(CH_2)_nNR^9C(=Y)NR^6NHZ$
$-(CH_2)_nNR^9C(=NR^{11})NHR^{11}$
$-(CH_2)_nNR^9C(=NR^{11})R^6$
$-(CH_2)_nNR^9SO_mR^6$
$-CONHR^6$

ou

$-COOR^7$

dans lesquelles :

m est 1 ou 2 ;

n est 0, 1 ou 2 ;

$R^6$ représente un atome d'hydrogène ; un groupe alkyle ayant 1 à 8 atomes de carbone ; un groupe alkyle substitué qui a 1 à 8 atomes de carbone et qui est substitué par au moins l'un des substituants (b) ; un groupe hydrocarboné aliphatique ayant 2 à 8 atomes de carbone et ayant une ou deux doubles ou triples liaisons carbone-carbone ; un groupe cycloalkyle ayant 2 à 8 atomes de carbone ; un groupe cycloalkyle substitué qui a 2 à 8 atomes de carbone et qui est substitué par au moins l'un des substituants (c) ; un groupe aryle qui a 6 à 14 atomes de carbone cycliques et qui n'est pas substitué ou

qui est substitué par au moins l'un des substituants (c) ; un groupe aryloxy qui a 6 à 14 atomes de carbone cycliques et qui n'est pas substitué ou qui est substitué par au moins l'un des substituants (c) ; ou un groupe arylthio qui a 6 à 14 atomes de carbone cycliques et qui n'est pas substitué ou qui est substitué par au moins l'un des substituants (c) ;

$R^7$ représente un groupe alkyle ayant 1 à 4 atomes de carbone, un groupe cycloalkyle ayant 3 à 7 atomes de carbone, ou un groupe aralkyle dans lequel un groupe alkyle ayant 1 à 4 atomes de carbone est substitué par 1 à 3 groupes aryle qui ont 6 à 10 atomes de carbone cycliques et qui ne sont pas substitués ou sont substitués par au moins l'un des substituants (c) ;

$R^9$ représente un atome d'hydrogène ou un groupe alkyle ayant 1 à 4 atomes de carbone ;

$R^{11}$ représente l'un quelconque des groupes ou atomes définis ci-dessus pour $R^6$, ou bien il représente un groupe cyano, un groupe nitro, un groupe de formule $-COOR^7$ (où $R^7$ est tel que défini ci-dessus), ou un groupe de formule $-COR^6$ (où $R^6$ est tel que défini ci-dessus) ;

Y représente un atome d'oxygène ou un atome de soufre ; et lorsqu'il y a deux ou plusieurs groupes représentés par Y, ceux-ci sont identiques ou différents entre eux ;

Z représente un groupe de formule $-COOR^7$ (où $R^7$ est tel que défini ci-dessus), un groupe de formule $-COR^6$ (où $R^6$ est tel que défini ci-dessus) ou un groupe de formule $-SO_2R^6$ (où $R^6$ est tel que défini ci-dessus) ;

A représente un groupe répondant à l'une des formules suivantes :

$-CHR^2-CHR^3-CHR^4-$

$-CR^2=CR^3-CHR^4-$

ou

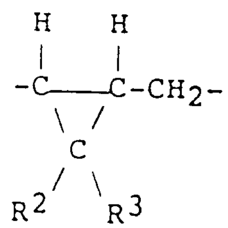

dans lesquelles $R^2$, $R^3$ et $R^4$ sont identiques ou différents et représentent chacun un atome d'hydrogène, un atome d'halogène, un groupe alkyle ayant 1 à 4 atomes de carbone ou un groupe alcoxy ayant 1 à 4 atomes de carbone ;

$R^5$ représente un groupe méthyle, un groupe éthyle, un groupe isopropyle ou un groupe *sec*-butyle ; et

X représente : un groupe hydroxyle ; un groupe alcanoyloxy qui a 1 à 5 atomes de carbone et qui n'est pas substitué ou est substitué par au moins l'un des substituants (d) ; ou un groupe hydroxyimino ;

substituants (a) :

atomes d'halogènes, groupes alcoxy ayant 1 à 4 atomes de carbone, groupes alkylthio ayant 1 à 4 atomes de carbone et groupes alcanoyloxy ayant 1 à 5 atomes de carbone ;

substituants (b) :

groupes cycloalkyle ayant 3 à 8 atomes de carbone ; groupes alcoxy ayant 1 à 4 atomes de carbone ; groupes alkylthio ayant 1 à 4 atomes de carbone ; groupes cyanoalkylthio ayant 2 à 5 atomes de carbone ; groupes alcoxycarbonyle ayant 2 à 5 atomes de carbone ; atomes d'halogènes ; groupes cyano ; groupes nitro ; groupes amino ; groupes aryle qui ont 6 à 14 atomes de carbone cycliques et qui ne sont pas substitués ou sont substitués par au moins l'un des substituants (c) ; un groupe pyridyle, thiényle, furyle, pyrrolyle, imidazolyle, triazolyle, tétrazolyle, thiazolyle, oxazolyle, indolyle, ou un tel groupe substitué par au moins l'un des substituants (c) ; groupes aryloxy qui ont 6 à 14 atomes de carbone cycliques et qui ne sont pas substitués ou sont substitués par au moins l'un des substituants (c) ; et groupes arylthio qui ont 6 à 14 atomes de carbone cycliques et qui ne sont pas substitues ou sont substitués par au moins l'un des substituants (c) ;

substituants (c) :

groupes alkyle ayant 1 à 4 atomes de carbone, groupes alcoxy ayant 1 à 4 atomes de carbone, groupes alkylthio ayant 1 à 4 atomes de carbone, groupes alcanoyloxy ayant 1 à 5 atomes de carbone, groupes alcoxycarbonyle ayant 2 à 5 atomes de carbone, atomes d'halogènes, groupes cyano, groupes nitro, groupes amino, groupes alkylamino dans lesquels la portion alkyle compte 1 à 4

atomes de carbone, groupes dialkylamino dans lesquels les portions alkyle sont identiques ou différentes et chacune représente un groupe alkyle ayant 1 à 4 atomes de carbone, un groupe carbamoyle, un groupe alkylcarbamoyle dans lequel la portion alkyle compte 1 à 4 atomes de carbone, un groupe dialkylcarbamoyle dans lequel chaque portion alkyle compte 1 à 4 atomes de carbone, et groupes alcanoylamino ayant 1 à 5 atomes de carbone ;

substituants (d) :

atomes d'halogènes, groupes alcoxy ayant 1 à 4 atomes de carbone, groupes alcoxycarbonyle ayant 2 à 5 atomes de carbone et groupes carboxyle ;

et leurs sels et esters, qui consiste à soumettre un composé dee formule (II) dans un ordre quelconque aux étapes (a) et (b) :

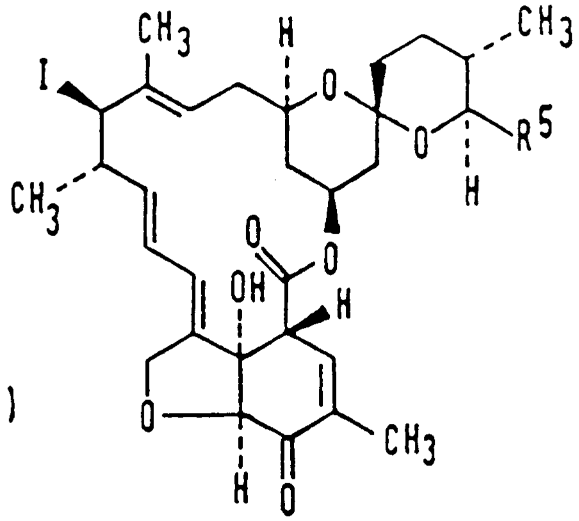

(II)

où $R^5$ est tel que défini ci-dessus,

(a) réaction avec un alcool de formule (IIa) :

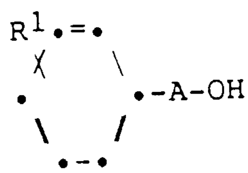

(IIa)

où $R^1$ est tel que défini ci-dessus ; et

(b) réaction soit

($b^i$) avec un agent réducteur pour réduire l'atome d'oxygène à la position 5 en un groupe hydroxyle, suivie, facultativement, d'une réaction avec un agent acylant, pour former un composé de formule (I) dans lequel X représente un groupe alcanoyloxy qui compte 1 à 5 atomes de carbone et qui n'est pas substitué ou qui est substitué par au moins l'un des substituants (d) ; soit

($b^{ii}$) avec l'hydroxylamine ou un de ses sels, pour former un composé de formule (I) dans lequel X représente un groupe hydroxyimino ;

puis, facultativement, à soumettre le produit à l'une des étapes (c) et (d) ou aux deux ;

(c) conversion d'un groupe représenté par $R^1$ en un quelconque autre groupe ainsi représenté ; et

(d) salification ou estérification du produit.

**2.** Procédé selon la revendication 1, dans lequel $R^1$ représente : un atome d'hydrogène, un groupe alkyle ayant 1 à 3 atomes de carbone ; un groupe alcoxy ayant 1 à 3 atomes de carbone ; les atomes de fluor ou de chlore ; ou les groupes nitro ou amino.

**3.** Procédé selon la revendication 1, dans lequel

$R^1$ représente un groupe de formule $-NR^{9a}COR^{6a}$ où :

$R^{9a}$ représente un atome d'hydrogène ou un groupe méthyle ; et

$R^{6a}$ représente : un atome d'hydrogène ; un groupe alkyle ayant 1 à 4 atomes de carbone ; un groupe cycloalkyle ayant 3 à 5 atomes de carbone ; un groupe alkyle substitué ayant 1 à 3 atomes de carbone

et substitué par un atome d'halogène, un groupe cyano, un groupe alcoxy ayant 1 à 3 atomes de carbone, un groupe alkylthio ayant 1 à 3 atomes de carbone, un groupe cyanométhylthio ou un groupe phénoxy ; un groupe alcényle ayant 2 à 4 atomes de carbone ; un groupe phényle ; ou un groupe phényle substitué par un groupe alkyle ayant 1 à 3 atomes de carbone, un groupe alcoxy ayant 1 à 3 atomes de carbone, un atome d'halogène ou un groupe nitro.

**4.** Procédé selon la revendication 1, dans lequel
$R^1$ représente un groupe de formule $-NR^{9a}COCOR^{6b}$ où :
$R^{9a}$ représente un atome d'hydrogène ou un groupe méthyle ; et
$R^{6b}$ représente ; un atome d'hydrogène ; un groupe alkyle ayant 1 à 4 atomes de carbone ; un groupe cycloalkyle ayant 3 à 5 atomes de carbone ; un groupe alcényle ayant 2 à 4 atomes de carbone ; un groupe phényle ; ou un groupe phényle substitué par un groupe alkyle ayant 1 à 3 atomes de carbone, un groupe alcoxy ayant 1 à 3 atomes de carbone, un atome d'halogène ou un groupe nitro.

**5.** Procédé selon la revendication 1, dans lequel
$R^1$ représente un groupe de formule $-NR^{9a}C(=Y)YR^{6c}$ où :
$R^{9a}$ représente un atome d'hydrogène ou un groupe méthyle ;
Y représente un atome d'oxygène ; et
$R^{6c}$ représente : un groupe alkyle ayant 1 à 4 atomes de carbone ; un groupe alkyle ayant 1 à 4 atomes de carbone et substitué par un atome d'halogène ou un groupe alcoxy ayant 1 à 3 atomes de carbone ; un groupe vinyle ; un groupe allyle ; un groupe benzyle ; un groupe méthoxybenzyle ; un groupe nitrobenzyle ; ou un groupe phényle.

**6.** Procédé selon la revendication 1, dans lequel
$R^1$ représente un groupe de formule $-NR^{9a}C(=Y)NR^{6d}R^{6e}$ où :
$R^{9a}$ représente un atome d'hydrogène ou un groupe méthyle ;
Y représente un atome d'oxygène ou un atome de soufre ; et
$R^{6d}$ et $R^{6e}$ sont identiques ou différents et représentent chacun : un atome d'hydrogène ; un groupe alkyle ayant 1 à 4 atomes de carbone ; un groupe cycloalkyle ayant 3 à 6 atomes de carbone ; un groupe phényle ; ou un groupe phényle substitué par un groupe alkyle ayant 1 à 3 atomes de carbone, un groupe alcoxy ayant 1 à 3 atomes de carbone, un atome d'halogène ou un groupe nitro.

**7.** Procédé selon la revendication 1, dans lequel
$R^1$ représente un groupe de formule $-NR^{9a}C(=Y)NR^{6f}NR^{6g}R^{6h}$ où :
$R^{9a}$ représente un atome d'hydrogène ou un groupe méthyle ;
Y représente un atome d'oxygène ou un atome de soufre ; et
$R^{6f}$, $R^{6g}$ et $R^{6h}$ sont identiques ou différents et représentent chacun : un atome d'hydrogène ; un groupe alkyle ayant 1 à 4 atomes de carbone ; un groupe cycloalkyle ayant 3 à 6 atomes de carbone ; un groupe phényle ; ou un groupe phényle substitué par un groupe alkyle ayant 1 à 3 atomes de carbone, un groupe alcoxy ayant 1 à 3 atomes de carbone, un atome d'halogène ou un groupe nitro.

**8.** Procédé selon la revendication 1, dans lequel
$R^1$ représente un groupe de formule $-NR^{9a}C(=Y)NR^{6j}NHZ$ où :
$R^{9a}$ représente un atome d'hydrogène ou un groupe méthyle ;
Y représente un atome d'oxygène ou un atome de soufre ;
$R^{6j}$ représente un groupe alkyle ayant 1 à 4 atomes de carbone ; ou un groupe cycloalkyle ayant 3 à 6 atomes de carbone ;
Z représente un groupe de formule $-COOR^{7a}$ (où $R^{7a}$ représente : un groupe alkyle ayant 1 à 4 atomes de carbone, un groupe cycloalkyle ayant 3 à 6 atomes de carbone ou un groupe benzyle) ; un groupe de formule $-COR^{6k}$ (où $R^{6k}$ représente : un groupe alkyle ayant 1 à 4 atomes de carbone ; un groupe cycloalkyle ayant 3 à 6 atomes de carbone ; un groupe phényle ; ou un groupe phényle substitué par un groupe alkyle ayant 1 à 3 atomes de carbone, un groupe alcoxy ayant 1 à 3 atomes de carbone, un atome d'halogène ou un groupe nitro), ou un groupe de formule $-SO_2R^{6m}$ (où $R^{6m}$ représente : un groupe alkyle ayant 1 à 4 atomes de carbone ; un groupe phényle ; ou un groupe phényle substitué par un groupe alkyle ayant 1 à 3 atomes de carbone, un groupe alcoxy ayant 1 à 3 atomes de carbone, un atome d'halogène ou un groupe nitro).

**9.** Procédé selon la revendication 1, dans lequel
$R^1$ représente un groupe de formule $-NR^9{}_aC(=NR^{11a})NHR^{11b}$ où :
$R^{9a}$ représente un atome d'hydrogène ou un groupe méthyle ;
$R^{11a}$ et $R^{11b}$ sont identiques ou différents et représentent chacun : un atome d'hydrogène ; un groupe alkyle ayant 1 à 4 atomes de carbone ; un groupe phényle ; un groupe phényle substitué par un groupe alkyle ayant 1 à 3 atomes de carbone, un groupe alcoxy ayant 1 à 3 atomes de carbone, un atome d'halogène ou un groupe nitro ; un groupe de formule $-COOR^{7b}$ (où $R^{7b}$ représente : un groupe alkyle

ayant 1 à 4 atomes de carbone ; un groupe cycloalkyle ayant 3 à 6 atomes de carbone ; ou un groupe benzyle) ; ou un groupe de formule $-COR^{6n}$ (où $R^{6n}$ représente : un groupe alkyle ayant 1 à 4 atomes de carbone ; un groupe cycloalkyle ayant 3 à 6 atomes de carbone ; un groupe phényle ; ou un groupe phényle substitué par un groupe alkyle ayant 1 à 3 atomes de carbone, un groupe alcoxy ayant 1 à 3 atomes de carbone, un atome d'halogène ou un groupe nitro).

**10.** Procédé selon la revendication 1, dans lequel

$R^1$ représente un groupe de formule $-NR^{9a}C(=NR^{11c})R^{6p}$ où :

$R^{9a}$ représente un atome d'hydrogène ou un groupe méthyle ;

$R^{11c}$ représente : un atome d'hydrogène ; un groupe alkyle ayant 1 à 4 atomes de carbone ; un groupe phényle ; un groupe phényle substitué par un groupe alkyle ayant 1 à 3 atomes de carbone, un groupe alcoxy ayant 1 à 3 atomes de carbone, un atome d'halogène ou un groupe nitro ; un groupe de formule $-COOR^{7c}$ (où $R^{7c}$ représente : un groupe alkyle ayant 1 à 4 atomes de carbone ; un groupe cycloalkyle ayant 3 à 6 atomes de carbone ; ou un groupe benzyle) ; ou un groupe de formule $-COR^{6q}$ (où $R^{6q}$ représente : un groupe alkyle ayant 1 à 4 atomes de carbone ; un groupe cycloalkyle ayant 3 à 6 atomes de carbone ; un groupe phényle ; ou un groupe phényle substitué par un groupe alkyle ayant 1 à 3 atomes de carbone, un groupe alcoxy ayant 1 à 3 atomes de carbone, un atome d'halogène ou un groupe nitro) ;

$R^{6p}$ représente : un groupe alkyle ayant 1 à 4 atomes de carbone ; un groupe cycloalkyle ayant 3 à 6 atomes de carbone ; un groupe phényle ; ou un groupe phényle substitué par un groupe alkyle ayant 1 à 3 atomes de carbone, un groupe alcoxy ayant 1 à 3 atomes de carbone, un atome d'halogène ou un groupe nitro.

**11.** Procédé selon la revendication 1, dans lequel

$R^1$ représente un groupe de formule $-NR^{9a}SO_mR^{6r}$ où :

$R^{9a}$ représente un atome d'hydrogène ou un groupe méthyle ;

$R^{6r}$ représente : un groupe alkyle ayant 1 à 4 atomes de carbone ; un groupe alkyle substitué par un groupe cyano ; un groupe phényle ; ou un groupe phényle substitué par un groupe alkyle ayant 1 à 3 atomes de carbone, un groupe alcoxy ayant 1 à 3 atomes de carbone, un atome d'halogène ou un groupe nitro ; et

m est 1 ou 2.

**12.** Procédé selon l'une quelconque des revendications 1 à 11, dans lequel A représente un groupe de formule :

$-CHR^2-CHR^3-CHR^4-$

où $R^2$, $R^3$ et $R^4$ sont identiques ou différents et representent chacun un atome d'hydrogène ou un groupe méthyle.

**13.** Procédé selon l'une quelconque des revendications 1 à 11, dans lequel A représente un groupe de formule :

$-CR^2=CR^3-CHR^4-$

où $R^2$, $R^3$ et $R^4$ sont identiques ou différents et représentent chacun un atome d'hydrogène ou un groupe méthyle.

**14.** Procédé selon l'une quelconque des revendications précédentes, dans lequel le substituant $R^1$ est en position *méta* ou *ortho*.

**15.** Procédé selon la revendication 1, dans lequel A représente un groupe de formule :

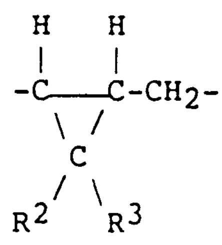

où $R^2$ et $R^3$ sont identiques ou différents et représentent chacun un atome d'hydrogène, un atome de

chlore ou un groupe méthyle.

**17.** Procédé selon l'une quelconque des revendications 1 à 16, dans lequel $R^5$ représente un groupe éthyle.

**18.** Procédé selon l'une quelconque des revendications 1 à 16, dans lequel la matière de départ est un mélange d'un composé dans lequel $R^5$ représente un groupe éthyle et du composé correspondant dans lequel $R^5$ représente un groupe méthyle, pour préparer un melange de composés de formule (I) dans l'un desquels $R^5$ représente un groupe éthyle et, dans l'autre, $R^5$ représente un groupe méthyle.

**19.** Procédé selon l'une quelconque des revendications précédentes, dans lequel chacun de $R^3$ et $R^4$ représente un atome d'hydrogène.

**20.** Procédé selon l'une quelconque des revendications précédentes, dans lequel X représente un groupe hydroxyle.

**21.** Procédé selon la revendication 1, dans lequel le composé préparé est :
la 13-(3-aminocinnamyloxy)milbémycine $A_4$
la 13-(2-aminocinnamyloxy)milbémycine $A_4$
la 13-(3-acétamidocinnamyloxy)milbémycine $A_4$
la 13-[3-(3-méthylthiouréido)cinnamyloxy]milbémycine $A_4$
la 13-(3-cyanoacétamidocinnamyloxy)milbémycine $A_4$
la 13-(3-méthanesulfonylaminocinnamyloxy)milbémycine $A_4$
la 13-[3-*N*-(méthanesulfonyl)méthylaminocinnamyloxy]milbémycine $A_4$
la 13-(3-méthylaminocinnamyloxy)milbémycine $A_4$ et
la 13-[3-(3,3-diméthylcarbazoylamino)cinnamyloxy]milbémycine $A_4$
ou un de leurs sels pharmaceutiquement acceptables.

**22.** Procédé selon la revendication 1, dans lequel le composé préparé est :
la 13-(3-cyanoacétamidocinnamyloxy)milbémycine $A_4$ ;
la 13-(3-méthanesulfonylaminocinnamyloxy)milbémycine $A_4$ ;
la 13-[3-*N*-(méthanesulfonyl)méthylaminocinnamyloxy]milbémycine $A_4$ ;
ou un de leurs sels pharmaceutiquement acceptables.

**23.** Utilisation d'un composé de formule (I) ou d'un sel ou ester de celui-ci, tel que défini dans l'une quelconque des revendications 1 à 23, pour la fabrication d'un médicament destiné au traitement d'animaux parasités par des helminthes, des acariens ou des insectes.

**24.** Utilisation selon la revendication 23, dans laquelle ledit composé est :
la 13-(3-aminocinnamyloxy)milbémycine $A_4$
la 13-(2-aminocinnamyloxy)milbémycine $A_4$
la 13-(3-acétamidocinnamyloxy)milbémycine $A_4$
la 13-[3-(3-méthylthiouréido)cinnamyloxy]milbémycine $A_4$
la 13-(3-cyanoacétamidocinnamyloxy)milbémycine $A_4$
la 13-(3-méthanesulfonylaminocinnamyloxy)milbémycine $A_4$
la 13-(3-*N*-(méthanesulfonyl)méthylaminocinnamyloxy]milbémycine $A_4$
la 13-(3-méthylaminocinnamyloxy)milbémycine $A_4$ et
la 13-[3-(3,3-diméthylcarbazoylamino)cinnamyloxy]milbémycine $A_4$
ou un de leurs sels pharmaceutiquement acceptables.